# EUROPÄISCHE PATENTANMELDUNG

(11) **EP 1 843 407 A1**
(43) Veröffentlichungstag der Anmeldung: **10.10.2007**
(21) Anmeldenummer: 06007415.0
(22) Anmeldetag: 07.04.2006
(51) Int. Cl.: H01L 51/00, H01L 51/05, H01L 51/42

(54) **Flüssig-kristalline Rylentetracarbonsäurederivate und deren Verwendung**

(71) Anmelder: BASF Aktiengesellschaft, 67056 Ludwigshafen (DE)
(72) Erfinder: Könemann, Martin, Dr., 68163 Mannheim (DE); Pschirer, Neil Gregory, Dr., 55116 Mainz (DE); Müllen, Klaus, Prof. Dr., 50939 Köln (DE); Nolde, Fabian, 65187 Wiesbaden (DE); Pisula, Wojcieh, 55118 Mainz (DE); Müller, Sybille, Dr., 55263 Wackernheim (DE); Kohl, Christopher, Dr., 55127 Mainz (DE)
(74) Vertreter: Reitstötter - Kinzebach

(57) **Zusammenfassung**

Die vorliegende Erfindung betrifft flüssig-kristalline Rylentetracarbonsäurederivate, Verfahren zu ihrer Herstellung und deren Verwendung als organische Halbleiter vom n-Typ zur Herstellung von organischen Feldeffekttransistoren und von Solarzellen.

## Beschreibung

Die vorliegende Erfindung betrifft flüssig-kristalline Rylentetracarbonsäurederivate, Verfahren zu ihrer Herstellung und deren Verwendung als organische Halbleiter vom n-Typ zur Herstellung von organischen Feldeffekttransistoren und von Solarzellen.

Es wird erwartet, dass zukünftig in vielen Bereichen der Elektronikindustrie neben den klassischen anorganischen Halbleitern zunehmend auch organische Halbleiter auf Basis von niedermolekularen oder polymeren Materialien eingesetzt werden. Diese weisen vielfach Vorteile gegenüber den klassischen anorganischen Halbleitern auf, beispielsweise eine bessere Substratkompatibilität und eine bessere Verarbeitbarkeit der auf ihnen basierenden Halbleiterbauteile. Sie erlauben die Verarbeitung auf flexiblen Substraten und ermöglichen es, ihre Grenzorbitalenergien mit den Methoden des Molecular Modellings auf den jeweiligen Anwendungsbereich genau anzupassen. Die deutlich verringerten Kosten derartiger Bauteile haben dem Forschungsgebiet der organischen Elektronik eine Renaissance gebracht. Die "Organische Elektronik" beschäftigt sich schwerpunktmäßig mit der Entwicklung neuer Materialien und Fertigungsprozesse für die Herstellung elektronischer Bauelemente auf der Basis organischer Halbleiterschichten. Dazu zählen vor allem organische Feldeffekttransistoren (Organic

Field-Effect Transistors, OFET) sowie organische Leuchtdioden (Organic Light Emitting Diodes, OLED; z.B. für einen Einsatz in Displays) und die organische Photovoltaik. Organischen Feldeffekttransistoren wird ein großes Entwicklungspotential, beispielsweise in Speicherelementen und integrierten optoelektronischen Vorrichtungen zugeschrieben. Es besteht daher ein großer Bedarf an organischen Verbindungen, die sich als organische Halbleiter, insbesondere Halbleiter vom n-Typ und speziell für einen Einsatz in organischen Feldeffekttransistoren und Solarzellen eignen.

Die Direktumwandlung von Solarenergie in elektrische Energie in Solarzellen beruht auf dem inneren Photoeffekt eines Halbleitermaterials, d.h. der Erzeugung von Elektron-Loch-Paaren durch Absorption von Photonen und der Trennung der negativen und positiven Ladungsträger an einem p-n-Übergang oder einem Schottky-Kontakt. Die so erzeugte Photospannung kann in einem äußeren Stromkreis einen Photostrom bewirken, durch den die Solarzelle ihre Leistung abgibt.

Von dem Halbleiter können dabei nur solche Photonen absorbiert werden, die eine Energie aufweisen, die größer als seine Bandlücke ist. Die Größe der Halbleiterband- lücke bestimmt also den Anteil des Sonnenlichts, der in elektrische Energie umgewandelt werden kann. Es wird zukünftig erwartet, dass organische Solarzellen die klassischen Solarzellen auf Siliziumbasis aufgrund geringerer Kosten, eines geringeren Gewichts, der Möglichkeit zur Herstellung flexibler und/oder farbiger Zellen, der besseren Möglichkeit zur Feinabstimmung des Bandabstands übertreffen werden. Es besteht somit ein großer Bedarf an organischen Halbleitern, die sich zur Herstellung organischer Solarzellen eignen.

Solarzellen bestehen normalerweise aus zwei absorbierenden Materialien mit unterschiedlichen Bandlücken, um die Sonnenenergie möglichst effektiv zu nutzen. Die ersten organischen Solarzellen bestanden aus einem zweilagigen System aus einem Kupfer-Phthalocyanin als p-Leiter und PTCBI als n-Leiter und zeigte einen Wirkungsgrad von 1 %. Um möglichst alle auftreffenden Photonen zu nutzen, werden relativ hohe Schichtdicken eingesetzt (z.B. 100 nm). Um Strom zu erzeugen, muss der durch die absorbierten Photonen erzeugte angeregte Zustand jedoch eine p-n junction erreichen, um ein Loch und ein Elektron zu erzeugen, welches dann zur Anode und Kathode fließt. Die meisten organische Halbleiter haben jedoch nur Diffusionslängen für den angeregten Zustand von bis zu 10 nm. Selbst durch die besten bisher bekannten Herstellverfahren kann die Distanz, über die der angeregte Zustand weitergeleitet werden muss auf mindestens 10 bis 30 nm verringert werden.

US 2005/0224905 beschreibt organische Photovoltaikzellen mit zwei organischen Lagen, wobei die Dicke der Lagen herstellungsbedingt maximal 0.8 charakteristische Transportlängen beträgt.

WO 2005/076383 beschreibt Phthalocyanin-Derivate, deren Verwendung als homöotrop orientierte Lagen in elektronischen Bauteilen und ein Verfahren zu ihrer Herstellung.

Die DE-A-19512773 beschreibt Quaterrylentetracarbonsäurediimide, die an den Imidstickstoffen sogenannte "Schwalbenschwanz"-Substituenten aufweisen und deren Verwendung als Fluoreszenzfarbstoffe. Konkret wird die Synthese von N,N'-di-(1-Hexylheptyl)-bis(dicarboximid) beschrieben. Unter einer Vielzahl von möglichen Anwendungsgebieten, die allesamt nicht durch Ausführungsbeispiele belegt sind, wird auch eine Verwendung als Farbstoffe zur Materialprüfung, z. B. bei der Herstellung von Halbleiterschaltungen, zur Untersuchung von Mikrostrukturen von integrierten Halbleiterbausteinen sowie in Fluoreszenz-Solarkollektoren beschrieben. Bezüglich der letztgenannten Anwendung wird auf H. Langhals, Nachr. Chem. Tech. Lab. 28 (1980) Seite 716 Bezug genommen. Danach ist ein Fluoreszenz-Solarkollektor eine Vorrichtung, die in der Lage ist diffuse Lichtstrahlung durch überwiegende Totalreflektion in der Vorrichtung zu konzentrieren. Eine mögliche Verwendung der genannten Verbindungen als n-Halbleiter ist nicht offenbart.

Die DE-A-10212358 beschreibt bichromophore Perylenderivate bei denen ein l-midstickstoff mit einem Akzeptor-Chromophor, z. B. einem Schwalbenschwanzrest, und der andere Amidstickstoff mit einem Donor-Chromophor, z. B. einem Aromaten, substituiert ist und deren Verwendung. Dabei ist auch ein Einsatz als Farbstoff in Fluoreszenz-Solakollektoren, zur Materialprüfung, z. B. bei der Herstellung von Halbleiterschaltungen, und zur Untersuchung von Mikrostrukturen von integrierten Halbleiterbausteinen erwähnt.

Die DE-A-10233179 beschreibt Perylentetracarbonsäurebisimide, bei denen einer der Imidstickstoffe einen Schwalbenschwanzrest und der andere einen ethylenisch ungesättigten Rest trägt.

Die DE-A-102004024909 beschreibt Perylentetracarbonsäurediimide mit höher verzweigten, gegebenenfalls substituierten Alkylsubstituenten an den Imidstickstoffatomen. Diese sollen sich unter anderem als Farbstoffe oder Fluoreszenzfarbstoffe als Teil einer integrierten Halbleiterschaltung eignen, die die Farbstoffe als solche oder in Verbindung mit anderen Halbleitern, z. B. in Form einer Epithaxie, enthält. Eine konkrete Befähigung dieser Verbindungen zum Einsatz als n-Halbleiter in organischen Feldeffekttransistoren und Solarzellen wird weder beschrieben, geschweige denn belegt.

In JP-2003138154 werden Terrylentetracarbonsäurediimide mit n-Alkylsubstituenten an den Imidstickstoffen beschrieben. Derartige Verbindungen sind jedoch in der Regel nicht flüssig-kristallin.

Die EP-A-0711812 beschreibt mehrfach chromophore Perylenimide, bei denen wenigstens zwei Perylentetracarbonsäuredümideinheiten über eines ihrer Imidstickstoffe an eine verbrückende Gruppe gebunden sind sowie ihre Verwendung unter anderem in Solarkollektoren.

Die DE-A-10225595 beschreibt 1,6,9,14-tetrasubstituierte Terrylentetracarbonsäurediimide und deren Verwendung unter anderem in der Photovoltaik. Ein Einsatz als n-Halbleiter zur Herstellung von Solarzellen ist nicht beschrieben.

DE-A-32 35 526 beschreibt Perylene-3,4,9,10-tetracarbonsäuredümide, bei denen der aromatische Kern mit wenigstens einer Gruppe, ausgewählt unter Alkoxy, Alkylthio, Aryloxy, Arylthio, =SO₂ und -SO₂-R Gruppen substituiert sein kann. Zusätzlich kann der aromatische Kern mit Chlor oder Brom substituiert sein. Ein Einsatz als n-Halbleiter in organischen Feldeffekttransistoren und Solarzellen ist nicht beschrieben.

DE-A-34 34 059 beschreibt chlorierte Perylenetetracarbonsäuredümide, wobei der aromatische Kern 2, 3, 5 oder 6 Chloratome trägt. Die Substituenten an den Imidstickstoffen sind ausgewählt unter a) geradkettigem oder verzweigtem C₁-C₁₈-Alkyl, das unsubstituiert oder mit Cyano, Hydroxyl, Cycloalkyl, Alkylcarbonyloxy, Alkenylcarbonyloxy oder Cycloalkylcarbonyloxy substituiert ist und wobei die Alkylkette durch O oder S unterbrochen sein kann, oder b) C₅-C₁₈-Cycloalkyl, das unsubstituiert oder mit Alkyl, Carboalkoxy oder Trifluormethyl substituiert ist. Ein Einsatz als n-Halbleiter in organischen Feldeffekttransistoren und Solarzellen ist nicht beschrieben.

DE-A-195 47 209 Beschreibt 1,7-disubstituierte Perylene-3,4,9,10-tetracarbonsäuredianhydride und Perylene-3,4,9,10-tetracarbonsäurediimide, bei denen der aromatische Kern mit wenigstens einer Gruppe, ausgewählt unter unsubstituiertem oder substituiertem Aryloxy, Arylthio, Hetaryloxy oder Hetarylthio, substituiert ist. Ein Einsatz als n-Halbleiter in organischen Feldeffekttransistoren und Solarzellen ist nicht beschrieben. H. Langhals and S. Kirner beschreiben in Eur. J. Org. Chem. 2000, 365-380 Fluoreszenzfarbstoffe auf Basis Kern-erweiterter Perylentetracarbonsäurebisimiden. Ein Einsatz als n-Halbleiter in organischen Feldeffekttransistoren und Solarzellen ist nicht beschrieben.

M. J. Ahrens, M. J. Fuller und M. R. Wasielewski beschreiben in Chem. Mater. 2003, 15, Seiten 2684 - 2686, cyanierte Perylen-3,4-dicarboximide und Perylen-3,4,9,10-bis(dicarboximide) als chromophore Oxidationsmittel für "Organic photonics and electronics". Konkrete Verbindungen, die an beiden Imidstickstoffen verzweigte Gruppen aufweisen und die flüssig kristallin sind, sind nicht beschrieben.

B. A. Jones et al. beschreiben in Angew. Chem. 2004, 116, Seiten 6523 - 6526, Dicyano-perylen-3,4,9,10-bis(dicarboximide) als luftstabile n-Halbleiter. Die Reste an den Imidstickstoffen sind Cyclohexyl und n-CH₂C₃F₇.

US 2005/0176970 A1 beschreibt die Verwendung von Perylen-3,4-dicarboximiden und

Perylen-3,4,9,10-bis(dicarboxi)imiden mit einer oder mehreren elektronenziehenden Gruppen als n-Halbleiter. Konkrete Verbindungen, die an beiden Imidstickstoffen verzweigte Gruppen aufweisen und die flüssig kristallin sind, sind nicht beschrieben.

In US 6,806,368 werden Perylentetracarbonsäuredümide mit Resten bschrieben, welche den Verbindungen flüssig kristalline Eigenschaften verleihen. Deren Verwendung in elektronsichen Bauteilen, in Transistoren wird erwähnt, die explizite Verwendung als n-Halbleiter zur Herstellung von organischen Feldeffekttransistoren und Solarzellen ist jedoch nicht beschrieben.

ChemPhysChem 2004, 5, 137 - 140 beschreibt Studien der strukturellen, elektrochemischen und Ladungstransporteigenschaften von Verbindungen der Formel 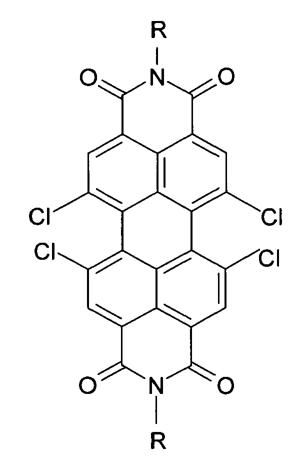 wobei R = n-C₁₂H₂₅, 4-(n- C₁₂H₂₅)C₆H₄, 2,6-(i-C₃H₇)₂C₆H₃. Organische Feldeffekttransistoren und Solarzellen sind nicht beschrieben.

J. Mater. Chem., 2005, 15, 1270 - 1276, isotrope Mobilitäten von flüssigkristallinen Rylencarbonsäureimiden der Formel 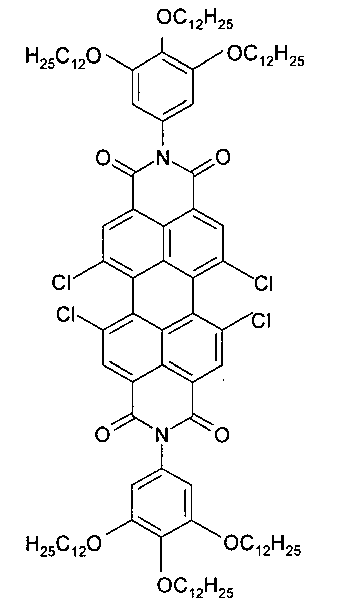 Organische Feldeffekttransistoren und Solarzellen sind nicht beschrieben.

US 2003/0181721 A1 beschreibt tetra-substituierte Perylentetracarbonsäurediimide der Formel 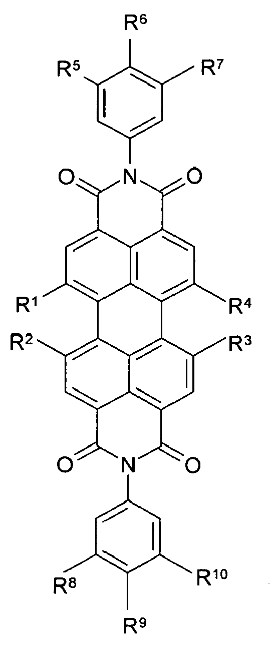 worin
R¹, R², R³ and R⁴ unabhängig Wasserstoff, Chlor, Brom, substituiertes oder unsubstituiertes Aryloxy, Arylthio, Arylamino, Hetaryloxy oder Hetarylthio sind,
R⁵, R⁶, R⁷, R⁸, R⁹ and R¹⁰ unabhängig Wasserstoff oder langkettiges Alkyl, Alkoxy oder Alkylthio sind mit der Maßgabe, daß wenigstens vier dieser Reste nicht Wasserstoff sind.
Organische Feldeffekttransistoren und Solarzellen sind nicht beschrieben.

WO 2005/124453 beschreibt mit Cyanogruppen substituierte Perylentetracarbonsäurediimide.

Organic Electronics 5 (2004), 237 - 249 vergleicht die elektrischen Eigenschaften dünner Filme von 1,6,7,12-Tetrachloro-N,N'-dimethylperylen-3,4,9,10-biscarboximid mit der nicht chlorierten Verbindung.

Der vorliegenden Erfindung liegt die Aufgabe zu Grunde, Verbindungen zur Verfügung zu stellen, die sich als n-Halbleiter, z. B. für einen Einsatz in organischen Feldeffekttransistoren und Solarzellen, eignen.

In allen genannten Schriften werden keine Aussagen über die Anordnung der Verbindungen auf den Substraten gemacht. Für die Eignung in OFETs und in Solarzellen ist die richtige Anordnung der Moleküle jedoch wichtig. Überraschenderweise wurde jetzt gefunden, dass die erfindungsgemäßen Verbindungen sowohl in der für Solarzellen günstigen face on, als auch in der für OFETs günstigen edge on Anordnung sich selbst organisieren können. Die Art der Anordnung kann gegebenenfalls dadurch beeinflusst werden, wie die Substratoberflächen vorbehandelt werden. Somit wird erstmals deutlich, dass die (im Allgemeinen flüssigkristallinen) Verbindungen auch tatsächlich für OFETs und in der Photovoltaik eingesetzt werden können.

Gelöst wird diese Aufgabe durch die Verwendung von Verbindungen der allgemeinen Formeln I und II 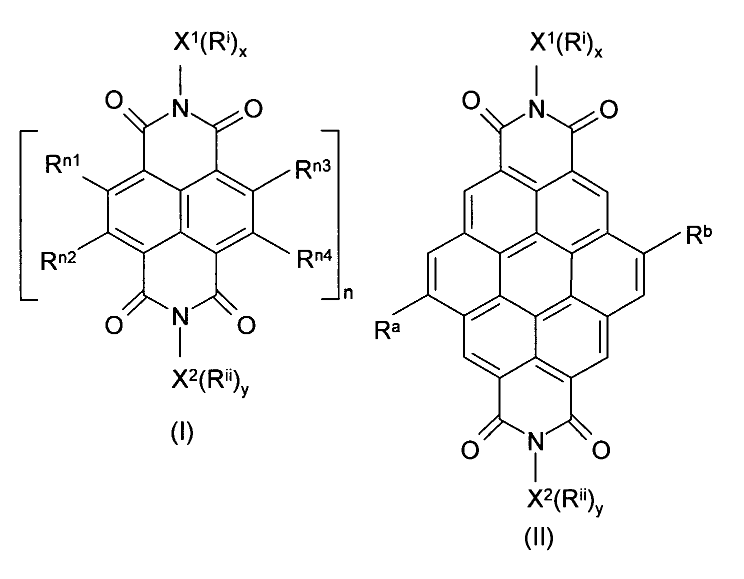 wobei
n für 1, 2, 3 oder 4 steht,
x und y unabhängig voneinander für eine ganze Zahl von 2 bis 6 stehen,
die Reste Rⁿ¹, Rⁿ², Rⁿ³ und Rⁿ⁴ für n = 1 oder 2 unabhängig voneinander ausgewählt sind unter Wasserstoff, F, Cl, Br und CN und für n = 3 oder 4 unabhängig voneinander ausgewählt sind unter Wasserstoff, F, Cl und Br,
die Reste R^{a} und R^{b} unabhängig voneinander ausgewählt sind unter Wasserstoff und Alkyl, ,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
als n-Halbleiter für organische Feldeffekttransistoren oder Solarzellen.

Die erfindungsgemäß verwendeten Verbindungen sind im Allgemeinen flüssig-kristallin. Aufgrund der damit verbundenen Fähigkeit geordnete Phasen auszubilden (flüssigkristalline Phasen, auch als Mesophasen bezeichnet), die zwischen Flüssigkeit und Feststoff liegen, und somit der prinzipiellen Befähigung zur Selbstorganisation eignen sie sich besonders vorteilhaft für die angestrebte Verwendung. Die erfindungsgemäßen Verbindungen weisen beispielsweise einen scheibenförmigen (diskotischen) Aufbau auf, der eine nematische oder kolumnare Anordnung haben kann. Sie zeichnen sich in der Regel durch eine hohe thermische Stabilität und hohe Phasenübergangstemperaturen für den Übergang vom geordneten zum isotropen Zustand aus, wobei mit zunehmender Größe des aromatischen Kerns die Phasenübergangstemperatur ansteigt. Sie eignen sich somit beispielsweise auch für einen Einsatz in elektronischen Bauteilen, wie Displays, die unter klimatisch ungünstigen Bedingungen betrieben werden, z.B. bei einem Außeneinsatz. In Abhängigkeit von ihrer Art (Rylen oder Corronen) und gegebenenfalls ihrem Substitutionsmuster können die erfindungsgemäß eingesetzten Verbindungen eine sogenannte "edge-on"-Anordnung annehmen, die sich besonders vorteilhaft bei Feldeffekttransistoren auswirkt, oder eine sogenannte "face-on"-Anordnung, die sich besonders vorteilhaft bei einem Einsatz in der Photovoltaik auswirkt.

Aufgrund ihrer hohen Ordnung und den damit im Allgemeinen verbundenen höheren charakteristischen Transportweiten für angeregte Zustände sowie höheren Ladungsmobilitäten, eignen sich die erfindungsgemäß eingesetzten organischen Halbleitermaterialien besonders vorteilhaft für einen Einsatz in Solarzellen. Sie eignen sich insbesondere zur Herstellung selbst-organisierender zwei- und mehrphasiger photovoltaischer Zellen mit sehr guten anwendungstechnischen Eigenschaften. Mit Solarzellen auf Basis dieser Halbleiter lassen sich in der Regel sehr gute Quantenausbeuten erzielen.

In den Verbindungen der Formel bezeichnet n die Anzahl der in der peri-Position verknüpften Naphthalineinheiten, die das Grundgerüst der erfindungsgemäßen Rylenverbindungen bilden. In den einzelnen Resten Rⁿ¹ bis Rⁿ⁴ bezeichnet n die jeweilige Naphthalingruppe des Rylengerüsts, an das die Reste gebunden sind. Reste Rⁿ¹ bis Rⁿ⁴, die an unterschiedliche Naphthalingruppen gebunden sind, können jeweils gleiche oder verschiedene Bedeutungen aufweisen. Demgemäß kann es sich bei den Verbindungen der allgemeinen Formel I um Naphthalindiimide, Perylendiimide, Terrylendiimide oder Quaterrylendiimide der folgenden Formel handeln:

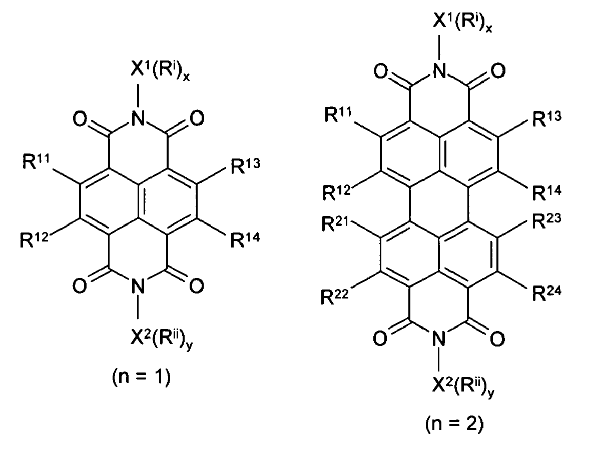

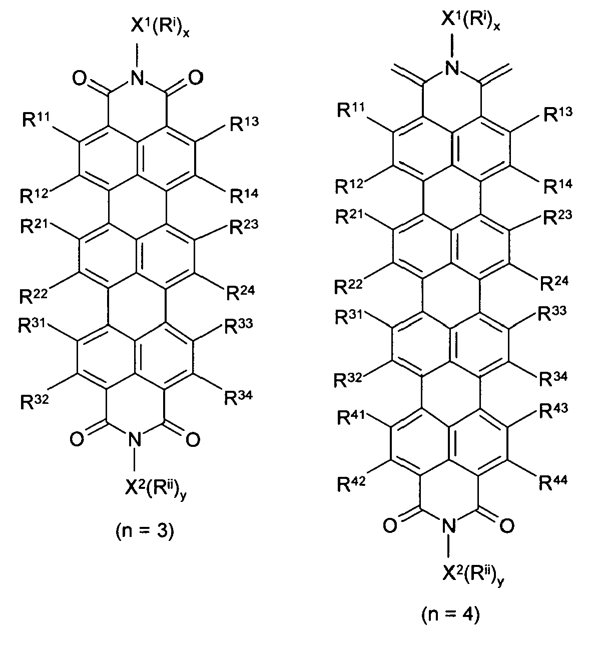

Vorzugsweise stehen in den Verbindungen der Formel I x und y unabhängig voneinander für 2 oder 3.

Unter einem (x+1)-wertigen Rest X¹ bzw. einem (y+1)-wertigen Rest X² wird im Rahmen der Erfindung eine Gruppe verstanden, die einerseits an den Imidstickstoff gebunden ist und andererseits x bzw. y an sie gebundene Alkylreste aufweist. Da x+1 bzw. y+1 immer für eine Zahl von wenigstens 3 steht, handelt es sich bei den erfindungsgemäßen Rylenen um Verbindungen, bei denen von zwei unmittelbar oder mittelbar Verzweigungszentrum zwei oder mehr Alkylketten ausgehen. X¹ bzw. X² kann dabei ein einzelnes Atom (z.B. ein tertiäres oder quartäres Kohlenstoffatom) oder eine Atomgruppe sein.

Die Gruppen X¹ und X² weisen vorzugsweise die folgenden Strukturelemente auf oder bestehen aus einer Kombination von diesen: -C-, -CH-, -CH₂-, -C=C-, -C≡C-, O, S, 4- bis 8-gliedrigen gesättigten oder ungesättigten Ringen, welche ein- oder mehrfach durch O, N, S unterbrochen sein können.

Die Atomanzahl der Gruppen X¹ und X² beträgt vorzugsweise höchstens 30, insbesondere höchstens 18.

In einer Verbindung der Formel I oder II können die Gruppen X¹(Rⁱ)ₓ und X²(Rⁱⁱ)_{y} gleiche oder verschiedene Bedeutungen besitzen. Bevorzugt besitzen die Gruppen X¹(Rⁱ)ₓ und X²(Rⁱⁱ)_{y} in einer Verbindung der Formel I oder II die gleiche Bedeutung.

Die verschiedenen Reste Rⁱ können jeweils gleiche oder verschiedene Bedeutungen aufweisen. Entsprechend können die Reste Rⁱⁱ jeweils gleiche oder verschiedene Bedeutungen aufweisen. Bevorzugt weisen alle Reste Rⁱ und Rⁱⁱ in einer Verbindung der Formel I oder II die gleiche Bedeutung auf.

Die Reste Rⁱ und Rⁱⁱ jeweils unabhängig voneinander ausgewählt sein unter linearem oder verzweigtem C₄- bis C₃₀-Alkyl, welches durch ein oder mehrere nicht benachbarte Sauerstoffatom(e) unterbrochen sein kann. Bevorzugt sind lineare Alkylreste. Bevorzugte Atomgruppen X¹(Rⁱ)ₓ sind die folgenden. Die Gruppen X²(Rⁱⁱ)_{y}, sofern von X¹ verschieden, werden analog gebildet (d.h. in den folgenden Formeln steht Rⁱⁱ statt Rⁱ und y statt x).

#―(A)ₚ―C(Rⁱ)ₓ (II.1)

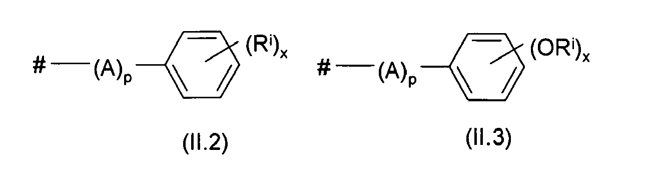

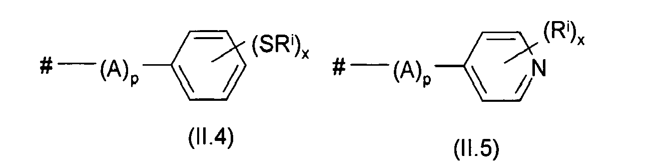

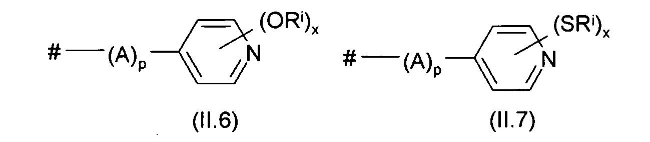

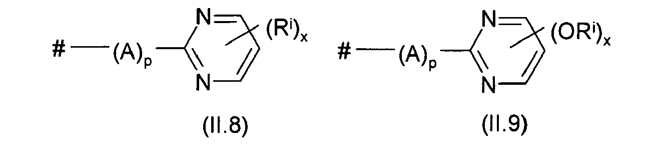

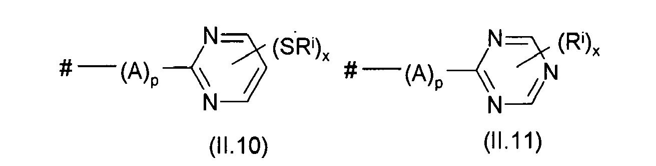

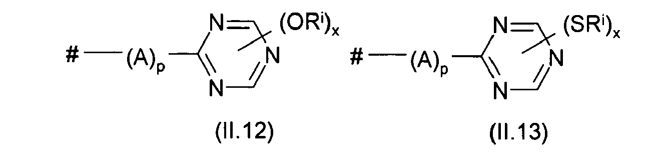

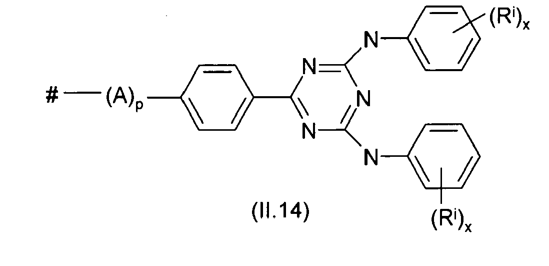

worin
- #: für die Verknüpfungsstelle zum Imidstickstoffatom steht,
- p: für 0 oder 1 steht,
- x: für 2 oder 3 steht, wobei in den Verbindungen der Formeln II.11, II.12 und II.13 x für 2 steht und wobei in den Verbindungen der Formel II.14 jedes x für 2 oder 3 stehen kann,
- A: soweit vorhanden, für eine C₁-C₁₀-Alkylengruppe steht, die durch eine oder mehrere nicht benachbarte Gruppen, die ausgewählt sind unter -O- und -S-, unterbrochen sein kann, und
- Rⁱ: die zuvor angegebene Bedeutung besitzt.

In einer bevorzugten Ausführung weisen die zuvor genannten Gruppen X¹(Rⁱ)ₓ bzw. X²(Rⁱⁱ)_{y} keine Alkylengruppe A auf. In einer weiteren bevorzugten Ausführung weisen die zuvor genannten Gruppen X¹(Rⁱ)ₓ bzw. X²(Rⁱⁱ)_{y} eine C₁-C₄-Alkylengruppe A auf, die durch 1, 2 oder 3 nicht benachbarte Gruppen, die ausgewählt sind unter -O- und -S-, unterbrochen sein kann.

Im Rahmen der vorliegenden Erfindung umfasst der Ausdruck "Alkyl" geradkettiges oder verzweigtes Alkyl. Vorzugsweise handelt es sich um geradkettiges oder verzweigtes C₁-C₃₀-Alkyl, insbesondere um C₁-C₂₀-Alkyl und ganz besonders bevorzugt

C₁-C₁₂-Alkyl. Beispiele für Alkylgruppen sind insbesondere Methyl, Ethyl, n-Propyl, Isopropyl, n-Butyl, Isobutyl, sec.-Butyl, tert.-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Hexadecyl, n-Octadecyl und n-Eicosyl.

Der Ausdruck Alkyl umfasst auch Alkylreste, deren Kohlenstoffketten durch eine oder mehrere nicht benachbarte Gruppen, die ausgewählt sind unter -O-, -S-, -NR^{e}-, -CO- und/oder -SO₂- unterbrochen sein kann. R^{e} steht vorzugsweise für Wasserstoff, Alkyl, Cycloalkyl, Heterocycloalkyl, Aryl oder Hetaryl. Der Ausdruck Alkyl umfasst auch substituierte Alkylreste.

Die vorstehenden Ausführungen zu Alkyl gelten auch für die Alkylteile in Alkoxy, Alkylamino, Alkylthio, etc.

Alkylen steht für eine lineare gesättigte Kohlenwasserstoffkette mit 1 bis 10 und insbesondere 1 bis 4 C-Atomen wie Ethan-1,2-diyl, Propan-1,3-diyl, Butan-1,4-diyl, Pentan-1,5-diyl oder Hexan-1,6-diyl.
Halogen steht für Fluor, Chlor, Brom oder Iod.

Als Beispiele für geeignete Gruppen X¹(Rⁱ)ₓ bzw. X²(Rⁱⁱ)_{y} seien im Einzelnen genannt:
Reste der Formel A 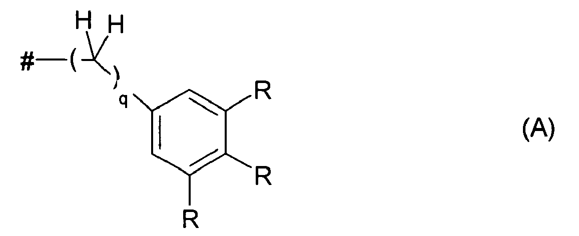 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.
Reste der Formel A umfassen solche, in denen q für 0 steht, wie z. B.
3,4,5-Tri(n-butyl)phenyl, 3,4,5-Tri(n-pentyl)phenyl, 3,4,5-Tri(n-hexyl)phenyl,
3,4,5-Tri(n-heptyl)phenyl, 3,4,5-Tri(n-octyl)phenyl, 3,4,5-Tri(n-nonyl)phenyl,
3,4,5-Tri(n-decyl)phenyl, 3,4,5-Tri(n-undecy)phenyl, 3,4,5-Tri(n-dodecyl)phenyl,
3,4,5-Tri(n-tridecyl)phenyl, 3,4,5-Tri(n-tetradecyl)phenyl, 3,4,5-Tri(n-pentadecyl)phenyl,
3,4,5-Tri(n-hexadecyl)phenyl, 3,4,5-Tri(n-heptadecyl)phenyl,
3,4,5-Tri(n-octadecyl)phenyl, 3,4,5-Tri(nonadecyl)phenyl, 3,4,5-Tri(eicosyl)phenyl,
3,4,5-Tri(docosanyl)phenyl, 3,4,5-Tri(tricosanyl)phenyl, 3,4,5-Tri(tetracosanyl)phenyl,
3,4,5-Tri(octacosanyl)phenyl;

3,4,5-Tri(n-butyloxy)phenyl, 3,4,5-Tri(n-pentyloxy)phenyl, 3,4,5-Tri(n-hexyloxy)phenyl,
3,4,5-Tri(n-heptyloxy)phenyl, 3,4,5-Tri(n-octyloxy)phenyl, 3,4,5-Tri(n-nonyloxy)phenyl,
3,4,5-Tri(n-decyloxy)phenyl, 3,4,5-Tri(n-undecyloxy)phenyl,
3,4,5-Tri(n-dodecyloxy)phenyl, 3,4,5-Tri(n-tridecyloxy)phenyl,
3,4,5-Tri(n-tetradecyloxy)phenyl, 3,4,5-Tri(n-pentadecyloxy)phenyl,
3,4,5-Tri(n-hexadecyloxy)phenyl, 3,4,5-Tri(n-heptadecyloxy)phenyl,
3,4,5-Tri(n-octadecyloxy)phenyl, 3,4,5-Tri(nonadecyloxy)phenyl,
3,4,5-Tri(eicosyloxy)phenyl, 3,4,5-Tri(docosanyloxy)phenyl,
3,4,5-Tri(tricosanyloxy)phenyl, 3,4,5-Tri(tetracosanyloxy)phenyl,
3,4,5-Tri(octacosanyloxy)phenyl;

3,4,5-Tri(n-butylthio)phenyl, 3,4,5-Tri(n-pentylthio)phenyl, 3,4,5-Tri(n-hexylthio)phenyl,
3,4,5-Tri(n-heptylthio)phenyl, 3,4,5-Tri(n-octylthio)phenyl, 3,4,5-Tri(n-nonylthio)phenyl,
3,4,5-Tri(n-decylthio)phenyl, 3,4,5-Tri(n-undecylthio)phenyl,
3,4,5-Tri(n-dodecylthio)phenyl, 3,4,5-Tri(n-tridecylthio)phenyl,
3,4,5-Tri(n-tetradecylthio)phenyl, 3,4,5-Tri(n-pentadecylthio)phenyl,
3,4,5-Tri(n-hexadecylthio)phenyl, 3,4,5-Tri(n-heptadecylthio)phenyl,
3,4,5-Tri(n-octadecylthio)phenyl, 3,4,5-Tri(nonadecylthio)phenyl,
3,4,5-Tri(eicosylthio)phenyl, 3,4,5-Tri(docosanylthio)phenyl,
3,4,5-Tri(tricosanylthio)phenyl, 3,4,5-Tri(tetracosanylthio)phenyl,
3,4,5-Tri(octacosanylthio)phenyl,
in denen q für 1 steht, wie z. B.
3,4,5-Tri(n-butyl)benzyl, 3,4,5-Tri(n-pentyl)benzyl, 3,4,5-Tri(n-hexyl)benzyl,
3,4,5-Tri(n-heptyl)benzyl, 3,4,5-Tri(n-octyl)benzyl, 3,4,5-Tri(n-nonyl)benzyl,
3,4,5-Tri(n-decyl)benzyl, 3,4,5-Tri(n-undecy)benzyl, 3,4,5-Tri(n-dodecyl)benzyl,
3,4,5-Tri(n-tridecyl)benzyl, 3,4,5-Tri(n-tetradecyl)benzyl, 3,4,5-Tri(n-pentadecyl)benzyl,
3,4,5-Tri(n-hexadecyl)benzyl, 3,4,5-Tri(n-heptadecyl)benzyl,
3,4,5-Tri(n-octadecyl)benzyl, 3,4,5-Tri(nonadecyl)benzyl, 3,4,5-Tri(eicosyl)benzyl,
3,4,5-Tri(docosanyl)benzyl, 3,4,5-Tri(tricosanyl)benzyl, 3,4,5-Tri(tetracosanyl)benzyl,
3,4,5-Tri(octacosanyl)benzyl;

3,4,5-Tri(n-butyloxy)benzyl, 3,4,5-Tri(n-pentyloxy)benzyl, 3,4,5-Tri(n-hexyloxy)benzyl,
3,4,5-Tri(n-heptyloxy)benzyl, 3,4,5-Tri(n-octyloxy)benzyl, 3,4,5-Tri(n-nonyloxy)benzyl,
3,4,5-Tri(n-decyloxy)benzyl, 3,4,5-Tri(n-undecyloxy)benzyl,
3,4,5-Tri(n-dodecyloxy)benzyl, 3,4,5-Tri(n-tridecyloxy)benzyl,
3,4,5-Tri(n-tetradecyloxy)benzyl, 3,4,5-Tri(n-pentadecyloxy)benzyl,
3,4,5-Tri(n-hexadecyloxy)benzyl, 3,4,5-Tri(n-heptadecyloxy)benzyl,
3,4,5-Tri(n-octadecyloxy)benzyl, 3,4,5-Tri(nonadecyloxy)benzyl,
3,4,5-Tri(eicosyloxy)benzyl, 3,4,5-Tri(docosanyloxy)benzyl,
3,4,5-Tri(tricosanyloxy)benzyl, 3,4,5-Tri(tetracosanyloxy)benzyl,
3,4,5-Tri(octacosanyloxy)benzyl;

3,4,5-Tri(n-butylthio)benzyl, 3,4,5-Tri(n-pentylthio)benzyl, 3,4,5-Tri(n-hexylthio)benzyl,
3,4,5-Tri(n-heptylthio)benzyl, 3,4,5-Tri(n-octylthio)benzyl, 3,4,5-Tri(n-nonylthio)benzyl,
3,4,5-Tri(n-decylthio)benzyl, 3,4,5-Tri(n-undecylthio)benzyl,
3,4,5-Tri(n-dodecylthio)benzyl, 3,4,5-Tri(n-tridecylthio)benzyl,
3,4,5-Tri(n-tetradecylthio)benzyl, 3,4,5-Tri(n-pentadecylthio)benzyl,
3,4,5-Tri(n-hexadecylthio)benzyl, 3,4,5-Tri(n-heptadecylthio)benzyl,
3,4,5-Tri(n-octadecylthio)benzyl, 3,4,5-Tri(nonadecylthio)benzyl,
3,4,5-Tri(eicosylthio)benzyl, 3,4,5-Tri(docosanylthio)benzyl,
3,4,5-Tri(tricosanylthio)benzyl, 3,4,5-Tri(tetracosanylthio)benzyl,
3,4,5-Tri(octacosanylthio)benzyl;
in denen q für 2 steht, wie z. B.
3,4,5-Tri(n-butyl)phenethyl, 3,4,5-Tri(n-pentyl)phenethyl, 3,4,5-Tri(n-hexyl)phenethyl,
3,4,5-Tri(n-heptyl)phenethyl, 3,4,5-Tri(n-octyl)phenethyl, 3,4,5-Tri(n-nonyl)phenethyl,
3,4,5-Tri(n-decyl)phenethyl, 3,4,5-Tri(n-undecy)phenethyl,
3,4,5-Tri(n-dodecyl)phenethyl, 3,4,5-Tri(n-tridecyl)phenethyl,
3,4,5-Tri(n-tetradecyl)phenethyl, 3,4,5-Tri(n-pentadecyl)phenethyl,
3,4,5-Tri(n-hexadecyl)phenethyl, 3,4,5-Tri(n-heptadecyl)phenethyl,
3,4,5-Tri(n-octadecyl)phenethyl, 3,4,5-Tri(nonadecyl)phenethyl,
3,4,5-Tri(eicosyl)phenethyl, 3,4,5-Tri(docosanyl)phenethyl,
3,4,5-Tri(tricosanyl)phenethyl, 3,4,5-Tri(tetracosanyl)phenethyl,
3,4,5-Tri(octacosanyl)phenethyl;

3,4,5-Tri(n-butyloxy)phenethyl, 3,4,5-Tri(n-pentyloxy)phenethyl,
3,4,5-Tri(n-hexyloxy)phenethyl, 3,4,5-Tri(n-heptyloxy)phenethyl,
3,4,5-Tri(n-octyloxy)phenethyl, 3,4,5-Tri(n-nonyloxy)phenethyl,
3,4,5-Tri(n-decyloxy)phenethyl, 3,4,5-Tri(n-undecyloxy)phenethyl,
3,4,5-Tri(n-dodecyloxy)phenethyl, 3,4,5-Tri(n-tridecyloxy)phenethyl,
3,4,5-Tri(n-tetradecyloxy)phenethyl, 3,4,5-Tri(n-pentadecyloxy)phenethyl,
3,4,5-Tri(n-hexadecyloxy)phenethyl, 3,4,5-Tri(n-heptadecyloxy)phenethyl,
3,4,5-Tri(n-octadecyloxy)phenethyl, 3,4,5-Tri(nonadecyloxy)phenethyl,
3,4,5-Tri(eicosyloxy)phenethyl, 3,4,5-Tri(docosanyloxy)phenethyl,
3,4,5-Tri(tricosanyloxy)phenethyl, 3,4,5-Tri(tetracosanyloxy)phenethyl,
3,4,5-Tri(octacosanyloxy)phenethyl;

3,4,5-Tri(n-butylthio)phenethyl, 3,4,5-Tri(n-pentylthio)phenethyl,
3,4,5-Tri(n-hexylthio)phenethyl, 3,4,5-Tri(n-heptylthio)phenethyl,
3,4,5-Tri(n-octylthio)phenethyl, 3,4,5-Tri(n-nonylthio)phenethyl,
3,4,5-Tri(n-decylthio)phenethyl, 3,4,5-Tri(n-undecylthio)phenethyl,
3,4,5-Tri(n-dodecylthio)phenethyl, 3,4,5-Tri(n-tridecylthio)phenethyl,
3,4,5-Tri(n-tetradecylthio)phenethyl, 3,4,5-Tri(n-pentadecylthio)phenethyl,
3,4,5-Tri(n-hexadecylthio)phenethyl, 3,4,5-Tri(n-heptadecylthio)phenethyl,
3,4,5-Tri(n-octadecylthio)phenethyl, 3,4,5-Tri(nonadecylthio)phenethyl,
3,4,5-Tri(eicosylthio)phenethyl, 3,4,5-Tri(docosanylthio)phenethyl,
3,4,5-Tri(tricosanylthio)phenethyl, 3,4,5-Tri(tetracosanylthio)phenethyl,
3,4,5-Tri(octacosanylthio)phenethyl;
in denen q für 3 steht, wie z. B.
3-(3,4,5-Tri(n-butyl)phenyl)propyl, 3-(3,4,5-Tri(n-pentyl)phenyl)propyl,
3-(3,4,5-Tri(n-hexyl)phenyl)propyl, 3-(3,4,5-Tri(n-heptyl)phenyl)propyl,
3-(3,4,5-Tri(n-octyl)phenyl)propyl, 3-(3,4,5-Tri(n-nonyl)phenyl)propyl,
3-(3,4,5-Tri(n-decyl)phenyl)propyl, 3-(3,4,5-Tri(n-undecy)phenyl)propyl,
3-(3,4,5-Tri(n-dodecyl)phenyl)propyl, 3-(3,4,5-Tri(n-tridecyl)phenyl)propyl,
3-(3,4,5-Tri(n-tetradecyl)phenyl)propyl, 3-(3,4,5-Tri(n-pentadecyl)phenyl)propyl,
3-(3,4,5-Tri(n-hexadecyl)phenyl)propyl, 3-(3,4,5-Tri(n-heptadecyl)phenyl)propyl,
3-(3,4,5-Tri(n-octadecyl)phenyl)propyl, 3-(3,4,5-Tri(nonadecyl)phenyl)propyl,
3-(3,4,5-Tri(eicosyl)phenyl)propyl, 3-(3,4,5-Tri(docosanyl)phenyl)propyl,
3-(3,4,5-Tri(tricosanyl)phenyl)propyl, 3-(3,4,5-Tri(tetracosanyl)phenyl)propyl,
3-(3,4,5-Tri(octacosanyl)phenyl)propyl;

3-(3,4,5-Tri(n-butyloxy)phenyl)propyl, 3-(3,4,5-Tri(n-pentyloxy)phenyl)propyl,
3-(3,4,5-Tri(n-hexyloxy)phenyl)propyl, 3-(3,4,5-Tri(n-heptyloxy)phenyl)propyl,
3-(3,4,5-Tri(n-octyloxy)phenyl)propyl, 3-(3,4,5-Tri(n-nonyloxy)phenyl)propyl,
3-(3,4,5-Tri(n-decyloxy)phenyl)propyl, 3-(3,4,5-Tri(n-undecyloxy)phenyl)propyl,
3-(3,4,5-Tri(n-dodecyloxy)phenyl)propyl, 3-(3,4,5-Tri(n-tridecyloxy)phenyl)propyl,
3-(3,4,5-Tri(n-tetradecyloxy)phenyl)propyl, 3-(3,4,5-Tri(n-pentadecyloxy)phenyl)propyl,
3-(3,4,5-Tri(n-hexadecyloxy)phenyl)propyl, 3-(3,4,5-Tri(n-heptadecyloxy)phenyl)propyl,
3-(3,4,5-Tri(n-octadecyloxy)phenyl)propyl, 3-(3,4,5-Tri(nonadecyloxy)phenyl)propyl,
3-(3,4,5-Tri(eicosyloxy)phenyl)propyl, 3-(3,4,5-Tri(docosanyloxy)phenyl)propyl,
3-(3,4,5-Tri(tricosanyloxy)phenyl)propyl, 3-(3,4,5-Tri(tetracosanyloxy)phenyl)propyl,
3-(3,4,5-Tri(octacosanyloxy)phenyl)propyl;

3-(3,4,5-Tri(n-butylthio)phenyl)propyl, 3-(3,4,5-Tri(n-pentylthio)phenyl)propyl,
3-(3,4,5-Tri(n-hexylthio)phenyl)propyl, 3-(3,4,5-Tri(n-heptylthio)phenyl)propyl,
3-(3,4,5-Tri(n-octylthio)phenyl)propyl, 3-(3,4,5-Tri(n-nonylthio)phenyl)propyl,
3-(3,4,5-Tri(n-decylthio)phenyl)propyl, 3-(3,4,5-Tri(n-undecylthio)phenyl)propyl,
3-(3,4,5-Tri(n-dodecylthio)phenyl)propyl, 3-(3,4,5-Tri(n-tridecylthio)phenyl)propyl,
3-(3,4,5-Tri(n-tetradecylthio)phenyl)propyf, 3-(3,4,5-Tri(n-pentadecylthio)phenyl)propyl,
3-(3,4,5-Tri(n-hexadecylthio)phenyl)propyl, 3-(3,4,5-Tri(n-heptadecylthio)phenyl)propyl,
3-(3,4,5-Tri(n-octadecylthio)phenyl)propyl, 3-(3,4,5-Tri(nonadecylthio)phenyl)propyl,
3-(3,4,5-Tri(eicosylthio)phenyl)propyl, 3-(3,4,5-Tri(docosanylthio)phenyl)propyl,
3-(3,4,5-Tri(tricosanylthio)phenyl)propyl, 3-(3,4,5-Tri(tetracosanylthio)phenyl)propyl,
3-(3,4,5-Tri(octacosanylthio)phenyl)propyl; und
in denen q für 4 steht, wie z. B.
4-(3,4,5-Tri(n-butyl)phenyl)butyl, 4-(3,4,5-Tri(n-pentyl)phenyl)butyl,
4-(3,4,5-Tri(n-hexyl)phenyl)butyl, 4-(3,4,5-Tri(n-heptyl)phenyl)butyl,
4-(3,4,5-Tri(n-octyl)phenyl)butyl, 4-(3,4,5-Tri(n-nonyl)phenyl)butyl,
4-(3,4,5-Tri(n-decyl)phenyl)butyl, 4-(3,4,5-Tri(n-undecy)phenyl)butyl,
4-(3,4,5-Tri(n-dodecyl)phenyl)butyl, 4-(3,4,5-Tri(n-tridecyl)phenyl)butyl,
4-(3,4,5-Tri(n-tetradecyl)phenyl)butyl, 4-(3,4,5-Tri(n-pentadecyl)phenyl)butyl,
4-(3,4,5-Tri(n-hexadecyl)phenyl)butyl, 4-(3,4,5-Tri(n-heptadecyl)phenyl)butyl,
4-(3,4,5-Tri(n-octadecyl)phenyl)butyl, 4-(3,4,5-Tri(nonadecyl)phenyl)butyl,
4-(3,4,5-Tri(eicosyl)phenyl)butyl, 4-(3,4,5-Tri(docosanyl)phenyl)butyl,
4-(3,4,5-Tri(tricosanyl)phenyl)butyl, 4-(3,4,5-Tri(tetracosanyl)phenyl)butyl,
4-(3,4,5-Tri(octacosanyl)phenyl)butyl;

4-(3,4,5-Tri(n-butyloxy)phenyl)butyl, 4-(3,4,5-Tri(n-pentyloxy)phenyl)butyl,
4-(3,4,5-Tri(n-hexyloxy)phenyl)butyl, 4-(3,4,5-Tri(n-heptyloxy)phenyl)butyl,
4-(3,4,5-Tri(n-octyloxy)phenyl)butyl, 4-(3,4,5-Tri(n-nonyloxy)phenyl)butyl,
4-(3,4,5-Tri(n-decyloxy)phenyl)butyl, 4-(3,4,5-Tri(n-undecyloxy)phenyl)butyl,
4-(3,4,5-Tri(n-dodecyloxy)phenyl)butyl, 4-(3,4,5-Tri(n-tridecyloxy)phenyl)butyl,
4-(3,4,5-Tri(n-tetradecyloxy)phenyl)butyl, 4-(3,4,5-Tri(n-pentadecyloxy)phenyl)butyl,
4-(3,4,5-Tri(n-hexadecyloxy)phenyl)butyl, 4-(3,4,5-Tri(n-heptadecyloxy)phenyl)butyl,
4-(3,4,5-Tri(n-octadecyloxy)phenyl)butyl, 4-(3,4,5-Tri(nonadecyloxy)phenyl)butyl,
4-(3,4,5-Tri(eicosyloxy)phenyl)butyl, 4-(3,4,5-Tri(docosanyloxy)phenyl)butyl,
4-(3,4,5-Tri(tricosanyloxy)phenyl)butyl, 4-(3,4,5-Tri(tetracosanyloxy)phenyl)butyl,
4-(3,4,5-Tri(octacosanyloxy)phenyl)butyl;

4-(3,4,5-Tri(n-butylthio)phenyl)butyl, 4-(3,4,5-Tri(n-pentylthio)phenyl)butyl,
4-(3,4,5-Tri(n-hexylthio)phenyl)butyl, 4-(3,4,5-Tri(n-heptylthio)phenyl)butyl,
4-(3,4,5-Tri(n-octylthio)phenyl)butyl, 4-(3,4,5-Tri(n-nonylthio)phenyl)butyl,
4-(3,4,5-Tri(n-decylthio)phenyl)butyl, 4-(3,4,5-Tri(n-undecylthio)phenyl)butyl,
4-(3,4,5-Tri(n-dodecylthio)phenyl)butyl, 4-(3,4,5-Tri(n-tridecylthio)phenyl)butyl,
4-(3,4,5-Tri(n-tetradecylthio)phenyl)butyl, 4-(3,4,5-Tri(n-pentadecylthio)phenyl)butyl,
4-(3,4,5-Tri(n-hexadecylthio)phenyl)butyl, 4-(3,4,5-Tri(n-heptadecylthio)phenyl)butyl,
4-(3,4,5-Tri(n-octadecylthio)phenyl)butyl, 4-(3,4,5-Tri(nonadecylthio)phenyl)butyl,
4-(3,4,5-Tri(eicosylthio)phenyl)butyl, 4-(3,4,5-Tri(docosanylthio)phenyl)butyl,
4-(3,4,5-Tri(tricosanylthio)phenyl)butyl, 4-(3,4,5-Tri(tetracosanylthio)phenyl)butyl,
4-(3,4,5-Tri(octacosanylthio)phenyl)butyl;
des Weiteren Reste der Formel B 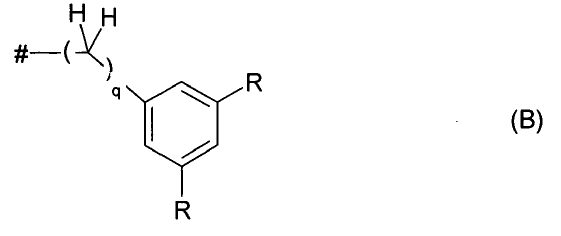 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Beispiele für Reste der Formel B umfassen solche, in denen q für 0 steht, wie z. B.
3,5-Di(n-butyl)phenyl, 3,5-Di(n-pentyl)phenyl, 3,5-Di(n-hexyl)phenyl,
3,4,5-Di(n-heptyl)phenyl, 3,5-Di(n-octyl)phenyl, 3,5-Di(n-nonyl)phenyl,
3,5-Dii(n-decyl)phenyl, 3,5-Di(n-undecy)phenyl, 3,5-Di(n-dodecyl)phenyl,
3,5-Di(n-tridecyl)phenyl, 3,5-Di(n-tetradecyl)phenyl, 3,5-Di(n-pentadecyl)phenyl,
3,5-Di(n-hexadecyl)phenyl, 3,5-Di(n-heptadecyl)phenyl, 3,5-Di(n-octadecyl)phenyl,
3,5-Di(nonadecyl)phenyl, 3,5-Di(eicosyl)phenyl, 3,5-Di(docosanyl)phenyl,
3,5-Di(tricosanyl)phenyl, 3,5-Di(tetracosanyl)phenyl, 3,5-Di(octacosanyl)phenyl;

3,5-Di(n-butyloxy)phenyl,3,5-Di(n-pentyloxy)phenyl, 3,5-Di(n-hexyloxy)phenyl,
3,4,5-Di(n-heptyloxy)phenyl, 3,5-Di(n-octyloxy)phenyl, 3,5-Di(n-nonyloxy)phenyl,
3,5-Dii(n-decyloxy)phenyl, 3,5-Di(n-undecyoxy)phenyl, 3,5-Di(n-dodecyloxy)phenyl,
3,5-Di(n-tridecyloxy)phenyl, 3,5-Di(n-tetradecyloxy)phenyl,
3,5-Di(n-pentadecyloxy)phenyl, 3,5-Di(n-hexadecyoloxy)phenyl,
3,5-Di(n-heptadecyloxy)phenyl, 3,5-Di(n-octadecyloxy)phenyl,
3,5-Di(nonadecyloxy)phenyl, 3,5-Di(eicosyloxy)phenyl, 3,5-Di(docosanyloxy)phenyl,
3,5-Di(tricosanyloxy)phenyl, 3,5-Di(tetracosanyloxy)phenyl,
3,5-Di(octacosanyloxy)phenyl;

3,5-Di(n-butylthio)phenyl, 3,5-Di(n-pentylthio)phenyl, 3,5-Di(n-hexylthio)phenyl,
3,4,5-Di(n-heptylthio)phenyl, 3,5-Di(n-octylthio)phenyl, 3,5-Di(n-nonylthio)phenyl,
3,5-Dii(n-decylthio)phenyl, 3,5-Di(n-undecythio)phenyl, 3,5-Di(n-dodecylthio)phenyl,
3,5-Di(n-tridecylthio)phenyl, 3,5-Di(n-tetradecylthio)phenyl,
3,5-Di(n-pentadecylthio)phenyl, 3,5-Di(n-hexadecyolthio)phenyl,
3,5-Di(n-heptadecylthio)phenyl, 3,5-Di(n-octadecylthio)phenyl,
3,5-Di(nonadecylthio)phenyl, 3,5-Di(eicosylthio)phenyl, Di(docosanylthio)phenyl,
Di(tricosanylthio)phenyl, Di(tetracosanylthio)phenyl, Di(octacosanylthio)phenyl;
in denen q für 1 steht, wie beispielsweise
3,5-Di(n-butyl)benzyl, 3,5-Di(n-pentyl)benzyl, 3,5-Di(n-hexyl)benzyl,
3,4,5-Di(n-heptyl)benzyl, 3,5-Di(n-octyl)benzyl, 3,5-Di(n-nonyl)benzyl,
3,5-Dii(n-decyl)benzyl, 3,5-Di(n-undecy)benzyl, 3,5-Di(n-dodecyl)benzyl,
3,5-Di(n-tridecyl)benzyl, 3,5-Di(n-tetradecyl)benzyl, 3,5-Di(n-pentadecyl)benzyl,
3,5-Di(n-hexadecyl)benzyl, 3,5-Di(n-heptadecyl)benzyl, 3,5-Di(n-octadecyl)benzyl,
3,5-Di(nonadecyl)benzyl, 3,5-Di(eicosyl)benzyl, 3,5-Di(docosanyl)benzyl,
3,5-Di(tricosanyl)benzyl, 3,5-Di(tetracosanyl)benzyl, 3,5-Di(octacosanyl)benzyl;

3,5-Di(n-butyloxy)benzyl, 3,5-Di(n-pentyloxy)benzyl, 3,5-Di(n-hexyloxy)benzyl,
3,4,5-Di(n-heptyloxy)benzyl, 3,5-Di(n-octyloxy)benzyl, 3,5-Di(n-nonyloxy)benzyl,
3,5-Dii(n-decyloxy)benzyl, 3,5-Di(n-undecyoxy)benzyl, 3,5-Di(n-dodecyloxy)benzyl,
3,5-Di(n-tridecyloxy)benzyl, 3,5-Di(n-tetradecyloxy)benzyl,
3,5-Di(n-pentadecyloxy)benzyl, 3,5-Di(n-hexadecyoloxy)benzyl,
3,5-Di(n-heptadecyloxy)benzyl, 3,5-Di(n-octadecyloxy)benzyl,
3,5-Di(nonadecyloxy)benzyl, 3,5-Di(eicosyloxy)benzyl, 3,5-Di(docosanyloxy)benzyl,
3,5-Di(tricosanyloxy)benzyl, 3,5-Di(tetracosanyloxy)benzyl,
3,5-Di(octacosanyloxy)benzyl;

3,5-Di(n-butylthio)benzyl, 3,5-Di(n-pentylthio)benzyl, 3,5-Di(n-hexylthio)benzyl,
3,4,5-Di(n-heptylthio)benzyl, 3,5-Di(n-octylthio)benzyl, 3,5-Di(n-nonylthio)benzyl,
3,5-Dii(n-decylthio)benzyl, 3,5-Di(n-undecythio)benzyl, 3,5-Di(n-dodecylthio)benzyl,
3,5-Di(n-tridecylthio)benzyl, 3,5-Di(n-tetradecylthio)benzyl,
3,5-Di(n-pentadecylthio)benzyl, 3,5-Di(n-hexadecyolthio)benzyl,
3,5-Di(n-heptadecylthio)benzyl, 3,5-Di(n-octadecylthio)benzyl,
3,5-Di(nonadecylthio)benzyl, 3,5-Di(eicosylthio)benzyl, 3,5-Di(docosanylthio)benzyl,
3,5-Di(tricosanylthio)benzyl, 3,5-Di(tetracosanylthio)benzyl,
3,5-Di(octacosanylthio)benzyl;
in denen q für 2 steht, wie z. B.
3,5-Di(n-butyl)phenethyl, 3,5-Di(n-pentyl)phenethyl, 3,5-Di(n-hexyl)phenethyl,
3,4,5-Di(n-heptyl)phenethyl, 3,5-Di(n-octyl)phenethyl, 3,5-Di(n-nonyl)phenethyl,
3,5-Dii(n-decyl)phenethyl, 3,5-Di(n-undecy)phenethyl, 3,5-Di(n-dodecyl)phenethyl,
3,5-Di(n-tridecyl)phenethyl, 3,5-Di(n-tetradecyl)phenethyl,
3,5-Di(n-pentadecyl)phenethyl, 3,5-Di(n-hexadecyl)phenethyl,
3,5-Di(n-heptadecyl)phenethyl, 3,5-Di(n-octadecyl)phenethyl,
3,5-Di(nonadecyl)phenethyl, 3,5-Di(eicosyl)phenethyl, 3,5-Di(docosanyl)phenethyl,
3,5-Di(tricosanyl)phenethyl, 3,5-Di(tetracosanyl)phenethyl,
3,5-Di(octacosanyl)phenethyl;

3,5-Di(n-butyloxy)phenethyl, 3,5-Di(n-pentyloxy)phenethyl,
3,5-Di(n-hexyloxy)phenethyl, 3,4,5-Di(n-heptyloxy)phenethyl,
3,5-Di(n-octyloxy)phenethyl, 3,5-Di(n-nonyloxy)phenethyl,
3,5-Dii(n-decyloxy)phenethyl, 3,5-Di(n-undecyoxy)phenethyl,
3,5-Di(n-dodecyloxy)phenethyl, 3,5-Di(n-tridecyloxy)phenethyl,
3,5-Di(n-tetradecyloxy)phenethyl, 3,5-Di(n-pentadecyloxy)phenethyl,
3,5-Di(n-hexadecyoloxy)phenethyl, 3,5-Di(n-heptadecyloxy)phenethyl,
3,5-Di(n-octadecyloxy)phenethyl, 3,5-Di(nonadecyloxy)phenethyl,
3,5-Di(eicosyloxy)phenethyl, 3,5-Di(docosanyloxy)phenethyl,
3,5-Di(tricosanyloxy)phenethyl, 3,5-Di(tetracosanyloxy)phenethyl,
3,5-Di(octacosanyloxy)phenethyl;

3,5-Di(n-butylthio)phenethyl, 3,5-Di(n-pentylthio)phenethyl,
3,5-Di(n-hexylthio)phenethyl, 3,,5-Di(n-heptylthio)phenethyl,
3,5-Di(n-octylthio)phenethyl, 3,5-Di(n-nonylthio)phenethyl,
3,5-Dii(n-decylthio)phenethyl, 3,5-Di(n-undecythio)phenethyl,
3,5-Di(n-dodecylthio)phenethyl, 3,5-Di(n-tridecylthio)phenethyl,
3,5-Di(n-tetradecylthio)phenethyl, 3,5-Di(n-pentadecylthio)phenethyl,
3,5-Di(n-hexadecyolthio)phenethyl, 3,5-Di(n-heptadecylthio)phenethyl,
3,5-Di(n-octadecylthio)phenethyl, 3,5-Di(nonadecylthio)phenethyl,
3,5-Di(eicosylthio)phenethyl, 3,5-Di(docosanylthio)phenethyl,
3,5-Di(tricosanylthio)phenethyl, 3,5-Di(tetracosanylthio)phenethyl,
3,5-Di(octacosanylthio)phenethyl;
in denen q für 3 steht, wie z. B.
4-(3,5-Di(n-butyl)phenyl)propyl, 4-(3,5-Di(n-pentyl)phenyl)propyl,
4-(3,5-Di(n-hexyl)phenyl)propyl, 4-(3,5-Di(n-heptyl)phenyl)propyl,
4-(3,5-Di(n-octyl)phenyl)propyl, 4-(3,5-Di(n-nonyl)phenyl)propyl,
4-(3,5-Dii(n-decyl)phenyl)propyl, 4-(3,5-Di(n-undecy)phenyl)propyl,
4-(3,5-Di(n-dodecyl)phenyl)propyl, 4-(3,5-Di(n-tridecyl)phenyl)propyl,
4-(3,5-Di(n-tetradecyl)phenyl)propyl, 4-(3,5-Di(n-pentadecyl)phenyl)propyl,
4-(3,5-Di(n-hexadecyl)phenyl)propyl, 4-(3,5-Di(n-heptadecyl)phenyl)propyl,
4-(3,5-Di(n-octadecyl)phenyl)propyl, 4-(3,5-Di(nonadecyl)phenyl)propyl,
4-(3,5-Di(eicosyl)phenyl)propyl, 4-(3,5-Di(docosanyl)phenyl)propyl,
4-(3,5-Di(tricosanyl)phenyl)propyl, 4-(3,5-Di(tetracosanyl)phenyl)propyl,
4-(3,5-Di(octacosanyl)phenyl)propyl;

4-(3,5-Di(n-butyloxy)phenyl)propyl, 4-(3,5-Di(n-pentyloxy)phenyl)propyl,
4-(3,5-Di(n-hexyloxy)phenyl)propyl, 4-(3,5-Di(n-heptyloxy)phenyl)propyl,
4-(3,5-Di(n-octyloxy)phenyl)propyl, 4-(3,5-Di(n-nonyloxy)phenyl)propyl,
4-(3,5-Dii(n-decyloxy)phenyl)propyl, 4-(3,5-Di(n-undecyoxy)phenyl)propyl,
4-(3,5-Di(n-dodecyloxy)phenyl)propyl, 4-(3,5-Di(n-tridecyloxy)phenyl)propyl,
4-(3,5-Di(n-tetradecyloxy)phenyl)propyl, 4-(3,5-Di(n-pentadecyloxy)phenyl)propyl,
4-(3,5-Di(n-hexadecyoloxy)phenyl)propyl, 4-(3,5-Di(n-heptadecyloxy)phenyl)propyl,
4-(3,5-Di(n-octadecyloxy)phenyl)propyl, 4-(3,5-Di(nonadecyloxy)phenyl)propyl,
4-(3,5-Di(eicosyloxy)phenyl)propyl, 4-(3,5-Di(docosanyloxy)phenyl)propyl,
4-(3,5-Di(tricosanyloxy)phenyl)propyl, 4-(3,5-Di(tetracosanyloxy)phenyl)propyl,
4-(3,5-Di(octacosanyloxy)phenyl)propyl;

4-(3,5-Di(n-butylthio)phenyl)propyl, 4-(3,5-Di(n-pentylthio)phenyl)propyl,
4-(3,5-Di(n-hexylthio)phenyl)propyl, 4-(3,5-Di(n-heptylthio)phenyl)propyl,
4-(3,5-Di(n-octylthio)phenyl)propyl, 4-(3,5-Di(n-nonylthio)phenyl)propyl,
4-(3,5-Dii(n-decylthio)phenyl)propyl, 4-(3,5-Di(n-undecythio)phenyl)propyl,
4-(3,5-Di(n-dodecylthio)phenyl)propyl, 4-(3,5-Di(n-tridecylthio)phenyl)propyl,
4-(3,5-Di(n-tetradecylthio)phenyl)propyl, 4-(3,5-Di(n-pentadecylthio)phenyl)propyl,
4-(3,5-Di(n-hexadecyolthio)phenyl)propyl, 4-(3,5-Di(n-heptadecylthio)phenyl)propyl,
4-(3,5-Di(n-octadecylthio)phenyl)propyl, 4-(3,5-Di(nonadecylthio)phenyl)propyl,
4-(3,5-Di(eicosylthio)phenyl)propyl, 4-(3,5-Di(docosanylthio)phenyl)propyl,
4-(3,5-Di(tricosanylthio)phenyl)propyl, 4-(3,5-Di(tetracosanylthio)phenyl)propyl,
4-(3,5-Di(octacosanylthio)phenyl)propyl; und
in denen q für 4 steht, wie z. B.
4-(3,5-Di(n-butyl)phenyl)butyl, 4-(3,5-Di(n-pentyl)phenyl)butyl,
4-(3,5-Di(n-hexyl)phenyl)butyl, 4-(3,5-Di(n-heptyl)phenyl)butyl,
4-(3,5-Di(n-octyl)phenyl)butyl, 4-(3,5-Di(n-nonyl)phenyl)butyl,
4-(3,5-Dii(n-decyl)phenyl)butyl, 4-(3,5-Di(n-undecy)phenyl)butyl,
4-(3,5-Di(n-dodecyl)phenyl)butyl, 4-(3,5-Di(n-tridecyl)phenyl)butyl,
4-(3,5-Di(n-tetradecyl)phenyl)butyl, 4-(3,5-Di(n-pentadecyl)phenyl)butyl,
4-(3,5-Di(n-hexadecyl)phenyl)butyl, 4-(3,5-Di(n-heptadecyl)phenyl)butyl,
4-(3,5-Di(n-octadecyl)phenyl)butyl, 4-(3,5-Di(nonadecyl)phenyl)butyl,
4-(3,5-Di(eicosyl)phenyl)butyl, 4-(3,5-Di(docosanyl)phenyl)butyl,
4-(3,5-Di(tricosanyl)phenyl)butyl, 4-(3,5-Di(tetracosanyl)phenyl)butyl,
4-(3,5-Di(octacosanyl)phenyl)butyl;

4-(3,5-Di(n-butyloxy)phenyl)butyl, 4-(3,5-Di(n-pentyloxy)phenyl)butyl,
4-(3,5-Di(n-hexyloxy)phenyl)butyl, 4-(3,5-Di(n-heptyloxy)phenyl)butyl,
4-(3,5-Di(n-octyloxy)phenyl)butyl, 4-(3,5-Di(n-nonyloxy)phenyl)butyl,
4-(3,5-Dii(n-decyloxy)phenyl)butyl, 4-(3,5-Di(n-undecyoxy)phenyl)butyl,
4-(3,5-Di(n-dodecyloxy)phenyl)butyl, 4-(3,5-Di(n-tridecyloxy)phenyl)butyl,
4-(3,5-Di(n-tetradecyloxy)phenyl)butyl, 4-(3,5-Di(n-pentadecyloxy)phenyl)butyl,
4-(3,5-Di(n-hexadecyoloxy)phenyl)butyl, 4-(3,5-Di(n-heptadecyloxy)phenyl)butyl,
4-(3,5-Di(n-octadecyloxy)phenyl)butyl, 4-(3,5-Di(nonadecyloxy)phenyl)butyl,
4-(3,5-Di(eicosyloxy)phenyl)butyl, 4-(3,5-Di(docosanyloxy)phenyl)butyl,
4-(3,5-Di(tricosanyloxy)phenyl)butyl, 4-(3,5-Di(tetracosanyloxy)phenyl)butyl,
4-(3,5-Di(octacosanyloxy)phenyl)butyl;

4-(3,5-Di(n-butylthio)phenyl)butyl, 4-(3,5-Di(n-pentylthio)phenyl)butyl,
4-(3,5-Di(n-hexylthio)phenyl)butyl, 4-(3,5-Di(n-heptylthio)phenyl)butyl,
4-(3,5-Di(n-octylthio)phenyl)butyl, 4-(3,5-Di(n-nonylthio)phenyl)butyl,
4-(3,5-Dii(n-decylthio)phenyl)butyl, 4-(3,5-Di(n-undecythio)phenyl)butyl,
4-(3,5-Di(n-dodecylthio)phenyl)butyl, 4-(3,5-Di(n-tridecylthio)phenyl)butyl,
4-(3,5-Di(n-tetradecylthio)phenyl)butyl, 4-(3,5-Di(n-pentadecylthio)phenyl)butyl,
4-(3,5-Di(n-hexadecyolthio)phenyl)butyl, 4-(3,5-Di(n-heptadecylthio)phenyl)butyl,
4-(3,5-Di(n-octadecylthio)phenyl)butyl, 4-(3,5-Di(nonadecylthio)phenyl)butyl,
4-(3,5-Di(eicosylthio)phenyl)butyl, 4-(3,5-Di(docosanylthio)phenyl)butyl,
4-(3,5-Di(tricosanylthio)phenyl)butyl, 4-(3,5-Di(tetracosanylthio)phenyl)butyl,
4-(3,5-Di(octacosanylthio)phenyl)butyl;
des Weiteren Reste der Formel C 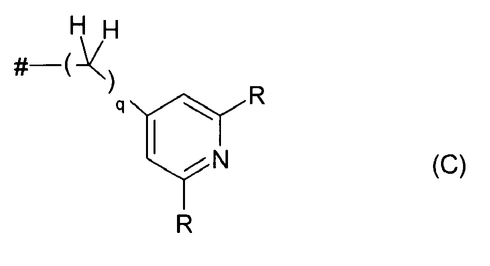 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Reste der Formel C umfassen solche, in denen q für 0 steht, wie z. B.
2,6-Di(n-butyl)-pyridin-4-yl, 2,6-Di(n-pentyl)-pyridin-4-yl, 2,6-Di(n-hexyl)-pyridin-4-yl,
2,6-Di(n-heptyl)-pyridin-4-yl, 2,6-Di(n-octyl)-pyridin-4-yl, 2,6-Di(n-nonyl)-pyridin-4-yl,
2,6-Dii(n-decyl)-pyridin-4-yl, 2,6-Di(n-undecy)-pyridin-4-yl,
2,6-Di(n-dodecyl)-pyridin-4-yl, 2,6-Di(n-tridecyl)-pyridin-4-yl,
2,6-Di(n-tetradecyl)-pyridin-4-yl, 2,6-Di(n-pentadecyl)-pyridin-4-yl,
2,6-Di(n-hexadecyl)-pyridin-4-yl, 2,6-Di(n-heptadecyl)-pyridin-4-yl,
2,6-Di(n-octadecyl)-pyridin-4-yl, 2,6-Di(nonadecyl)-pyridin-4-yl,
2,6-Di(eicosyl)-pyridin-4-yl, 2,6-Di(docosanyl)-pyridin-4-yl,
2,6-Di(tricosanyl)-pyridin-4-yl, 2,6-Di(tetracosanyl)-pyridin-4-yl,
2,6-Di(octacosanyl)-pyridin-4-yl;
in denen q für 1 steht, wie z. B.
2,6-Di(n-butyl)-pyridin-4-yl-methyl, 2,6-Di(n-pentyl)-pyridin-4-yl-methyl,
2,6-Di(n-hexyl)-pyridin-4-yl-methyl, 2,6-Di(n-heptyl)-pyridin-4-yl-methyl,
2,6-Di(n-octyl)-pyridin-4-yl-methyl, 2,6-Di(n-nonyl)-pyridin-4-yl-methyl,
2,6-Dii(n-decyl)-pyridin-4-yl-methyl, 2,6-Di(n-undecy)-pyridin-4-yl-methyl,
2,6-Di(n-dodecyl)-pyridin-4-yl-methyl, 2,6-Di(n-tridecyl)-pyridin-4-yl-methyl,
2,6-Di(n-tetradecyl)-pyridin-4-yl-methyl, 2,6-Di(n-pentadecyl)-pyridin-4-yl-methyl,
2,6-Di(n-hexadecyl)-pyridin-4-yl-methyl, 2,6-Di(n-heptadecyl)-pyridin-4-yl-methyl,
2,6-Di(n-octadecyl)-pyridin-4-yl-methyl, 2,6-Di(nonadecyl)-pyridin-4-yl-methyl,
2,6-Di(eicosyl)-pyridin-4-yl-methyl, 2,6-Di(docosanyl)-pyridin-4-yl-methyl,
2,6-Di(tricosanyl)-pyridin-4-yl-methyl, 2,6-Di(tetracosanyl)-pyridin-4-yl-methyl,
2,6-Di(octacosanyl)-pyridin-4-yl-methyl;
in denen q für 2 steht, wie z. B.
2,6-Di(n-butyl)-pyridin-4-yl-ethyl, 2,6-Di(n-pentyl)-pyridin-4-yl-ethyl,
2,6-Di(n-hexyl)-pyridin-4-yl-ethyl, 2,6-Di(n-heptyl)-pyridin-4-yl-ethyl,
2,6-Di(n-octyl)-pyridin-4-yl-ethyl, 2,6-Di(n-nonyl)-pyridin-4-yl-ethyl,
2,6-Dii(n-decyl)-pyridin-4-yl-ethyl, 2,6-Di(n-undecy)-pyridin-4-yl-ethyl,
2,6-Di(n-dodecyl)-pyridin-4-yl-ethyl, 2,6-Di(n-tridecyl)-pyridin-4-yl-ethyl,
2,6-Di(n-tetradecyl)-pyridin-4-yl-ethyl, 2,6-Di(n-pentadecyl)-pyridin-4-yl-ethyl,
2,6-Di(n-hexadecyl)-pyridin-4-yl-ethyl, 2,6-Di(n-heptadecyl)-pyridin-4-yl-ethyl,
2,6-Di(n-octadecyl)-pyridin-4-yl-ethyl, 2,6-Di(nonadecyl)-pyridin-4-yl-ethyl,
2,6-Di(eicosyl)-pyridin-4-yl-ethyl, 2,6-Di(docosanyl)-pyridin-4-yl-ethyl,
2,6-Di(tricosanyl)-pyridin-4-yl-ethyl, 2,6-Di(tetracosanyl)-pyridin-4-yl-ethyl,
2,6-Di(octacosanyl)-pyridin-4-yl-ethyl;
in denen q für 3 steht, wie z. B.
3-(2,6-Di(n-butyl)-pyridin-4-yl)-propyl, 3-(2,6-Di(n-pentyl)-pyridin-4-yl)-propyl,
3-(2,6-Di(n-hexyl)-pyridin-4-yl)-propyl, 3-(2,6-Di(n-heptyl)-pyridin-4-yl)-propyl,
3-(2,6-Di(n-octyl)-pyridin-4-yl)-propyl, 3-(2,6-Di(n-nonyl)-pyridin-4-yl)-propyl,
3-(2,6-Dii(n-decyl)-pyridin-4-yl)-propyl, 3-(2,6-Di(n-undecy)-pyridin-4-yl)-propyl,
3-(2,6-Di(n-dodecyl)-pyridin-4-yl)-propyl, 3-(2,6-Di(n-tridecyl)-pyridin-4-yl)-propyl,
3-(2,6-Di(n-tetradecyl)-pyridin-4-yl)-propyl, 3-(2,6-Di(n-pentadecyl)-pyridin-4-yl)-propyl,
3-(2,6-Di(n-hexadecyl)-pyridin-4-yl)-propyl, 3-(2,6-Di(n-heptadecyl)-pyridin-4-yl)-propyl,
3-(2,6-Di(n-octadecyl)-pyridin-4-yl)-propyl, 3-(2,6-Di(nonadecyl)-pyridin-4-yl)-propyl,
3-(2,6-Di(eicosyl)-pyridin-4-yl)-propyl, 3-(2,6-Di(docosanyl)-pyridin-4-yl)-propyl,
3-(2,6-Di(tricosanyl)-pyridin-4-yl)-propyl, 3-(2,6-Di(tetracosanyl)-pyridin-4-yl)-propyl,
3-(2,6-Di(octacosanyl)-pyridin-4-yl)-propyl;
in denen q für 4 steht, wie z. B.
4-(2,6-Di(n-butyl)-pyridin-4-yl)-butyl, 4-(2,6-Di(n-pentyl)-pyridin-4-yl)-butyl,
4-(2,6-Di(n-hexyl)-pyridin-4-yl)-butyl, 4-(2,6-Di(n-heptyl)-pyridin-4-yl)-butyl,
4-(2,6-Di(n-octyl)-pyridin-4-yl)-butyl, 4-(2,6-Di(n-nonyl)-pyridin-4-yl)-butyl,
4-(2,6-Dii(n-decyl)-pyridin-4-yl)-butyl, 4-(2,6-Di(n-undecy)-pyridin-4-yl)-butyl,
4-(2,6-Di(n-dodecyl)-pyridin-4-yl)-butyl, 4-(2,6-Di(n-tridecyl)-pyridin-4-yl)-butyl,
4-(2,6-Di(n-tetradecyl)-pyridin-4-yl)-butyl, 4-(2,6-Di(n-pentadecyl)-pyridin-4-yl)-butyl,
4-(2,6-Di(n-hexadecyl)-pyridin-4-yl)-butyl, 4-(2,6-Di(n-heptadecyl)-pyridin-4-yl)-butyl,
4-(2,6-Di(n-octadecyl)-pyridin-4-yl)-butyl, 4-(2,6-Di(nonadecyl)-pyridin-4-yl)-butyl,
4-(2,6-Di(eicosyl)-pyridin-4-yl)-butyl, 4-(2,6-Di(docosanyl)-pyridin-4-yl)-butyl,
4-(2,6-Di(tricosanyl)-pyridin-4-yl)-butyl, 4-(2,6-Di(tetracosanyl)-pyridin-4-yl)-butyl,
4-(2,6-Di(octacosanyl)-pyridin-4-yl)-butyl;
des Weiteren Reste der Formel D 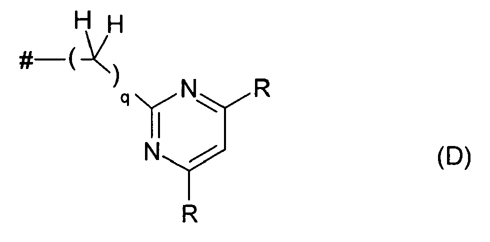 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Reste der Formel D umfassen solche, in denen q für 0 steht, wie z. B.
4,6-Di(n-butyl)-pyrimidin-2-yl, 4,6-Di(n-pentyl)-pyrimidin-2-yl,
4,6-Di(n-hexyl)-pyrimidin-2-yl, 4,6-Di(n-heptyl)-pyrimidin-2-yl,
4,6-Di(n-octyl)-pyrimidin-2-yl, 4,6-Di(n-nonyl)-pyrimidin-2-yl,
4,6-Dii(n-decyl)-pyrimidin-2-yl, 4,6-Di(n-undecy)-pyrimidin-2-yl,
4,6-Di(n-dodecyl)-pyrimidin-2-yl, 4,6-Di(n-tridecyl)-pyrimidin-2-yl,
4,6-Di(n-tetradecyl)-pyrimidin-2-yl, 4,6-Di(n-pentadecyl)-pyrimidin-2-yl,
4,6-Di(n-hexadecyl)-pyrimidin-2-yl, 4,6-Di(n-heptadecyl)-pyrimidin-2-yl,
4,6-Di(n-octadecyl)-pyrimidin-2-yl, 4,6-Di(nonadecyl)-pyrimidin-2-yl,
4,6-Di(eicosyl)-pyrimidin-2-yl, 4,6-Di(docosanyl)-pyrimidin-2-yl,
4,6-Di(tricosanyl)-pyrimidin-2-yl, 4,6-Di(tetracosanyl)-pyrimidin-2-yl,
4,6-Di(octacosanyl)-pyrimidin-2-yl;
in denen q für 1 steht, wie z. B.
4,6-Di(n-butyl)-pyrimidin-2-yl-methyl, 4,6-Di(n-pentyl)-pyrimidin-2-yl-methyl,
4,6-Di(n-hexyl)-pyrimidin-2-yl-methyl, 4,6-Di(n-heptyl)-pyrimidin-2-yl-methyl,
4,6-Di(n-octyl)-pyrimidin-2-yl-methyl, 4,6-Di(n-nonyl)-pyrimidin-2-yl-methyl,
4,6-Dii(n-decyl)-pyrimidin-2-yl-methyl, 4,6-Di(n-undecy)-pyrimidin-2-yl-methyl,
4,6-Di(n-dodecyl)-pyrimidin-2-yl-methyl, 4,6-Di(n-tridecyl)-pyrimidin-2-yl-methyl,
4,6-Di(n-tetradecyl)-pyrimidin-2-yl-methyl, 4,6-Di(n-pentadecyl)-pyrimidin-2-yl-methyl,
4,6-Di(n-hexadecyl)-pyrimidin-2-yl-methyl, 4,6-Di(n-heptadecyl)-pyrimidin-2-yl-methyl,
4,6-Di(n-octadecyl)-pyrimidin-2-yl-methyl, 4,6-Di(nonadecyl)-pyrimidin-2-yl-methyl,
4,6-Di(eicosyl)-pyrimidin-2-yl-methyl, 4,6-Di(docosanyl)-pyrimidin-2-yl-methyl,
4,6-Di(tricosanyl)-pyrimidin-2-yl-methyl, 4,6-Di(tetracosanyl)-pyrimidin-2-yl-methyl,
4,6-Di(octacosanyl)-pyrimidin-2-yl-methyl;
in denen q für 2 steht, wie z. B.
4,6-Di(n-butyl)-pyrimidin-2-yl-ethyl, 4,6-Di(n-pentyl)-pyrimidin-2-yl-ethyl,
4,6-Di(n-hexyl)-pyrimidin-2-yl-ethyl, 4,6-Di(n-heptyl)-pyrimidin-2-yl-ethyl,
4,6-Di(n-octyl)-pyrimidin-2-yl-ethyl, 4,6-Di(n-nonyl)-pyrimidin-2-yl-ethyl,
4,6-Dii(n-decyl)-pyrimidin-2-yl-ethyl, 4,6-Di(n-undecy)-pyrimidin-2-yl-ethyl,
4,6-Di(n-dodecyl)-pyrimidin-2-yl-ethyl, 4,6-Di(n-tridecyl)-pyrimidin-2-yl-ethyl,
4,6-Di(n-tetradecyl)-pyrimidin-2-yl-ethyl, 4,6-Di(n-pentadecyl)-pyrimidin-2-yl-ethyl,
4,6-Di(n-hexadecyl)-pyrimidin-2-yl-ethyl, 4,6-Di(n-heptadecyl)-pyrimidin-2-yl-ethyl,
4,6-Di(n-octadecyl)-pyrimidin-2-yl-ethyl, 4,6-Di(nonadecyl)-pyrimidin-2-yl-ethyl,
4,6-Di(eicosyl)-pyrimidin-2-yl-ethyl, 4,6-Di(docosanyl)-pyrimidin-2-yl-ethyl,
4,6-Di(tricosanyl)-pyrimidin-2-yl-ethyl, 4,6-Di(tetracosanyl)-pyrimidin-2-yl-ethyl,
4,6-Di(octacosanyl)-pyrimidin-2-yl-ethyl;
in denen q für 3 steht, wie z. B.
3-(4,6-Di(n-butyl)-pyrimidin-2-yl)-propyl, 3-(4,6-Di(n-pentyl)-pyrimidin-2-yl)-propyl,
3-(4,6-Di(n-hexyl)-pyrimidin-2-yl)-propyl, 3-(4,6-Di(n-heptyl)-pyrimidin-2-yl)-propyl,
3-(4,6-Di(n-octyl)-pyrimidin-2-yl)-propyl, 3-(4,6-Di(n-nonyl)-pyrimidin-2-yl)-propyl,
3-(4,6-Dii(n-decyl)-pyrimidin-2-yl)-propyl, 3-(4,6-Di(n-undecy)-pyrimidin-2-yl)-propyl,
3-(4,6-Di(n-dodecyl)-pyrimidin-2-yl)-propyl, 3-(4,6-Di(n-tridecyl)-pyrimidin-2-yl)-propyl,
3-(4,6-Di(n-tetradecyl)-pyrimidin-2-yl)-propyl,
3-(4,6-Di(n-pentadecyl)-pyrimidin-2-yl)-propyl,
3-(4,6-Di(n-hexadecyl)-pyrimidin-2-yl)-propyl,
3-(4,6-Di(n-heptadecyl)-pyrimidin-2-yl)-propyl,
3-(4,6-Di(n-octadecyl)-pyrimidin-2-yl)-propyl,
3-(4,6-Di(nonadecyl)-pyrimidin-2-yl)-propyl, 3-(4,6-Di(eicosyl)-pyrimidin-2-yl)-propyl,
3-(4,6-Di(docosanyl)-pyrimidin-2-yl)-propyl, 3-(4,6-Di(tricosanyl)-pyrimidin-2-yl)-propyl,
3-(4,6-Di(tetracosanyl)-pyrimidin-2-yl)-propyl,
3-(4,6-Di(octacosanyl)-pyrimidin-2-yl)-propyl;
in denen q für 4 steht, wie z. B.
4-(4,6-Di(n-butyl)-pyrimidin-2-yl)-butyl, 4-(4,6-Di(n-pentyl)-pyrimidin-2-yl)-butyl,
4-(4,6-Di(n-hexyl)-pyrimidin-2-yl)-butyl, 4-(4,6-Di(n-heptyl)-pyrimidin-2-yl)-butyl,
4-(4,6-Di(n-octyl)-pyrimidin-2-yl)-butyl, 4-(4,6-Di(n-nonyl)-pyrimidin-2-yl)-butyl,
4-(4,6-Dii(n-decyl)-pyrimidin-2-yl)-butyl, 4-(4,6-Di(n-undecy)-pyrimidin-2-yl)-butyl,
4-(4,6-Di(n-dodecyl)-pyrimidin-2-yl)-butyl, 4-(4,6-Di(n-tridecyl)-pyrimidin-2-yl)-butyl,
4-(4,6-Di(n-tetradecyl)-pyrimidin-2-yl)-butyl,
4-(4,6-Di(n-pentadecyl)-pyrimidin-2-yl)-butyl,
4-(4,6-Di(n-hexadecyl)-pyrimidin-2-yl)-butyl,
4-(4,6-Di(n-heptadecyl)-pyrimidin-2-yl)-butyl,
4-(4,6-Di(n-octadecyl)-pyrimidin-2-yl)-butyl, 4-(4,6-Di(nonadecyl)-pyrimidin-2-yl)-butyl,
4-(4,6-Di(eicosyl)-pyrimidin-2-yl)-butyl, 4-(4,6-Di(docosanyl)-pyrimidin-2-yl)-butyl,
4-(4,6-Di(tricosanyl)-pyrimidin-2-yl)-butyl, 4-(4,6-Di(tetracosanyl)-pyrimidin-2-yl)-butyl,
4-(4,6-Di(octacosanyl)-pyrimidin-2-yl)-butyl;
des Weiteren Reste der Formel E 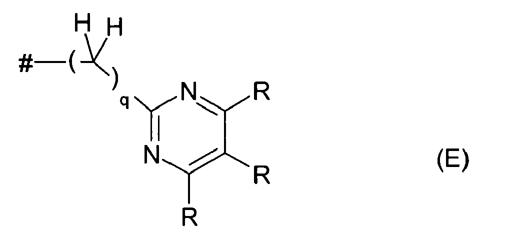 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Reste der Formel F umfassen solche, in denen q für 0 steht, wie z. B.
4,5,6-Tri(n-butyl)-pyrimidin-2-yl, 4,5,6-Tri(n-pentyl)-pyrimidin-2-yl,
4,5,6-Tri(n-hexyl)-pyrimidin-2-yl, 4,5,6-Tri(n-heptyl)-pyrimidin-2-yl,
4,5,6-Tri(n-octyl)-pyrimidin-2-yl, 4,5,6-Tri(n-nonyl)-pyrimidin-2-yl,
4,5,6-Trii(n-decyl)-pyrimidin-2-yl, 4,5,6-Tri(n-undecy)-pyrimidin-2-yl,
4,5,6-Tri(n-dodecyl)-pyrimidin-2-yl, 4,5,6-Tri(n-tridecyl)-pyrimidin-2-yl,
4,5,6-Tri(n-tetradecyl)-pyrimidin-2-yl, 4,5,6-Tri(n-pentadecyl)-pyrimidin-2-yl,
4,5,6-Tri(n-hexadecyl)-pyrimidin-2-yl, 4,5,6-Tri(n-heptadecyl)-pyrimidin-2-yl,
4,5,6-Tri(n-octadecyl)-pyrimidin-2-yl, 4,5,6-Tri(nonadecyl)-pyrimidin-2-yl,
4,5,6-Tri(eicosyl)-pyrimidin-2-yl, 4,5,6-Tri(docosanyl)-pyrimidin-2-yl,
4,5,6-Tri(tricosanyl)-pyrimidin-2-yl, 4,5,6-Tri(tetracosanyl)-pyrimidin-2-yl,
4,5,6-Tri(octacosanyl)-pyrimidin-2-yl;
in denen q für 1 steht, wie z. B.
4,5,6-Tri(n-butyl)-pyrimidin-2-yl-methyl, 4,5,6-Tri(n-pentyl)-pyrimidin-2-yl-methyl,
4,5,6-Tri(n-hexyl)-pyrimidin-2-yl-methyl, 4,5,6-Tri(n-heptyl)-pyrimidin-2-yl-methyl,
4,5,6-Tri(n-octyl)-pyrimidin-2-yl-methyl, 4,5,6-Tri(n-nonyl)-pyrimidin-2-yl-methyl,
4,5,6-Trii(n-decyl)-pyrimidin-2-yl-methyl, 4,5,6-Tri(n-undecy)-pyrimidin-2-yl-methyl,
4,5,6-Tri(n-dodecyl)-pyrimidin-2-yl-methyl, 4,5,6-Tri(n-tridecyl)-pyrimidin-2-yl-methyl,
4,5,6-Tri(n-tetradecyl)-pyrimidin-2-yl-methyl,
4,5,6-Tri(n-pentadecyl)-pyrimidin-2-yl-methyl,
4,5,6-Tri(n-hexadecyl)-pyrimidin-2-yl-methyl,
4,5,6-Tri(n-heptadecyl)-pyrimidin-2-yl-methyl,
4,5,6-Tri(n-octadecyl)-pyrimidin-2-yl-methyl, 4,5,6-Tri(nonadecyl)-pyrimidin-2-yl-methyl,
4,5,6-Tri(eicosyl)-pyrimidin-2-yl-methyl, 4,5,6-Tri(docosanyl)-pyrimidin-2-yl-methyl,
4,5,6-Tri(tricosanyl)-pyrimidin-2-yl-methyl, 4,5,6-Tri(tetracosanyl)-pyrimidin-2-yl-methyl,
4,5,6-Tri(octacosanyl)-pyrimidin-2-yl-methyl;
in denen q für 2 steht, wie z. B.
4,5,6-Tri(n-butyl)-pyrimidin-2-yl-ethyl, 4,5,6-Tri(n-pentyl)-pyrimidin-2-yl-ethyl,
4,5,6-Tri(n-hexyl)-pyrimidin-2-yl-ethyl, 4,5,6-Tri(n-heptyl)-pyrimidin-2-yl-ethyl,
4,5,6-Tri(n-octyl)-pyrimidin-2-yl-ethyl, 4,5,6-Tri(n-nonyl)-pyrimidin-2-yl-ethyl,
4,5,6-Trii(n-decyl)-pyrimidin-2-yl-ethyl, 4,5,6-Tri(n-undecy)-pyrimidin-2-yl-ethyl,
4,5,6-Tri(n-dodecyl)-pyrimidin-2-yl-ethyl, 4,5,6-Tri(n-tridecyl)-pyrimidin-2-yl-ethyl,
4,5,6-Tri(n-tetradecyl)-pyrimidin-2-yl-ethyl, 4,5,6-Tri(n-pentadecyl)-pyrimidin-2-yl-ethyl,
4,5,6-Tri(n-hexadecyl)-pyrimidin-2-yl-ethyl, 4,5,6-Tri(n-heptadecyl)-pyrimidin-2-yl-ethyl,
4,5,6-Tri(n-octadecyl)-pyrimidin-2-yl-ethyl, 4,5,6-Tri(nonadecyl)-pyrimidin-2-yl-ethyl,
4,5,6-Tri(eicosyl)-pyrimidin-2-yl-ethyl, 4,5,6-Tri(docosanyl)-pyrimidin-2-yl-ethyl,
4,5,6-Tri(tricosanyl)-pyrimidin-2-yl-ethyl, 4,5,6-Tri(tetracosanyl)-pyrimidin-2-yl-ethyl,
4,5,6-Tri(octacosanyl)-pyrimidin-2-yl-ethyl;
in denen q für 3 steht, wie z. B.
3-(4,5,6-Tri(n-butyl)-pyrimidin-2-yl)-propyl, 3-(4,5,6-Tri(n-pentyl)-pyrimidin-2-yl)-propyl,
3-(4,5,6-Tri(n-hexyl)-pyrimidin-2-yl)-propyl, 3-(4,5,6-Tri(n-heptyl)-pyrimidin-2-yl)-propyl,
3-(4,5,6-Tri(n-octyl)-pyrimidin-2-yl)-propyl, 3-(4,5,6-Tri(n-nonyl)-pyrimidin-2-yl)-propyl,
3-(4,5,6-Trii(n-decyl)-pyrimidin-2-yl)-propyl,
3-(4,5,6-Tri(n-undecy)-pyrimidin-2-yl)-propyl,
3-(4,5,6-Tri(n-dodecyl)-pyrimidin-2-yl)-propyl,
3-(4,5,6-Tri(n-tridecyl)-pyrimidin-2-yl)-propyl,
3-(4,5,6-Tri(n-tetradecyl)-pyrimidin-2-yl)-propyl,
3-(4,5,6-Tri(n-pentadecyl)-pyrimidin-2-yl)-propyl,
3-(4,5,6-Tri(n-hexadecyl)-pyrimidin-2-yl)-propyl,
3-(4,5,6-Tri(n-heptadecyl)-pyrimidin-2-yl)-propyl,
3-(4,5,6-Tri(n-octadecyl)-pyrimidin-2-yl)-propyl,
3-(4,5,6-Tri(nonadecyl)-pyrimidin-2-yl)-propyl,
3-(4,5,6-Tri(eicosyl)-pyrimidin-2-yl)-propyl,
3-(4,5,6-Tri(docosanyl)-pyrimidin-2-yl)-propyl,
3-(4,5,6-Tri(tricosanyl)-pyrimidin-2-yl)-propyl,
3-(4,5,6-Tri(tetracosanyl)-pyrimidin-2-yl)-propyl,
3-(4,5,6-Tri(octacosanyl)-pyrimidin-2-yl)-propyl;
in denen q für 4 steht, wie z. B.
4-(4,5,6-Tri(n-butyl)-pyrimidin-2-yl)-butyl, 4-(4,5,6-Tri(n-pentyl)-pyrimidin-2-yl)-butyl,
4-(4,5,6-Tri(n-hexyl)-pyrimidin-2-yl)-butyl, 4-(4,5,6-Tri(n-heptyl)-pyrimidin-2-yl)-butyl,
4-(4,5,6-Tri(n-octyl)-pyrimidin-2-yl)-butyl, 4-(4,5,6-Tri(n-nonyl)-pyrimidin-2-yl)-butyl,
4-(4,5,6-Trii(n-decyl)-pyrimidin-2-yl)-butyl, 4-(4,5,6-Tri(n-undecy)-pyrimidin-2-yl)-butyl,
4-(4,5,6-Tri(n-dodecyl)-pyrimidin-2-yl)-butyl, 4-(4,5,6-Tri(n-tridecyl)-pyrimidin-2-yl)-butyl,
4-(4,5,6-Tri(n-tetradecyl)-pyrimidin-2-yl)-butyl,
4-(4,5,6-Tri(n-pentadecyl)-pyrimidin-2-yl)-butyl,
4-(4,5,6-Tri(n-hexadecyl)-pyrimidin-2-yl)-butyl,
4-(4,5,6-Tri(n-heptadecyl)-pyrimidin-2-yl)-butyl,
4-(4,5,6-Tri(n-octadecyl)-pyrimidin-2-yl)-butyl,
4-(4,5,6-Tri(nonadecyl)-pyrimidin-2-yl)-butyl, 4-(4,5,6-Tri(eicosyl)-pyrimidin-2-yl)-butyl,
4-(4,5,6-Tri(docosanyl)-pyrimidin-2-yl)-butyl,
4-(4,5,6-Tri(tricosanyl)-pyrimidin-2-yl)-butyl,
4-(4,5,6-Tri(tetracosanyl)-pyrimidin-2-yl)-butyl,
4-(4,5,6-Tri(octacosanyl)-pyrimidin-2-yl)-butyl;
des Weiteren Reste der Formel F 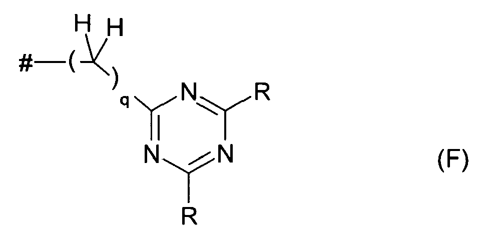 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Reste der Formel F umfassen solche, in denen q für 0 steht, wie z. B.
4,6-Di(n-butyl)-1,3,5-triazin-2-yl, 4,6-Di(n-pentyl)-1,3,5-triazin-2-yl,
4,6-Di(n-hexyl)-1,3,5-triazin-2-yl, 4,6-Di(n-heptyl)-1,3,5-triazin-2-yl,
4,6-Di(n-octyl)-1,3,5-triazin-2-yl, 4,6-Di(n-nonyl)-1,3,5-triazin-2-yl,
4,6-Dii(n-decyl)-1,3,5-triazin-2-yl, 4,6-Di(n-undecy)-1,3,5-triazin-2-yl,
4,6-Di(n-dodecyl)-1,3,5-triazin-2-yl, 4,6-Di(n-tridecyl)-1,3,5-triazin-2-yl,
4,6-Di(n-tetradecyl)-1,3,5-triazin-2-yl, 4,6-Di(n-pentadecyl)-1,3,5-triazin-2-yl,
4,6-Di(n-hexadecyl)-1,3,5-triazin-2-yl, 4,6-Di(n-heptadecyl)-1,3,5-triazin-2-yl,
4,6-Di(n-octadecyl)-1,3,5-triazin-2-yl, 4,6-Di(nonadecyl)-1,3,5-triazin-2-yl,
4,6-Di(eicosyl)-1,3,5-triazin-2-yl, 4,6-Di(docosanyl)-1,3,5-triazin-2-yl,
4,6-Di(tricosanyl)-1,3,5-triazin-2-yl, 4,6-Di(tetracosanyl)-1,3,5-triazin-2-yl,
4,6-Di(octacosanyl)-1,3,5-triazin-2-yl;
in denen q für 1 steht, wie z. B.
4,6-Di(n-butyl)-1,3,5-triazin-2-yl-methyl, 4,6-Di(n-pentyl)-1,3,5-triazin-2-yl-methyl,
4,6-Di(n-hexyl)-1,3,5-triazin-2-yl-methyl, 4,6-Di(n-heptyl)-1,3,5-triazin-2-yl-methyl,
4,6-Di(n-octyl)-1,3,5-triazin-2-yl-methyl, 4,6-Di(n-nonyl)-1,3,5-triazin-2-yl-methyl,
4,6-Dii(n-decyl)-1,3,5-triazin-2-yl-methy), 4,6-Di(n-undecy)-1,3,5-triazin-2-yl-methyl,
4,6-Di(n-dodecyl)-1,3,5-triazin-2-yl-methyl, 4,6-Di(n-tridecyl)-1,3,5-triazin-2-yl-methyl,
4,6-Di(n-tetradecyl)-1,3,5-triazin-2-yl-methyl,
4,6-Di(n-pentadecyl)-1,3,5-triazin-2-yl-methyl,
4,6-Di(n-hexadecyl)-1,3,5-triazin-2-yl-methyl,
4,6-Di(n-heptadecyl)-1,3,5-triazin-2-yl-methyl,
4,6-Di(n-octadecyl)-1,3,5-triazin-2-yl-methyl,
4,6-Di(nonadecyl)-1,3,5-triazin-2-yl-methyl, 4,6-Di(eicosyl)-1,3,5-triazin-2-yl-methyl,
4,6-Di(docosanyl)-1,3,5-triazin-2-yl-methyl, 4,6-Di(tricosanyl)=1,3,5-triazin-2-yl-methyl,
4,6-Di(tetracosanyl)-1,3,5-triazin-2-yl-methyl,
4,6-Di(octacosanyl)-1,3,5-triazin-2-yl-methyl;
in denen q für 2 steht, wie z. B.
4,6-Di(n-butyl)-1,3,5-triazin-2-yl-ethyl, 4,6-Di(n-pentyl)-1,3,5-triazin-2-yl-ethyl,
4,6-Di(n-hexyl)-1,3,5-triazin-2-yl-ethyl, 4,6-Di(n-heptyl)-1,3,5-triazin-2-yl-ethyl,
4,6-Di(n-octyl)-1,3,5-triazin-2-yl-ethyl, 4,6-Di(n-nonyl)-1,3,5-triazin-2-yl-ethyl,
4,6-Dii(n-decyl)-1,3,5-triazin-2-yl-ethyl, 4,6-Di(n-undecy)-1,3,5-triazin-2-yl-ethyl,
4,6-Di(n-dodecyl)-1,3,5-triazin-2-yl-ethyl, 4,6-Di(n-tridecyl)-1,3,5-triazin-2-yl-ethyl,
4,6-Di(n-tetradecyl)-1,3,5-triazin-2-yl-ethyl, 4,6-Di(n-pentadecyl)-1,3,5-triazin-2-yl-ethyl,
4,6-Di(n-hexadecyl)-1,3,5-triazin-2-yl-ethyl, 4,6-Di(n-heptadecyl)-1,3,5-triazin-2-yl-ethyl,
4,6-Di(n-octadecyl)-1,3,5-triazin-2-yl-ethyl, 4,6-Di(nonadecyl)-1,3,5-triazin-2-yl-ethyl,
4,6-Di(eicosyl)-1,3,5-triazin-2-yl-ethyl, 4,6-Di(docosanyl)-1,3,5-triazin-2-yl-ethyl,
4,6-Di(tricosanyl)-1,3,5-triazin-2-yl-ethyl, 4,6-Di(tetracosanyl)-1,3,5-triazin-2-yl-ethyl,
4,6-Di(octacosanyl)-1,3,5-triazin-2-yl-ethyl;
in denen q für 3 steht, wie z. B.
3-(4,6-Di(n-butyl)-1,3,5-triazin-2-yl)-propyl, 3-(4,6-Di(n-pentyl)-1,3,5-triazin-2-yl)-propyl,
3-(4,6-Di(n-hexyl)-1,3,5-triazin-2-yl)-propyl, 3-(4,6-Di(n-heptyl)-1,3,5-triazin-2-yl)-propyl,
3-(4,6-Di(n-octyl)-1,3,5-triazin-2-yl)-propyl, 3-(4,6-Di(n-nonyl)-1,3,5-triazin-2-yl)-propyl,
3-(4,6-Dii(n-decyl)-1,3,5-triazin-2-yl)-propyl,
3-(4,6-Di(n-undecy)-1,3,5-triazin-2-yl)-propyl,
3-(4,6-Di(n-dodecyl)-1,3,5-triazin-2-yl)-propyl,
3-(4,6-Di(n-tridecyl)-1,3,5-triazin-2-yl)-propyl,
3-(4,6-Di(n-tetradecyl)-1,3,5-triazin-2-yl)-propyl,
3-(4,6-Di(n-pentadecyl)-1,3,5-triazin-2-yl)-propyl,
3-(4,6-Di(n-hexadecyl)-1,3,5-triazin-2-yl)-propyl,
3-(4,6-Di(n-heptadecyl)-1,3,5-triazin-2-yl)-propyl,
3-(4,6-Di(n-octadecyl)-1,3,5-triazin-2-yl)-propyl,
3-(4,6-Di(nonadecyl)-1,3,5-triazin-2-yl)-propyl,
3-(4,6-Di(eicosyl)-1,3,5-triazin-2-yl)-propyl,
3-(4,6-Di(docosanyl)-1,3,5-triazin-2-yl)-propyl,
3-(4,6-Di(tricosanyl)-1,3,5-triazin-2-yl)-propyl,
3-(4,6-Di(tetracosanyl)-1,3,5-triazin-2-y1)-propyl,
3-(4,6-Di(octacosanyl)-1,3,5-triazin-2-yl)-propyl;
in denen q für 4 steht, wie z. B.
4-(4,6-Di(n-butyl)-1,3,5-triazin-2-yl)-butyl, 4-(4,6-Di(n-pentyl)-1,3,5-triazin-2-yl)-butyl,
4-(4,6-Di(n-hexyl)-1,3,5-triazin-2-yl)-butyl, 4-(4,6-Di(n-heptyl)-1,3,5-triazin-2-yl)-butyl,
4-(4,6-Di(n-octyl)-1,3,5-triazin-2-yl)-butyl, 4-(4,6-Di(n-nonyl)-1,3,5-triazin-2-yl)-butyl,
4-(4,6-Dii(n-decyl)-1,3,5-triazin-2-yl)-butyl, 4-(4,6-Di(n-undecy)-1,3,5-triazin-2-yl)-butyl,
4-(4,6-Di(n-dodecyl)-1,3,5-triazin-2-yl)-butyl,
4-(4,6-Di(n-tridecyl)-1,3,5-triazin-2-yl)-butyl,
4-(4,6-Di(n-tetradecyl)-1,3,5-triazin-2-yl)-butyl,
4-(4,6-Di(n-pentadecyl)-1,3,5-triazin-2-yl)-butyl,
4-(4,6-Di(n-hexadecyl)-1,3,5-triazin-2-yl)-butyl,
4-(4,6-Di(n-heptadecyl)-1,3,5-triazin-2-yl)-butyl,
4-(4,6-Di(n-octadecyl)-1,3,5-triazin-2-yl)-butyl,
4-(4,6-Di(nonadecyl)-1,3,5-triazin-2-yl)-butyl, 4-(4,6-Di(eicosyl)-1,3,5-triazin-2-yl)-butyl,
4-(4,6-Di(docosanyl)-1,3,5-triazin-2-yl)-butyl,
4-(4,6-Di(tricosanyl)-1,3,5-triazin-2-yl)-butyl,
4-(4,6-Di(tetracosanyl)-1,3,5-triazin-2-yl)-butyl,
4-(4,6-Di(octacosanyl)-1,3,5-triazin-2-yl)-butyl;
des Weiteren Reste der Formel G 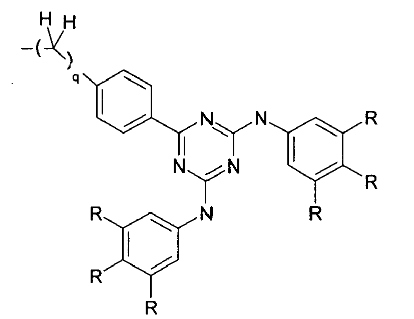 worin # die Verknüpfungsstelle mit dem Imidstickstoffatom des Rylentetracarbonsäurediimids ist, q für eine ganze Zahl 0, 1, 2, 3 oder 4 steht und R für C₄-C₃₀-Alkyl,
C₄-C₃₀-Alkylthio oder C₄-C₃₀-Alkoxy steht.

Reste der Formel G umfassen solche, in denen q für 0 steht, wie z. B.
4-[4,6-Di-(3,4,5-tri(n-butyl)phenylamino)-1,3,5-triazin-2-yl]-phenyl,
4-[4,6-Di-(3,4,5-tri(n-pentyl)phenylamino)-1,3,5-triazin-2-yl]-phenyl,
4-[4,6-Di-(3,4,5-tri(n-hexyl)phenylamino)-1,3,5-triazin-2-yl]-phenyl,
4-[4,6-Di-(3,4,5-tri(n-heptyl)phenylamino)-1,3,5-triazin-2-yl]-phenyl,
4-[4,6-Di-(3,4,5-tri(n-octyl)phenylamino)-1,3,5-triazin-2-yl]-phenyl,
4-[4,6-Di-(3,4,5-tri(n-nonyl)phenylamino)-1,3,5-triazin-2-yl]-phenyl,
4-[4,6-Di-(3,4,5-tri(n-decyl)phenylamino)-1,3,5-triazin-2-yl]-phenyl,
4-[4,6-Di-(3,4,5-tri(n-undecy)phenylamino)-1,3,5-triazin-2-yl]-phenyl,
4-[4,6-Di-(3,4,5-tri(n-dodecyl)phenylamino)-1,3,5-triazin-2-yl]-phenyl,
4-[4,6-Di-(3,4,5-tri(n-tridecyl)phenylamino)-1,3,5-triazin-2-yl]-phenyl,
4-[4,6-Di-(3,4,5-tri(n-tetradecyl)phenylamino)-1,3,5-triazin-2-yl]-phenyl,
4-[4,6-Di-(3,4,5-tri(n-pentadecyl)phenylamino)-1,3,5-triazin-2-yl]-phenyl,
4-[4,6-Di-(3,4,5-tri(n-hexadecyl)phenylamino)-1,3,5-triazin-2-yl]-phenyl,
4-[4,6-Di-(3,4,5-tri(n-heptadecyl)phenylamino)-1,3,5-triazin-2-yl]-phenyl,
4-[4,6-Di-(3,4,5-tri(n-octadecyl)phenylamino)-1,3,5-triazin-2-yl]-phenyl,
4-[4,6-Di-(3,4,5-tri(nonadecyl)phenylamino)-1,3,5-triazin-2-yl]-phenyl,
4-[4,6-Di-(3,4,5-tri(eicosyl)phenylamino)-1,3,5-triazin-2-yl]-phenyl,
4-[4,6-Di-(3,4,5-tri(docosanyl)phenylamino)-1,3,5-triazin-2-yl]-phenyl,
4-[4,6-Di-(3,4,5-tri(tricosanyl)phenylamino)-1,3,5-triazin-2-yl]-phenyl,
4-[4,6-Di-(3,4,5-tri(tetracosanyl)phenylamino)-1,3,5-triazin-2-yl]-phenyl,
4-[4,6-Di-(3,4,5-tri(octacosanyl)phenylamino)-1,3,5-triazin-2-yl]-phenyl;

4-[4,6-Di(3,4,5-tri(n-butyloxy)phenylamino)-1,3,5-triazin-2-yl]-phenyl,
4-[4,6-Di(3,4,5-tri(n-pentyloxy)phenylamino)-1,3,5-triazin-2-yl]-phenyl,
4-[4,6-Di(3,4,5-tri(n-hexyloxy)phenylamino)-1,3,5-triazin-2-yl]-phenyl,
4-[4,6-Di(3,4,5-tri(n-heptyloxy)phenylamino)-1,3,5-triazin-2-yl]-phenyl,
4-[4,6-Di(3,4,5-tri(n-octyloxy)phenylamino)-1,3,5-triazin-2-yl]-phenyl,
4-[4,6-Di(3,4,5-tri(n-nonyloxy)phenylamino)-1,3,5-triazin-2-yl]-phenyl,
4-[4,6-Di(3,4,5-tri(n-decyloxy)phenylamino)-1,3,5-triazin-2-yl]-phenyl,
4-[4,6-Di(3,4,5-tri(n-undecyloxy)phenylamino)-1,3,5-triazin-2-yl]-phenyl,
4-[4,6-Di(3,4,5-tri(n-dodecyloxy)phenylamino)-1,3,5-triazin-2-yl]-phenyl,
4-[4,6-Di(3,4,5-tri(n-tridecyloxy)phenylamino)-1,3,5-triazin-2-yl]-phenyl,
4-[4,6-Di(3,4,5-tri(n-tetradecyloxy)phenylamino)-1,3,5-triazin-2-yl]-phenyl,
4-[4,6-Di(3,4,5-tri(n-pentadecyloxy)phenylamino)-1,3,5-triazin-2-yl]-phenyl,
4-[4,6-Di(3,4,5-tri(n-hexadecyloxy)phenylamino)-1,3,5-triazin-2-yl]-phenyl,
4-[4,6-Di(3,4,5-tri(n-heptadecyloxy)phenylamino)-1,3,5-triazin-2-yl]-phenyl,
4-[4,6-Di(3,4,5-tri(n-octadecyloxy)phenylamino)-1,3,5-triazin-2-yl]-phenyl,
4-[4,6-Di(3,4,5-tri(nonadecyloxy)phenylamino)-1,3,5-triazin-2-yl]-phenyl,
4-[4,6-Di(3,4,5-tri(eicosyloxy)phenylamino)-1,3,5-triazin-2-yl]-phenyl,
4-[4,6-Di(3,4,5-tri(docosanyloxy)phenylamino)-1,3,5-triazin-2-yl]-phenyl,
4-[4,6-Di(3,4,5-tri(tricosanyloxy)phenylamino)-1,3,5-triazin-2-yl]-phenyl,
4-[4,6-Di(3,4,5-tri(tetracosanyloxy)phenylamino)-1,3,5-triazin-2-yl]-phenyl,
4-[4,6-Di(3,4,5-tri(octacosanyloxy)phenylamino)-1,3,5-triazin-2-yl]-phenyl;

4-[4,6-Di(3,4,5-tri(n-butylthio)phenylamino)-1,3,5-triazin-2-yl]-phenyl,
4-[4,6-Di(3,4,5-tri(n-pentylthio)phenylamino)-1,3,5-triazin-2-yl]-phenyl,
4-[4,6-Di(3,4,5-tri(n-hexylthio)phenylamino)-1,3,5-triazin-2-yl]-phenyl,
4-[4,6-Di(3,4,5-tri(n-heptylthio)phenylamino)-1,3,5-triazin-2-yl]-phenyl,
4-[4,6-Di(3,4,5-tri(n-octylthio)phenylamino)-1,3,5-triazin-2-yl]-phenyl,
4-[4,6-Di(3,4,5-tri(n-nonylthio)phenylamino)-1,3,5-triazin-2-yl]-phenyl,
4-[4,6-Di(3,4,5-tri(n-decylthio)phenylamino)-1,3,5-triazin-2-yl]-phenyl,
4-[4,6-Di(3,4,5-tri(n-undecylthio)phenylamino)-1,3,5-triazin-2-yl]-phenyl,
4-[4,6-Di(3,4,5-tri(n-dodecylthio)phenylamino)-1,3,5-triazin-2-yl]-phenyl,
4-[4,6-Di(3,4,5-tri(n-tridecylthio)phenylamino)-1,3,5-triazin-2-yl]-phenyl,
4-[4,6-Di(3,4,5-tri(n-tetradecylthio)phenylamino)-1,3,5-triazin-2-yl]-phenyl,
4-[4,6-Di(3,4,5-tri(n-pentadecylthio)phenylamino)-1,3,5-triazin-2-yl]-phenyl,
4-[4,6-Di(3,4,5-tri(n-hexadecylthio)phenylamino)-1,3,5-triazin-2-yl]-phenyl,
4-[4,6-Di(3,4,5-tri(n-heptadecylthio)phenylamino)-1,3,5-triazin-2-yl]-phenyl,
4-[4,6-Di(3,4,5-tri(n-octadecylthio)phenylamino)-1,3,5-triazin-2-yl]-phenyl,
4-[4,6-Di(3,4,5-tri(nonadecylthio)phenylamino)-1,3,5-triazin-2-yl]-phenyl,
4-[4,6-Di(3,4,5-tri(eicosylthio)phenylamino)-1,3,5-triazin-2-yl]-phenyl,
4-[4,6-Di(3,4,5-tri(docosanylthio)phenylamino)-1,3,5-triazin-2-yl]-phenyl,
4-[4,6-Di(3,4,5-tri(tricosanylthio)phenylamino)-1,3,5-triazin-2-yl]-phenyl,
4-[4,6-Di(3,4,5-tri(tetracosanylthio)phenylamino)-1,3,5-triazin-2-yl]-phenyl,
4-[4,6-Di(3,4,5-tri(octacosanylthio)phenylamino)-1,3,5-triazin-2-yl]-phenyl,
in denen q für 1 steht, wie z. B.
4-[4,6-Di-(3,4,5-tri(n-butyl)phenylamino)-1,3,5-triazin-2-yl]-benzyl,
4-[4,6-Di-(3,4,5-tri(n-pentyl)phenylamino)-1,3,5-triazin-2-yl]-benzyl,
4-[4,6-Di-(3,4,5-tri(n-hexyl)phenylamino)-1,3,5-triazin-2-yl]-benzyl,
4-[4,6-Di-(3,4,5-tri(n-heptyl)phenylamino)-1,3,5-triazin-2-yl]-benzyl,
4-[4,6-Di-(3,4,5-tri(n-octyl)phenylamino)-1,3,5-triazin-2-yl]-benzyl,
4-[4,6-Di-(3,4,5-tri(n-nonyl)phenylamino)-1,3,5-triazin-2-yl]-benzyl,
4-[4,6-Di-(3,4,5-tri(n-decyl)phenylamino)-1,3,5-triazin-2-yl]-benzyl,
4-[4,6-Di-(3,4,5-tri(n-undecy)phenylamino)-1,3,5-triazin-2-yl]-benzyl,
4-[4,6-Di-(3,4,5-tri(n-dodecyl)phenylamino)-1,3,5-triazin-2-yl]-benzyl,
4-[4,6-Di-(3,4,5-tri(n-tridecyl)phenylamino)-1,3,5-triazin-2-yl]-benzyl,
4-[4,6-Di-(3,4,5-tri(n-tetradecyl)phenylamino)-1,3,5-triazin-2-yl]-benzyl,
4-[4,6-Di-(3,4,5-tri(n-pentadecyl)phenylamino)-1,3,5-triazin-2-yl]-benzyl,
4-[4,6-Di-(3,4,5-tri(n-hexadecyl)phenylamino)-1,3,5-triazin-2-yl]-benzyl,
4-[4,6-Di-(3,4,5-tri(n-heptadecyl)phenylamino)-1,3,5-triazin-2-yl]-benzyl,
4-[4,6-Di-(3,4,5-tri(n-octadecyl)phenylamino)-1,3,5-triazin-2-yl]-benzyl,
4-[4,6-Di-(3,4,5-tri(nonadecyl)phenylamino)-1,3,5-triazin-2-yl]-benzyl,
4-[4,6-Di-(3,4,5-tri(eicosyl)phenylamino)-1,3,5-triazin-2-yl]-benzyl,
4-[4,6-Di-(3,4,5-tri(docosanyl)phenylamino)-1,3,5-triazin-2-yl]-benzyl,
4-[4,6-Di-(3,4,5-tri(tricosanyl)phenylamino)-1,3,5-triazin-2-yl]-benzyl,
4-[4,6-Di-(3,4,5-tri(tetracosanyl)phenylamino)-1,3,5-triazin-2-yl]-benzyl,
4-[4,6-Di-(3,4,5-tri(octacosanyl)phenylamino)-1,3,5-triazin-2-yl]-benzyl;

4-[4,6-Di(3,4,5-tri(n-butyloxy)phenylamino)-1,3,5-triazin-2-yl]-benzyl,
4-[4,6-Di(3,4,5-tri(n-pentyloxy)phenylamino)-1,3,5-triazin-2-yl]-benzyl,
4-[4,6-Di(3,4,5-tri(n-hexyloxy)phenylamino)-1,3,5-triazin-2-yl]-benzyl,
4-[4,6-Di(3,4,5-tri(n-heptyloxy)phenylamino)-1,3,5-triazin-2-yl]-benzyl,
4-[4,6-Di(3,4,5-tri(n-octyloxy)phenylamino)-1,3,5-triazin-2-yl]-benzyl,
4-[4,6-Di(3,4,5-tri(n-nonyloxy)phenylamino)-1,3,5-triazin-2-yl]-benzyl,
4-[4,6-Di(3,4,5-tri(n-decyloxy)phenylamino)-1,3,5-triazin-2-yl]-benzyl,
4-[4,6-Di(3,4,5-tri(n-undecyloxy)phenylamino)-1,3,5-triazin-2-yl]-benzyl,
4-[4,6-Di(3,4,5-tri(n-dodecyloxy)phenylamino)-1,3,5-triazin-2-yl]-benzyl,
4-[4,6-Di(3,4,5-tri(n-tridecyloxy)phenylamino)-1,3,5-triazin-2-yl]-benzyl,
4-[4,6-Di(3,4,5-tri(n-tetradecyloxy)phenylamino)-1,3,5-triazin-2-yl]-benzyl,
4-[4,6-Di(3,4,5-tri(n-pentadecyloxy)phenylamino)-1,3,5-triazin-2-yl]-benzyl,
4-[4,6-Di(3,4,5-tri(n-hexadecyloxy)phenylamino)-1,3,5-triazin-2-yl]-benzyl,
4-[4,6-Di(3,4,5-tri(n-heptadecyloxy)phenylamino)-1,3,5-triazin-2-yl]-benzyl,
4-[4,6-Di(3,4,5-tri(n-octadecyloxy)phenylamino)-1,3,5-triazin-2-yl]-benzyl,
4-[4,6-Di(3,4,5-tri(nonadecyloxy)phenylamino)-1,3,5-triazin-2-yl]-benzyl,
4-[4,6-Di(3,4,5-tri(eicosyloxy)phenylamino)-1,3,5-triazin-2-yl]-benzyl,
4-[4,6-Di(3,4,5-tri(docosanyloxy)phenylamino)-1,3,5-triazin-2-yl]-benzyl,
4-[4,6-Di(3,4,5-tri(tricosanyloxy)phenylamino)-1,3,5-triazin-2-yl]-benzyl,
4-[4,6-Di(3,4,5-tri(tetracosanyloxy)phenylamino)-1,3,5-triazin-2-yl]-benzyl,
4-[4,6-Di(3,4,5-tri(octacosanyloxy)phenylamino)-1,3,5-triazin-2-yl]-benzyl;

4-[4,6-Di(3,4,5-tri(n-butylthio)phenylamino)-1,3,5-triazin-2-yl]-benzyl,
4-[4,6-Di(3,4,5-tri(n-pentylthio)phenylamino)-1,3,5-triazin-2-yl]-benzyl,
4-[4,6-Di(3,4,5-tri(n-hexylthio)phenylamino)-1,3,5-triazin-2-yl]-benzyl,
4-[4,6-Di(3,4,5-tri(n-heptylthio)phenylamino)-1,3,5-triazin-2-yl]-benzyl,
4-[4,6-Di(3,4,5-tri(n-octylthio)phenylamino)-1,3,5-triazin-2-yl]-benzyl,
4-[4,6-Di(3,4,5-tri(n-nonylthio)phenylamino)-1,3,5-triazin-2-yl]-benzyl,
4-[4,6-Di(3,4,5-tri(n-decylthio)phenylamino)-1,3,5-triazin-2-yl]-benzyl,
4-[4,6-Di(3,4,5-tri(n-undecylthio)phenylamino)-1,3,5-triazin-2-yl]-benzyl,
4-[4,6-Di(3,4,5-tri(n-dodecylthio)phenylamino)-1,3,5-triazin-2-yl]-benzyl,
4-[4,6-Di(3,4,5-tri(n-tridecylthio)phenylamino)-1,3,5-triazin-2-yl]-benzyl,
4-[4,6-Di(3,4,5-tri(n-tetradecylthio)phenylamino)-1,3,5-triazin-2-yl]-benzyl,
4-[4,6-Di(3,4,5-tri(n-pentadecylthio)phenylamino)-1,3,5-triazin-2-yl]-benzyl,
4-[4,6-Di(3,4,5-tri(n-hexadecylthio)phenylamino)-1,3,5-triazin-2-yl]-benzyl,
4-[4,6-Di(3,4,5-tri(n-heptadecylthio)phenylamino)-1,3,5-triazin-2-yl]-benzyl,
4-[4,6-Di(3,4,5-tri(n-octadecylthio)phenylamino)-1,3,5-triazin-2-yl]-benzyl,
4-[4,6-Di(3,4,5-tri(nonadecylthio)phenylamino)-1,3,5-triazin-2-yl]-benzyl,
4-[4,6-Di(3,4,5-tri(eicosylthio)phenylamino)-1,3,5-triazin-2-yl]-benzyl,
4-[4,6-Di(3,4,5-tri(docosanylthio)phenylamino)-1,3,5-triazin-2-yl]-benzyl,
4-[4,6-Di(3,4,5-tri(tricosanylthio)phenylamino)-1,3,5-triazin-2-yl]-benzyl,
4-[4,6-Di(3,4,5-tri(tetracosanylthio)phenylamino)-1,3,5-triazin-2-yl]-benzyl,
4-[4,6-Di(3,4,5-tri(octacosanylthio)phenylamino)-1,3,5-triazin-2-yl]-benzyl,
in denen q für 2 steht, wie z. B.
4-[4,6-Di-(3,4,5-tri(n-butyl)phenylamino)-1,3,5-triazin-2-yl]-phenethyl,
4-[4,6-Di-(3,4,5-tri(n-pentyl)phenylamino)-1,3,5-triazin-2-yl]-phenethyl,
4-[4,6-Di-(3,4,5-tri(n-hexyl)phenylamino)-1,3,5-triazin-2-yl]-phenethyl,
4-[4,6-Di-(3,4,5-tri(n-heptyl)phenylamino)-1,3,5-triazin-2-yl]-phenethyl,
4-[4,6-Di-(3,4,5-tri(n-octyl)phenylamino)-1,3,5-triazin-2-yl]-phenethyl,
4-[4,6-Di-(3,4,5-tri(n-nonyl)phenylamino)-1,3,5-triazin-2-yl]-phenethyl,
4-[4,6-Di-(3,4,5-tri(n-decyl)phenylamino)-1,3,5-triazin-2-yl]-phenethyl,
4-[4,6-Di-(3,4,5-tri(n-undecy)phenylamino)-1,3,5-triazin-2-yl]-phenethyl,
4-[4,6-Di-(3,4,5-tri(n-dodecyl)phenylamino)-1,3,5-triazin-2-yl]-phenethyl,
4-[4,6-Di-(3,4,5-tri(n-tridecyl)phenylamino)-1,3,5-triazin-2-yl]-phenethyl,
4-[4,6-Di-(3,4,5-tri(n-tetradecyl)phenylamino)-1,3,5-triazin-2-yl]-phenethyl,
4-[4,6-Di-(3,4,5-tri(n-pentadecyl)phenylamino)-1,3,5-triazin-2-yl]-phenethyl,
4-[4,6-Di-(3,4,5-tri(n-hexadecyl)phenylamino)-1,3,5-triazin-2-yl]-phenethyl,
4-[4,6-Di-(3,4,5-tri(n-heptadecyl)phenylamino)-1,3,5-triazin-2-yl]-phenethyl,
4-[4,6-Di-(3,4,5-tri(n-octadecyl)phenylamino)-1,3,5-triazin-2-yl]-phenethyl,
4-[4,6-Di-(3,4,5-tri(nonadecyl)phenylamino)-1,3,5-triazin-2-yl]-phenethyl,
4-[4,6-Di-(3,4,5-tri(eicosyl)phenylamino)-1,3,5-triazin-2-yl]-phenethyl,
4-[4,6-Di-(3,4,5-tri(docosanyl)phenylamino)-1,3,5-triazin-2-yl]-phenethyl,
4-[4,6-Di-(3,4,5-tri(tricosanyl)phenylamino)-1,3,5-triazin-2-yl]-phenethyl,
4-[4,6-Di-(3,4,5-tri(tetracosanyl)phenylamino)-1,3,5-triazin-2-yl]-phenethyl,
4-[4,6-Di-(3,4,5-tri(octacosanyl)phenylamino)-1,3,5-triazin-2-yl]-phenethyl;

4-[4,6-Di(3,4,5-tri(n-butyloxy)phenylamino)-1,3,5-triazin-2-yl]-phenethyl,
4-[4,6-Di(3,4,5-tri(n-pentyloxy)phenylamino)-1,3,5-triazin-2-yl]-phenethyl,
4-[4,6-Di(3,4,5-tri(n-hexyloxy)phenylamino)-1,3,5-triazin-2-yl]-phenethyl,
4-[4,6-Di(3,4,5-tri(n-heptyloxy)phenylamino)-1,3,5-triazin-2-yl]-phenethyl,
4-[4,6-Di(3,4,5-tri(n-octyloxy)phenylamino)-1,3,5-triazin-2-yl]-phenethyl,
4-[4,6-Di(3,4,5-tri(n-nonyloxy)phenylamino)-1,3,5-triazin-2-yl]-phenethyl,
4-[4,6-Di(3,4,5-tri(n-decyloxy)phenylamino)-1,3,5-triazin-2-yl]-phenethyl,
4-[4,6-Di(3,4,5-tri(n-undecyloxy)phenylamino)-1,3,5-triazin-2-yl]-phenethyl,
4-[4,6-Di(3,4,5-tri(n-dodecyloxy)phenylamino)-1,3,5-triazin-2-yl]-phenethyl,
4-[4,6-Di(3,4,5-tri(n-tridecyloxy)phenylamino)-1,3,5-triazin-2-yl]-phenethyl,
4-[4,6-Di(3,4,5-tri(n-tetradecyloxy)phenylamino)-1,3,5-triazin-2-yl]-phenethyl,
4-[4,6-Di(3,4,5-tri(n-pentadecyloxy)phenylamino)-1,3,5-triazin-2-yl]-phenethyl,
4-[4,6-Di(3,4,5-tri(n-hexadecyloxy)phenylamino)-1,3,5-triazin-2-yl]-phenethyl,
4-[4,6-Di(3,4,5-tri(n-heptadecyloxy)phenylamino)-1,3,5-triazin-2-yl]-phenethyl,
4-[4,6-Di(3,4,5-tri(n-octadecyloxy)phenylamino)-1,3,5-triazin-2-yl]-phenethyl,
4-[4,6-Di(3,4,5-tri(nonadecyloxy)phenylamino)-1,3,5-triazin-2-yl]-phenethyl,
4-[4,6-Di(3,4,5-tri(eicosyloxy)phenylamino)-1,3,5-triazin-2-yl]-phenethyl,
4-[4,6-Di(3,4,5-tri(docosanyloxy)phenylamino)-1,3,5-triazin-2-yl]-phenethyl,
4-[4,6-Di(3,4,5-tri(tricosanyloxy)phenylamino)-1,3,5-triazin-2-yl]-phenethyl,
4-[4,6-Di(3,4,5-tri(tetracosanyloxy)phenylamino)-1,3,5-triazin-2-yl]-phenethyl,
4-[4,6-Di(3,4,5-tri(octacosanyloxy)phenylamino)-1,3,5-triazin-2-yl]-phenethyl;

4-[4,6-Di(3,4,5-tri(n-butylthio)phenylamino)-1,3,5-triazin-2-yl]-phenethyl,
4-[4,6-Di(3,4,5-tri(n-pentylthio)phenylamino)-1,3,5-triazin-2-yl]-phenethyl,
4-[4,6-Di(3,4,5-tri(n-hexylthio)phenylamino)-1,3,5-triazin-2-yl]-phenethyl,
4-[4,6-Di(3,4,5-tri(n-heptylthio)phenylamino)-1,3,5-triazin-2-yl]-phenethyl,
4-[4,6-Di(3,4,5-tri(n-octylthio)phenylamino)-1,3,5-triazin-2-yl]-phenethyl,
4-[4,6-Di(3,4,5-tri(n-nonylthio)phenylamino)-1,3,5-triazin-2-yl]-phenethyl,
4-[4,6-Di(3,4,5-tri(n-decylthio)phenylamino)-1,3,5-triazin-2-yl]-phenethyl,
4-[4,6-Di(3,4,5-tri(n-undecylthio)phenylamino)-1,3,5-triazin-2-yl]-phenethyl,
4-[4,6-Di(3,4,5-tri(n-dodecylthio)phenylamino)-1,3,5-triazin-2-yl]-phenethyl,
4-[4,6-Di(3,4,5-tri(n-tridecylthio)phenylamino)-1,3,5-triazin-2-yl]-phenethyl,
4-[4,6-Di(3,4,5-tri(n-tetradecylthio)phenylamino)-1,3,5-triazin-2-yl]-phenethyl,
4-[4,6-Di(3,4,5-tri(n-pentadecylthio)phenylamino)-1,3,5-triazin-2-yl]-phenethyl,
4-[4,6-Di(3,4,5-tri(n-hexadecylthio)phenylamino)-1,3,5-triazin-2-yl]-phenethyl,
4-[4,6-Di(3,4,5-tri(n-heptadecylthio)phenylamino)-1,3,5-triazin-2-yl]-phenethyl,
4-[4,6-Di(3,4,5-tri(n-octadecylthio)phenylamino)-1,3,5-triazin-2-yl]-phenethyl,
4-[4,6-Di(3,4,5-tri(nonadecylthio)phenylamino)-1,3,5-triazin-2-yl]-phenethyl,
4-[4,6-Di(3,4,5-tri(eicosylthio)phenylamino)-1,3,5-triazin-2-yl]-phenethyl,
4-[4,6-Di(3,4,5-tri(docosanylthio)phenylamino)-1,3,5-triazin-2-yl]-phenethyl,
4-[4,6-Di(3,4,5-tri(tricosanylthio)phenylamino)-1,3,5-triazin-2-yl]-phenethyl,
4-[4,6-Di(3,4,5-tri(tetracosanylthio)phenylamino)-1,3,5-triazin-2-yl]-phenethyl,
4-[4,6-Di(3,4,5-tri(octacosanylthio)phenylamino)-1,3,5-triazin-2-yl]-phenethyl,
des Weiteren Reste der Formel H 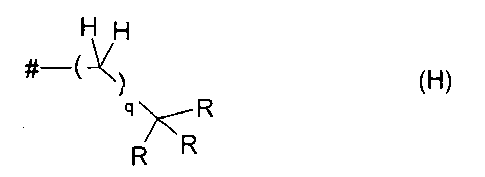 worin # die Verknüpfungsstelle mit dem Imidstickstoffatom des Rylentetracarbonsäurediimids ist, q für eine ganze Zahl 0, 1, 2, 3, 4, 5 oder6 steht und R für C₄-C₃₀-Alkyl, C₄-C₃₀-Alkylthio oder C₄-C₃₀-Alkoxy steht.

Beispiele für geeignete Reste der Formel H umfassen die Formeln H-0.a, H-0.b, H-0.c, H-1.a, H-1.b, H-1.c, H-2.a, H-2.b, H-2.c, H-3.a, H-3.b, H-3.c, H-4.a, H-4.b, H-4.c, H-5.a, H-5.b, H-5.c, H-6.a, H-6.b, H-6.c 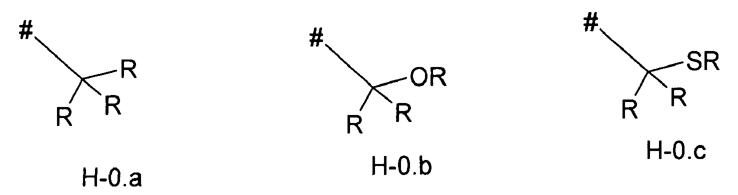 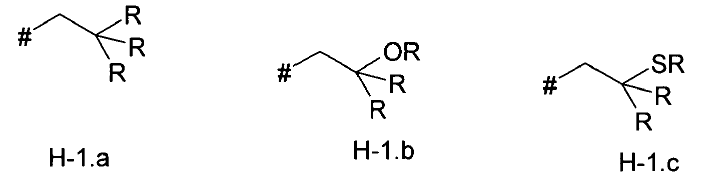 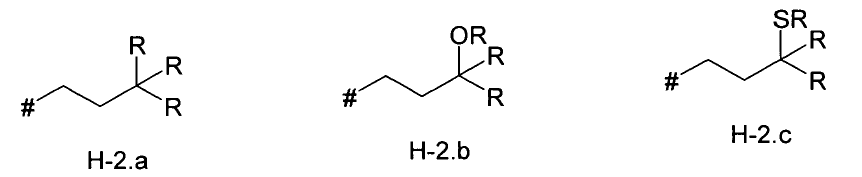 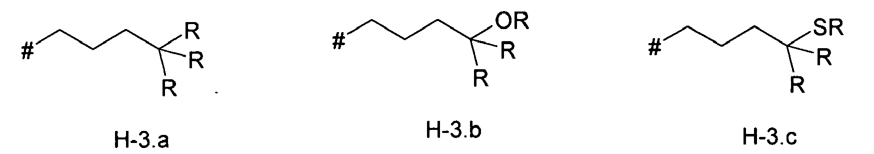 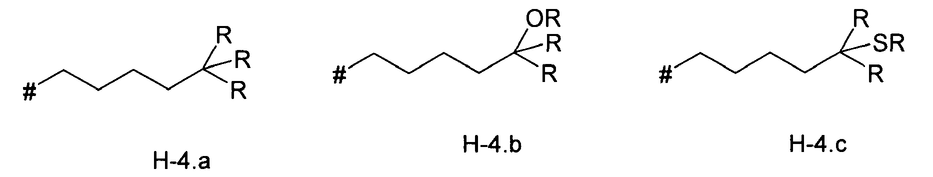 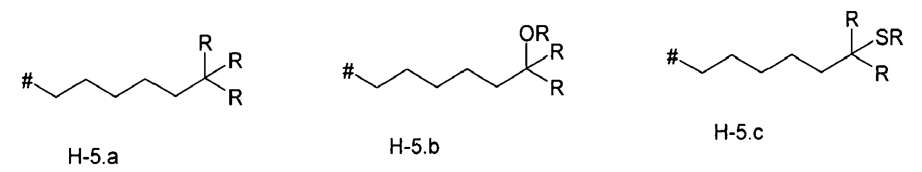 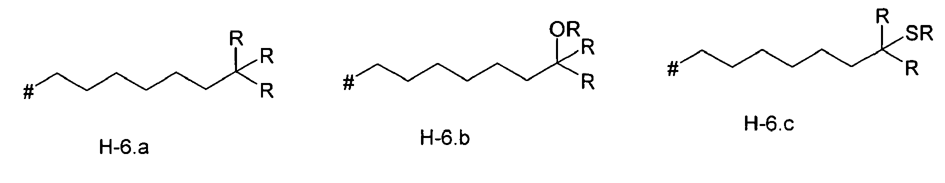 worin # die Verknüpfungsstelle mit dem Imidstickstoffatom des Rylentetracarbonsäurediimids ist, und R unabhängig voneinander für n-Butyl, n-Pentyl, n-Hexyl, n-Heptyl, n-Octyl, n-Nonyl, n-Decyl, n-Undecyl, n-dodecyl, n-Tridecyl, n-Tetradecyl, n-Pentadecyl, n-Hexadecyl, n-Heptadecyl, n-Octadecyl, n-Nonadecyl, n-Eicosyl, n- Docosanyl, n-Tricosanyl, n-Tetracosanyl, n-Octacosanyl steht,
des Weiteren Verbindungen der Formel K 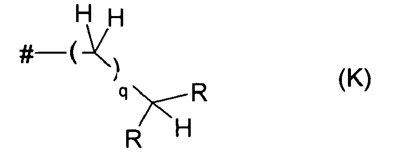 worin # die Verknüpfungsstelle mit dem Imidstickstoffatom des Rylentetracarbonsäurediimids ist, q für eine ganze Zahl 0, 1, 2, 3, 4, 5 oder6 steht und R für C₄-C₃₀-Alkyl, C₄-C₃₀-Alkylthio oder C₄-C₃₀-Alkoxy steht.

Reste der Formel K umfassen solche, in denen q für 0 steht, wie z. B.
1-Ethylpropyl, 1-Methylpropyl, 1-Propylbutyl, 1-Ethylbutyl, 1-Methylbutyl, 1-Butylpentyl, 1-Propylpentyl, 1-Ethylpentyl, 1-Methylpentyl, 1-Pentylhexyl, 1-Butylhexyl, 1-Propylhexyl, 1-Ethylhexyl, 1-Methylhexyl, 1-Hexylheptyl, 1-Pentylheptyl, 1-Butylheptyl, 1-Propylheptyl, 1-Ethylheptyl, 1-Methylheptyl, 1-Heptyloctyl, 1-Hexyloctyl, 1-Pentyloctyl, 1-Butyloctyl, 1-Propyloctyl, 1-Ethyloctyl, 1-Methyloctyl, 1-Octylnonyl, 1-Heptylnonyl, 1-Hexylnonyl, 1-Pentylnonyl, 1-Butylnonyl, 1-Propylnonyl, 1-Ethylnonyl, 1-Methylnonyl, 1-Nonyldecyl, 1-Octyldecyl, 1-Heptyldecyl, 1-Hexyldecyl, 1-Pentyldecyl, 1-Butyldecyl, 1-Propyldecyl, 1-Ethyldecyl, 1-Methyldecyl, 1-Decylundecyl, 1-Nonylundecyl, 1-Octylundecyl, 1-Heptylundecyl, 1-Hexylundecyl, 1-Pentylundecyl, 1-Butylundecyl, 1-Propylundecyl, 1-Ethylundecyl, 1-Methylundecyl, 1-Undecyldodecyl, 1-Decyldodecyl, 1-Nonyldodecyl, 1-Octyldodecyl, 1-Heptyldodecyl, 1-Hexyldodecyl, 1-Pentyldodecyl, 1-Butyldodecyl, 1-Propyldodecyl, 1-Ethyldodecyl, 1-Methyldodecyl, 1-Dodecyltridecyl, 1-Undecyltridecyl, 1-Decyltridecyl, 1-Nonyltridecyl, 1-Octyltridecyl, 1-Heptyltridecyl, 1-Hexyltridecyl, 1-Pentyltridecyl, 1-Butyltridecyl, 1-Propyltridecyl, 1-Ethyltridecyl, 1-Methyltridecyl, 1-Tridecyltetrdecyl, 1-Undecyltetradecyl, 1-Decyltetradecyl, 1-Nonyltetradecyl, 1-Octyltetradecyl, 1-Hetyltetradecyl, 1-Hexyltetradecyl, 1-Pentytetradecyl, 1-Butyltetradecyl, 1-Propyltetradecyl, 1-Ethyltetradecyl, 1-Methyltetradecyl, 1-Pentadecylhexadecyl, 1-Tetradecylhexadecyl, 1-Tridecylhexadecyl, 1-Dodecylhexadecyl, 1-Undecylhexadecyl, 1-Decylhexadecyl, 1-Nonylhexadecyl, 1-Octylhexadecyl, 1-Heptylhexadecyl, 1-Hexylhexadecyl, 1-Pentylhexadecyl, 1-Butylhexadecyl, 1-Propylhexadecyl, 1-Ethylhexadecyl, 1-Methylhexadecyl, 1-Hexadecyloctadecyl, 1-Pentadecyloctadecyl, 1-Tetradecyloctadecyl, 1-Tridecyloctadecyl, 1-Dodecyloctadecyl, 1-Undecyloctadecyl, 1-Decyloctadecyl, 1-Nonyloctadecyl, 1-Octylocatedecyl, 1-Heptyloctadecyl, 1-Hexyloctadecyl, 1-Pentyloctadecyl, 1-Butyloctaedecyl, 1-Propyloctadecyl, 1-Ethyloctadecyl, 1-Methyloctadecyl 1-Nonadecyleicosanyl, 1-Octadecyleicosanyl, 1-Heptadecyleicosanyl, 1-Hexadecyleicosanyl, 1-Pentadecyleicosanyl, 1-Tetradecyleicosanyl, 1-Tridecyleicosanyl, 1-Dodecyleicosanyl, 1-Undecyleicosanyl, 1-Decyleicosanyl, 1-Nonyleicosanyl, 1-Octyleicosanyl, 1-Heptyleicosanyl, 1-Hexyleicosanyl, 1-Pentyleicosanyl, 1-Butyleicosanyl, 1-Propyleicosanyl, 1-Ethyleicosanyl, 1-Methyeicosanyl 1-Eicosanyldocosanyl, 1-Nonadecyldocosanyl, 1-Octadecyldocosanyl, 1-Heptadecyldocosanyl, 1-Hexadecyldocosanyl, 1-Pentadecyldocosanyl, 1-Tetradecyldocosanyl, 1-Tridecyldocosanyl, 1-Undecyldocosanyl, 1-Decyldocosanyl, 1-Nonyldocosanyl, 1-Octyldocosanyl, 1-Heptyldocosanyl, 1-Hexyldocosanyl, 1-Pentyldocosanyl, 1-Butyldocosanyl, 1-Propyldocosanyl, 1-Ethyldocosanyl, 1-Methyldocosanyl 1-Tricosanyltetracosanyl, 1-Docosanyltetracosanyl, 1-Nonadecyltetracosanyl, 1-Octadecyltetracosanyl, 1-Heptadecyltetracosanyl, 1-Hexadecyltetracosanyl, 1-Pentadecyltetracosanyl, 1-Pentadecyltetracosanyl, 1-Tetradecyltetracosanyl, 1-Tridecyltetracosanyl, 1-Dodecyltetracosanyl, 1-Undecyltetracosanyl, 1-Decyltetracosanyl, 1-Nonyltetracosanyl, 1-Octyltetracosanyl, 1-Heptyltetracosanyl, 1-Hexyltetracosanyl, 1-Pentyltetracosanyl, 1-Butyltetracosanyl, 1-Propyltetracosanyl, 1-Ethyltetracosanyl, 1-Methyltetracosanyl 1-Heptacosanyloctacosanyl, 1-Hexacosanyloctacosanyl, 1-Pentcosanyloctacosanyl, 1-Tetracosanyloctcosanyl, 1-Tricosanyloctacosanyl, 1-Docosanyloctacosanyl, 1-Nonadecyloctacosanyl, 1-Octadecyloctacosanyl, 1-Heptadecyloctacosanyl, 1-Hexadecyloctacosanyl, 1-Hexadecyloctacosanyl, 1-Pentadecyloctacosanyl, 1-Tetradecyloctacosanyl, 1-Tridecyloctacosanyl, 1-Dodecyloctacosanyl, 1-Undecyloctacosanyl, 1-Decyloctacosanyl, 1-Nonyloctacosanyl, 1-Octyloctacosanyl, 1-Heptyloctacosanyl, 1-Hexyloctacosanyl, 1-Pentyloctacosanyl, 1-Butyloctacosanyl, 1-Propyloctacosanyl, 1-Ethyloctacosanyl, 1-Methyloctacosanyl;
1-Ethyloxypropyl, 1-Methyloxypropyl, 1-Propylbutyl, 1-Ethyloxybutyl, 1-Methyloxybutyl, 1-Butyloxypentyl, 1-Propylpentyl, 1-Ethyloxypentyl, 1-Methyloxypentyl, 1-Pentyloxyhexyl, 1-Butyloxyhexyl, 1-Propylhexyl, 1-Ethyloxyhexyl, 1-Methyloxyhexyl, 1-Hexyloxyheptyl, 1-Pentyloxyheptyl, 1-Butyloxyheptyl, 1-Propylheptyl, 1-Ethyloxyheptyl, 1-Methyloxyheptyl, 1-Heptyloctyl, 1-Hexyloxyoctyl, 1-Pentyloxyoctyl, 1-Butyloxyoctyl, 1-Propyloctyl, 1-Ethyloxyoctyl, 1-Methyloxyoctyl, 1-Octyloxynonyl, 1-Heptylnonyl, 1-Hexyloxynonyl, 1-Pentyloxynonyl, 1-Butyloxynonyl, 1-Propylnonyl, 1-Ethyloxynonyl, 1-Methyloxynonyl, 1-Nonyloxydecyl, 1-Octyloxydecyl, 1-Heptyldecyl, 1-Hexyloxydecyl, 1-Pentyloxydecyl, 1-Butyloxydecyl, 1-Propyldecyl, 1-Ethyloxydecyl, 1-Methyloxydecyl, 1-Decyloxyundecyl, 1-Nonyloxyundecyl, 1-Octyloxyundecyl, 1-Heptylundecyl, 1-Hexyloxyundecyl, 1-Pentyloxyundecyl, 1-Butyloxyundecyl, 1-Propylundecyl, 1-Ethyloxyundecyl, 1-Methyloxyundecyl, 1-Undecyloxydodecyl, 1-Decyloxydodecyl, 1-Nonyloxydodecyl, 1-Octyloxydodecyl, 1-Heptyldodecyl, 1-Hexyloxydodecyl, 1-Pentyloxydodecyl, 1-Butyloxydodecyl, 1-Propyldodecyl, 1-Ethyloxydodecyl, 1-Methyloxydodecyl, 1-Dodecyloxytridecyl, 1-Undecyloxytridecyl, 1-Decyloxytridecyl, 1-Nonyloxytridecyl, 1-Octyloxytridecyl, 1-Heptyltridecyl, 1-Hexyloxytridecyl, 1-Pentyloxytridecyl, 1-Butyloxytridecyl, 1-Propyltridecyl, 1-Ethyloxytridecyl, 1-Methyloxytridecyl, 1-Tridecyloxytetrdecyl, 1-Undecyloxytetradecyl, 1-Decyloxytetradecyl, 1-Nonyloxytetradecyl, 1-Octyloxytetradecyl, 1-Hetyltetradecyl, 1-Hexyloxytetradecyl, 1-Pentytetradecyl, 1-Butyloxytetradecyl, 1-Propyltetradecyl, 1-Ethyloxytetradecyl, 1-Methyloxytetradecyl, 1-Pentadecyloxyhexadecyl, 1-Tetradecyloxyhexadecyl, 1-Tridecyloxyhexadecyl, 1-Dodecyloxyhexadecyl, 1-Undecyloxyhexadecyl, 1-Decyloxyhexadecyl, 1-Nonyloxyhexadecyl, 1-Octyloxyhexadecyl, 1-Heptylhexadecyl, 1-Hexyloxyhexadecyl, 1-Pentyloxyhexadecyl, 1-Butyloxyhexadecyl, 1-Propylhexadecyl, 1-Ethyloxyhexadecyl, 1-Methyloxyhexadecyl, 1-Hexadecyloxyoctadecyl, 1-Pentadecyloxyoctadecyl, 1-Tetradecyloxyoctadecyl, 1-Tridecyloxyoctadecyl, 1-Dodecyloxyoctadecyl, 1-Undecyloxyoctadecyl, 1-Decyloxyoctadecyl, 1-Nonyloxyoctadecyl, 1-Octyloxyocatedecyl, 1-Heptyloctadecyl, 1-Hexyloxyoctadecyl, 1-Pentyloxyoctadecyl, 1-Butyloxyoctaedecyl, 1-Propyloctadecyl, 1-Ethyloxyoctadecyl, 1-Methyloxyoctadecyl 1-nonadecyloxyeicosanyl, 1-Octadecyloxyeicosanyl, 1-Heptadecyloxyeicosanyl, 1-Hexadecyloxyeicosanyl, 1-Pentadecyloxyeicosanyl, 1-Tetradecyloxyeicosanyl, 1-Tridecyloxyeicosanyl, 1-Dodecyloxyeicosanyl, 1-Undecyloxyeicosanyl, 1-Decyloxyeicosanyl, 1-Nonyloxyeicosanyl, 1-Octyloxyeicosanyl, 1-Heptyleicosanyl, 1-Hexyloxyeicosanyl, 1-Pentyloxyeicosanyl, 1-Butyloxyeicosanyl, 1-Propyleicosanyl, 1-Ethyloxyeicosanyl, 1-Methyeicosanyl 1-Eicosanyloxydocosanyl, 1-nonadecyloxydocosanyl, 1-Octadecyloxydocosanyl, 1-Heptadecyloxydocosanyl, 1-Hexadecyloxydocosanyl, 1-Pentadecyloxydocosanyl, 1-Tetradecyloxydocosanyl, 1-Tridecyloxydocosanyl, 1-Undecyloxydocosanyl, 1-Decyloxydocosanyl, 1-Nonyloxydocosanyl, 1-Octyloxydocosanyl, 1-Heptyldocosanyl, 1-Hexyloxydocosanyl, 1-Pentyloxydocosanyl, 1-Butyloxydocosanyl, 1-Propyldocosanyl, 1-Ethyloxydocosanyl, 1-Methyloxydocosanyl 1-Tricosanyloxytetracosanyl, 1-Docosanyloxytetracosanyl, 1-nonadecyloxytetracosanyl, 1-Octadecyloxytetracosanyl, 1-Heptadecyloxytetracosanyl, 1-Hexadecyloxytetracosanyl, 1-Pentadecyloxytetracosanyl, 1-Pentadecyloxytetracosanyl, 1-Tetradecyloxytetracosanyl, 1-Tridecyloxytetracosanyl, 1-Dodecyloxytetracosanyl, 1-Undecyloxytetracosanyl, 1-Decyloxytetracosanyl, 1-Nonyloxytetracosanyl, 1-Octyloxytetracosanyl, 1-Heptyltetracosanyl, 1-Hexyloxytetracosanyl, 1-Pentyloxytetracosanyl, 1-Butyloxytetracosanyl, 1-Propyltetracosanyl, 1-Ethyloxytetracosanyl, 1-Methyloxytetracosanyl 1-Heptacosanyloxyoctacosanyl, 1-Hexacosanyloxyoctacosanyl, 1-Pentcosanyloxyoctacosanyl, 1-Tetracosanyloxyoctcosanyl, 1-Tricosanyloxyoctacosanyl, 1-Docosanyloxyoctacosanyl, 1-Nonadecyloxyoctacosanyl, 1-Octadecyloxyoctacosanyl, 1-Heptadecyloxyoctacosanyl, 1-Hexadecyloxyoctacosanyl, 1-Hexadecyloxyoctacosanyl, 1-Pentadecyloxyoctacosanyl, 1-Tetradecyloxyoctacosanyl, 1-Tridecyloxyoctacosanyl, 1-Dodecyloxyoctacosanyl, 1-Undecyloxyoctacosanyl, 1-Decyloxyoctacosanyl, 1-Nonyloxyoctacosanyl, 1-Octyloxyoctacosanyl, 1-Heptyloctacosanyl, 1-Hexyloxyoctacosanyl, 1-Pentyloxyoctacosanyl, 1-Butyloxyoctacosanyl, 1-Propyloxyoctacosanyl, 1-Ethyloxyoctacosanyl, 1-Methyloxyoctacosanyl;
1-Ethylthiopropyl, 1-Methylthiopropyl, 1-Propylbutyl, 1-Ethylthiobutyl, 1-Methylthiobutyl, 1-Butylthiopentyl, 1-Propylpentyl, 1-Ethylthiopentyl, 1-Methylthiopentyl, 1-Pentylthiohexyl, 1-Butylthiohexyl, 1-Propylhexyl, 1-Ethylthiohexyl, 1-Methylthiohexyl, 1-Hexylthioheptyl, 1-Pentylthioheptyl, 1-Butylthioheptyl, 1-Propylheptyl, 1-Ethylthioheptyl, 1-Methylthioheptyl, 1-Heptyloctyl, 1-Hexylthiooctyl, 1-Pentylthiooctyl, 1-Butylthiooctyl, 1-Propyloctyl, 1-Ethylthiooctyl, 1-Methylthiooctyl, 1-Octylthiononyl, 1-Heptylnonyl, 1-Hexylthiononyl, 1-Pentylthiononyl, 1-Butylthiononyl, 1-Propylnonyl, 1-Ethylthiononyl, 1-Methylthiononyl, 1-Nonylthiodecyl, 1-Octylthiodecyl, 1-Heptyldecyl, 1-Hexylthiodecyl, 1-Pentylthiodecyl, 1-Butylthiodecyl, 1-Propyldecyl, 1-Ethylthiodecyl, 1-Methylthiodecyl, 1-Decylthioundecyl, 1-Nonylthioundecyl, 1-Octylthioundecyl, 1-Heptylundecyl, 1-Hexylthioundecyl, 1-Pentylthioundecyl, 1-Butylthioundecyl, 1-Propylundecyl, 1-Ethylthioundecyl, 1-Methylthioundecyl, 1-Undecylthiododecyl, 1-Decylthiododecyl, 1-Nonylthiododecyl, 1-Octylthiododecyl, 1-Heptyldodecyl, 1-Hexylthiododecyl, 1-Pentylthiododecyl, 1-Butylthiododecyl, 1-Propyldodecyl, 1-Ethylthiododecyl, 1-Methylthiododecyl, 1-Dodecylthiotridecyl, 1-Undecylthiotridecyl, 1-Decylthiotridecyl, 1-Nonylthiotridecyl, 1-Octylthiotridecyl, 1-Heptyltridecyl, 1-Hexylthiotridecyl, 1-Pentylthiotridecyl, 1-Butylthiotridecyl, 1-Propyltridecyl, 1-Ethylthiotridecyl, 1-Methylthiotridecyl, 1-Tridecylthiotetrdecyl, 1-Undecylthiotetradecyl, 1-Decylthiotetradecyl, 1-Nonylthiotetradecyl, 1-Octylthiotetradecyl, 1-Hetyltetradecyl, 1-Hexylthiotetradecyl, 1-Pentytetradecyl, 1-Butylthiotetradecyl, 1-Propyltetradecyl, 1-Ethylthiotetradecyl, 1-Methylthiotetradecyl, 1-Pentadecylthiohexadecyl, 1-Tetradecylthiohexadecyl, 1-Tridecylthiohexadecyl, 1-Dodecylthiohexadecyl, 1-Undecylthiohexadecyl, 1-Decylthiohexadecyl, 1-Nonylthiohexadecyl, 1-Octylthiohexadecyl, 1-Heptylhexadecyl, 1-Hexylthiohexadecyl, 1-Pentylthiohexadecyl, 1-Butylthiohexadecyl, 1-Propylhexadecyl, 1-Ethylthiohexadecyl, 1-Methylthiohexadecyl, 1-Hexadecylthiooctadecyl, 1-Pentadecylthiooctadecyl, 1-Tetradecylthiooctadecyl, 1-Tridecylthiooctadecyl, 1-Dodecylthiooctadecyl, 1-Undecylthiooctadecyl, 1-Decylthiooctadecyl, 1-Nonylthiooctadecyl, 1-Octylthioocatedecyl, 1-Heptyloctadecyl, 1-Hexylthiooctadecyl, 1-Pentylthiooctadecyl, 1-Butylthiooctaedecyl, 1-Propyloctadecyl, 1-Ethylthiooctadecyl, 1-Methylthiooctadecyl 1-nonadecylthioeicosanyl, 1-Octadecylthioeicosanyl, 1-Heptadecylthioeicosanyl, 1-Hexadecylthioeicosanyl, 1-Pentadecylthioeicosanyl, 1-Tetradecylthioeicosanyl, 1-Tridecylthioeicosanyl, 1-Dodecylthioeicosanyl, 1-Undecylthioeicosanyl, 1-Decylthioeicosanyl, 1-Nonylthioeicosanyl, 1-Octylthioeicosanyl, 1-Heptyleicosanyl, 1-Hexylthioeicosanyl, 1-Pentylthioeicosanyl, 1-Butylthioeicosanyl, 1-Propyleicosanyl, 1-Ethylthioeicosanyl, 1-Methyeicosanyl 1-Eicosanylthiodocosanyl, 1-nonadecylthiodocosanyl, 1-Octadecylthiodocosanyl, 1-Heptadecylthiodocosanyl, 1-Hexadecylthiodocosanyl, 1-Pentadecylthiodocosanyl, 1-Tetradecylthiodocosanyl, 1-Tridecylthiodocosanyl, 1-Undecylthiodocosanyl, 1-Decylthiodocosanyl, 1-Nonylthiodocosanyl, 1-Octylthiodocosanyl, 1-Heptyldocosanyl, 1-Hexylthiodocosanyl, 1-Pentylthiodocosanyl, 1-Butylthiodocosanyl, 1-Propyldocosanyl, 1-Ethylthiodocosanyl, 1-Methylthiodocosanyl 1-Tricosanylthiotetracosanyl, 1-Docosanylthiotetracosanyl, 1-nonadecylthiotetracosanyl, 1-Octadecylthiotetracosanyl, 1-Heptadecylthiotetracosanyl, 1-Hexadecylthiotetracosanyl, 1-Pentadecylthiotetracosanyl, 1-Pentadecylthiotetracosanyl, 1-Tetradecylthiotetracosanyl, 1-Tridecylthiotetracosanyl, 1-Dodecylthiotetracosanyl, 1-Undecylthiotetracosanyl, 1-Decylthiotetracosanyl, 1-Nonylthiotetracosanyl, 1-Octylthiotetracosanyl, 1-Heptyltetracosanyl, 1-Hexylthiotetracosanyl, 1-Pentylthiotetracosanyl, 1-Butylthiotetracosanyl, 1-Propyltetracosanyl, 1-Ethylthiotetracosanyl, 1-Methylthiotetracosanyl 1-Heptacosanylthiooctacosanyl, 1-Hexacosanylthiooctacosanyl, 1-Pentcosanylthiooctacosanyl, 1-Tetracosanylthiooctcosanyl, 1-Tricosanylthiooctacosanyl, 1-Docosanylthiooctacosanyl, 1-Nonadecylthiooctacosanyl, 1-Octadecylthiooctacosanyl, 1-Heptadecylthiooctacosanyl, 1-Hexadecylthiooctacosanyl, 1-Hexadecylthiooctacosanyl, 1-Pentadecylthiooctacosanyl, 1-Tetradecylthiooctacosanyl, 1-Tridecylthiooctacosanyl, 1-Dodecylthiooctacosanyl, 1-Undecylthiooctacosanyl, 1-Decylthiooctacosanyl, 1-Nonylthiooctacosanyl, 1-Octylthiooctacosanyl, 1-Heptyloctacosanyl, 1-Hexylthiooctacosanyl, 1-Pentylthiooctacosanyl, 1-Butylthiooctacosanyl, 1-Propylthiooctacosanyl, 1-Ethylthiooctacosanyl, 1-Methylthiooctacosanyl;
in denen q für 1 steht, wie z. B.
2-Ethylpropyl, 2-Methylpropyl, 2-Propylbutyl, 2-Ethylbutyl, 2-Methylbutyl, 2-Butylpentyl, 2-Propylpentyl, 2-Ethylpentyl, 2-Methylpentyl, 2-Pentylhexyl, 2-Butylhexyl, 2-Propylhexyl, 2-Ethylhexyl, 2-Methylhexyl, 2-Hexylheptyl, 2-Pentylheptyl, 2-Butylheptyl, 2-Propylheptyl, 2-Ethylheptyl, 2-Methylheptyl, 2-Heptyloctyl, 2-Hexyloctyl, 2-Pentyloctyl, 2-Butyloctyl, 2-Propyloctyl, 2-Ethyloctyl, 2-Methyloctyl, 2-Octylnonyl, 2-Heptylnonyl, 2-Hexylnonyl, 2-Pentylnonyl, 2-Butylnonyl, 2-Propylnonyl, 2-Ethylnonyl, 2-Methylnonyl, 2-Nonyldecyl, 2-Octyldecyl, 2-Heptyldecyl, 2-Hexyldecyl, 2-Pentyldecyl, 2-Butyldecyl, 2-Propyldecyl, 2-Ethyldecyl, 2-Methyldecyl, 2-Decylundecyl, 2-Nonylundecyl, 2-Octylundecyl, 2-Heptylundecyl, 2-Hexylundecyl, 2-Pentylundecyl, 2-Butylundecyl, 2-Propylundecyl, 2-Ethylundecyl, 2-Methylundecyl, 2-Undecyldodecyl, 2-Decyldodecyl, 2-Nonyldodecyl, 2-Octyldodecyl, 2-Heptyldodecyl, 2-Hexyldodecyl, 2-Pentyldodecyl, 2-Butyldodecyl, 2-Propyldodecyl, 2-Ethyldodecyl, 2-Methyldodecyl, 2-Dodecyltridecyl, 2-Undecyltridecyl, 2-Decyltridecyl, 2-Nonyltridecyl, 2-Octyltridecyl, 2-Heptyltridecyl, 2-Hexyltridecyl, 2-Pentyltridecyl, 2-Butyltridecyl, 2-Propyltridecyl, 2-Ethyltridecyl, 2-Methyltridecyl, 2-Tridecyltetrdecyl, 2-Undecyltetradecyl, 2-Decyltetradecyl, 2-Nonyltetradecyl, 2-Octyltetradecyl, 2-Hetyltetradecyl, 2-Hexyltetradecyl, 2-Pentytetradecyl, 2-Butyltetradecyl, 2-Propyltetradecyl, 2-Ethyltetradecyl, 2-Methyltetradecyl, 2-Pentadecylhexadecyl, 2-Tetradecylhexadecyl, 2-Tridecylhexadecyl, 2-Dodecylhexadecyl, 2-Undecylhexadecyl, 2-Decylhexadecyl, 2-Nonylhexadecyl, 2-Octylhexadecyl, 2-Heptylhexadecyl, 2-Hexylhexadecyl, 2-Pentylhexadecyl, 2-Butylhexadecyl, 2-Propylhexadecyl, 2-Ethylhexadecyl, 2-Methylhexadecyl, 2-Hexadecyloctadecyl, 2-Pentadecyloctadecyl, 2-Tetradecyloctadecyl, 2-Tridecyloctadecyl, 2-Dodecyloctadecyl, 2-Undecyloctadecyl, 2-Decyloctadecyl, 2-Nonyloctadecyl, 2-Octylocatedecyl, 2-Heptyloctadecyl, 2-Hexyloctadecyl, 2-Pentyloctadecyl, 2-Butyloctaedecyl, 2-Propyloctadecyl, 2-Ethyloctadecyl, 2-Methyloctadecyl 2-Nonadecyleicosanyl, 2-Octadecyleicosanyl, 2-Heptadecyleicosanyl, 2-Hexadecyleicosanyl, 2-Pentadecyleicosanyl, 2-Tetradecyleicosanyl, 2-Tridecyleicosanyl, 2-Dodecyleicosanyl, 2-Undecyleicosanyl, 2-Decyleicosanyl, 2-Nonyleicosanyl, 2-Octyleicosanyl, 2-Heptyleicosanyl, 2-Hexyleicosanyl, 2-Pentyleicosanyl, 2-Butyleicosanyl, 2-Propyleicosanyl, 2-Ethyleicosanyl, 2-Methyeicosanyl 2-Eicosanyldocosanyl, 2-Nonadecyldocosanyl, 2-Octadecyldocosanyl, 2-Heptadecyldocosanyl, 2-Hexadecyldocosanyl, 2-Pentadecyldocosanyl, 2-Tetradecyldocosanyl, 2-Tridecyldocosanyl, 2-Undecyldocosanyl, 2-Decyldocosanyl, 2-Nonyldocosanyl, 2-Octyldocosanyl, 2-Heptyldocosanyl, 2-Hexyldocosanyl, 2-Pentyldocosanyl, 2-Butyldocosanyl, 2-Propyldocosanyl, 2-Ethyldocosanyl, 2-Methyldocosanyl 2-Tricosanyltetracosanyl, 2-Docosanyltetracosanyl, 2-Nonadecyltetracosanyl, 2-Octadecyltetracosanyl, 2-Heptadecyltetracosanyl, 2-Hexadecyltetracosanyl, 2-Pentadecyltetracosanyl, 2-Pentadecyltetracosanyl, 2-Tetradecyltetracosanyl, 2-Tridecyltetracosanyl, 2-Dodecyltetracosanyl, 2-Undecyltetracosanyl, 2-Decyltetracosanyl, 2-Nonyltetracosanyl, 2-Octyltetracosanyl, 2-Heptyltetracosanyl, 2-Hexyltetracosanyl, 2-Pentyltetracosanyl, 2-Butyltetracosanyl, 2-Propyltetracosanyl, 2-Ethyltetracosanyl, 2-Methyltetracosanyl 2-Heptacosanyloctacosanyl, 2-Hexacosanyloctacosanyl, 2-Pentcosanyloctacosanyl, 2-Tetracosanyloctcosanyl, 2-Tricosanyloctacosanyl, 2-Docosanyloctacosanyl, 2-Nonadecyloctacosanyl, 2-Octadecyloctacosanyl, 2-Heptadecyloctacosanyl, 2-Hexadecyloctacosanyl, 2-Hexadecyloctacosanyl, 2-Pentadecyloctacosanyl, 2-Tetradecyloctacosanyl, 2-Tridecyloctacosanyl, 2-Dodecyloctacosanyl, 2-Undecyloctacosanyl, 2-Decyloctacosanyl, 2-Nonyloctacosanyl, 2-Octyloctacosanyl, 2-Heptyloctacosanyl, 2-Hexyloctacosanyl, 2-Pentyloctacosanyl, 2-Butyloctacosanyl, 2-Propyloctacosanyl, 2-Ethyloctacosanyl, 2-Methyloctacosanyl;
in denen q für 2 steht, wie z. B.
3-Ethylpropyl, 3-Methylpropyl, 3-Propylbutyl, 3-Ethylbutyl, 3-Methylbutyl, 3-Butylpentyl, 3-Propylpentyl, 3-Ethylpentyl, 3-Methylpentyl, 3-Pentylhexyl, 3-Butylhexyl, 3-Propylhexyl, 3-Ethylhexyl, 3-Methylhexyl, 3-Hexylheptyl, 3-Pentylheptyl, 3-Butylheptyl, 3-Propylheptyl, 3-Ethylheptyl, 3-Methylheptyl, 3-Heptyloctyl, 3-Hexyloctyl, 3-Pentyloctyl, 3-Butyloctyl, 3-Propyloctyl, 3-Ethyloctyl, 3-Methyloctyl, 3-Octylnonyl, 3-Heptylnonyl, 3-Hexylnonyl, 3-Pentylnonyl, 3-Butylnonyl, 3-Propylnonyl, 3-Ethylnonyl, 3-Methylnonyl, 3-Nonyldecyl, 3-Octyldecyl, 3-Heptyldecyl, 3-Hexyldecyl, 3-Pentyldecyl, 3-Butyldecyl, 3-Propyldecyl, 3-Ethyldecyl, 3-Methyldecyl, 3-Decylundecyl, 3-Nonylundecyl, 3-Octylundecyl, 3-Heptylundecyl, 3-Hexylundecyl, 3-Pentylundecyl, 3-Butylundecyl, 3-Propylundecyl, 3-Ethylundecyl, 3-Methylundecyl, 3-Undecyldodecyl, 3-Decyldodecyl, 3-Nonyldodecyl, 3-Octyldodecyl, 3-Heptyldodecyl, 3-Hexyldodecyl, 3-Pentyldodecyl, 3-Butyldodecyl, 3-Propyldodecyl, 3-Ethyldodecyl, 3-Methyldodecyl, 3-Dodecyltridecyl, 3-Undecyltridecyl, 3-Decyltridecyl, 3-Nonyltridecyl, 3-Octyltridecyl, 3-Heptyltridecyl, 3-Hexyltridecyl, 3-Pentyltridecyl, 3-Butyltridecyl, 3-Propyltridecyl, 3-Ethyltridecyl, 3-Methyltridecyl, 3-Tridecyltetrdecyl, 3-Undecyltetradecyl, 3-Decyltetradecyl, 3-Nonyltetradecyl, 3-Octyltetradecyl, 3-Hetyltetradecyl, 3-Hexyltetradecyl, 3-Pentytetradecyl, 3-Butyltetradecyl, 3-Propyltetradecyl, 3-Ethyltetradecyl, 3-Methyltetradecyl, 3-Pentadecylhexadecyl, 3-Tetradecylhexadecyl, 3-Tridecylhexadecyl, 3-Dodecylhexadecyl, 3-Undecylhexadecyl, 3-Decylhexadecyl, 3-Nonylhexadecyl, 3-Octylhexadecyl, 3-Heptylhexadecyl, 3-Hexylhexadecyl, 3-Pentylhexadecyl, 3-Butylhexadecyl, 3-Propylhexadecyl, 3-Ethylhexadecyl, 3-Methylhexadecyl, 3-Hexadecyloctadecyl, 3-Pentadecyloctadecyl, 3-Tetradecyloctadecyl, 3-Tridecyloctadecyl, 3-Dodecyloctadecyl, 3-Undecyloctadecyl, 3-Decyloctadecyl, 3-Nonyloctadecyl, 3-Octylocatedecyl, 3-Heptyloctadecyl, 3-Hexyloctadecyl, 3-Pentyloctadecyl, 3-Butyloctaedecyl, 3-Propyloctadecyl, 3-Ethyloctadecyl, 3-Methyloctadecyl 3-Nonadecyleicosanyl, 3-Octadecyleicosanyl, 3-Heptadecyleicosanyl, 3-Hexadecyleicosanyl, 3-Pentadecyleicosanyl, 3-Tetradecyleicosanyl, 3-Tridecyleicosanyl, 3-Dodecyleicosanyl, 3-Undecyleicosanyl, 3-Decyleicosanyl, 3-Nonyleicosanyl, 3-Octyleicosanyl, 3-Heptyleicosanyl, 3-Hexyleicosanyl, 3-Pentyleicosanyl, 3-Butyleicosanyl, 3-Propyleicosanyl, 3-Ethyleicosanyl, 3-Methyeicosanyl 3-Eicosanyldocosanyl, 3-Nonadecyldocosanyl, 3-Octadecyldocosanyl, 3-Heptadecyldocosanyl, 3-Hexadecyldocosanyl, 3-Pentadecyldocosanyl, 3-Tetradecyldocosanyl, 3-Tridecyldocosanyl, 3-Undecyldocosanyl, 3-Decyldocosanyl, 3-Nonyldocosanyl, 3-Octyldocosanyl, 3-Heptyldocosanyl, 3-Hexyldocosanyl, 3-Pentyldocosanyl, 3-Butyldocosanyl, 3-Propyldocosanyl, 3-Ethyldocosanyl, 3-Methyldocosanyl 3-Tricosanyltetracosanyl, 3-Docosanyltetracosanyl, 3-Nonadecyltetracosanyl, 3-Octadecyltetracosanyl, 3-Heptadecyltetracosanyl, 3-Hexadecyltetracosanyl, 3-Pentadecyltetracosanyl, 3-Pentadecyltetracosanyl, 3-Tetradecyltetracosanyl, 3-Tridecyltetracosanyl, 3-Dodecyltetracosanyl, 3-Undecyltetracosanyl, 3-Decyltetracosanyl, 3-Nonyltetracosanyl, 3-Octyltetracosanyl, 3-Heptyltetracosanyl, 3-Hexyltetracosanyl, 3-Pentyltetracosanyl, 3-Butyltetracosanyl, 3-Propyltetracosanyl, 3-Ethyltetracosanyl, 3-Methyltetracosanyl 3-Heptacosanyloctacosanyl, 3-Hexacosanyloctacosanyl, 3-Pentcosanyloctacosanyl, 3-Tetracosanyloctcosanyl, 3-Tricosanyloctacosanyl, 3-Docosanyloctacosanyl, 3-Nonadecyloctacosanyl, 3-Octadecyloctacosanyl, 3-Heptadecyloctacosanyl, 3-Hexadecyloctacosanyl, 3-Hexadecyloctacosanyl, 3-Pentadecyloctacosanyl, 3-Tetradecyloctacosanyl, 3-Tridecyloctacosanyl, 3-Dodecyloctacosanyl, 3-Undecyloctacosanyl, 3-Decyloctacosanyl, 3-Nonyloctacosanyl, 3-Octyloctacosanyl, 3-Heptyloctacosanyl, 3-Hexyloctacosanyl, 3-Pentyloctacosanyl, 3-Butyloctacosanyl, 3-Propyloctacosanyl, 3-Ethyloctacosanyl, 3-Methyloctacosanyl,
in denen q für 3 steht, wie
4-Butylpentyl, 4-Propylpentyl, 4-Ethylpentyl, 4-Methylpentyl, 4-Pentylhexyl, 4-Butylhexyl, 4-Propylhexyl, 4-Ethylhexyl, 4-Methylhexyl, 4-Hexylheptyl, 4-Pentylheptyl, 4-Butylheptyl, 4-Propylheptyl, 4-Ethylheptyl, 4-Methylheptyl, 4-Heptyloctyl, 4-Hexyloctyl, 4-Pentyloctyl, 4-Butyloctyl, 4-Propyloctyl, 4-Ethyloctyl, 4-Methyloctyl, 4-Octylnonyl, 4-Heptylnonyl, 4-Hexyinonyl, 4-Pentylnonyl, 4-Butylnonyl, 4-Propylnonyl, 4-Ethylnonyl, 4-Methylnonyl, 4-Nonyldecyl, 4-Octyldecyl, 4-Heptyldecyl, 4-Hexyldecyl, 4-Pentyldecyl, 4-Butyldecyl, 4-Propyldecyl, 4-Ethyldecyl, 4-Methyldecyl, 4-Decylundecyl, 4-Nonylundecyl, 4-Octylundecyl, 4-Heptylundecyl, 4-Hexylundecyl, 4-Pentylundecyl, 4-Butylundecyl, 4-Propylundecyl, 4-Ethylundecyl, 4-Methylundecyl, 4-Undecyldodecyl, 4-Decyldodecyl, 4-Nonyldodecyl, 4-Octyldodecyl, 4-Heptyldodecyl, 4-Hexyldodecyl, 4-Pentyldodecyl, 4-Butyldodecyl, 4-Propyldodecyl, 4-Ethyldodecyl, 4-Methyldodecyl, 4-Dodecyltridecyl, 4-Undecyltridecyl, 4-Decyltridecyl, 4-Nonyltridecyl, 4-Octyltridecyl, 4-Heptyltridecyl, 4-Hexyltridecyl, 4-Pentyltridecyl, 4-Butyltridecyl, 4-Propyltridecyl, 4-Ethyltridecyl, 4-Methyltridecyl, 4-Tridecyltetrdecyl, 4-Undecyltetradecyl, 4-Decyltetradecyl, 4-Nonyltetradecyl, 4-Octyltetradecyl, 4-Hetyltetradecyl, 4-Hexyltetradecyl, 4-Pentytetradecyl, 4-Butyltetradecyl, 4-Propyltetradecyl, 4-Ethyltetradecyl, 4-Methyltetradecyl, 4-Pentadecylhexadecyl, 4-Tetradecylhexadecyl, 4-Tridecylhexadecyl, 4-Dodecylhexadecyl, 4-Undecylhexadecyl, 4-Decylhexadecyl, 4-Nonylhexadecyl, 4-Octylhexadecyl, 4-Heptylhexadecyl, 4-Hexylhexadecyl, 4-Pentylhexadecyl, 4-Butylhexadecyl, 4-Propylhexadecyl, 4-Ethylhexadecyl, 4-Methylhexadecyl, 4-Hexadecyloctadecyl, 4-Pentadecyloctadecyl, 4-Tetradecyloctadecyl, 4-Tridecyloctadecyl, 4-Dodecyloctadecyl, 4-Undecyloctadecyl, 4-Decyloctadecyl, 4-Nonyloctadecyl, 4-Octylocatedecyl, 4-Heptyloctadecyl, 4-Hexyloctadecyl, 4-Pentyloctadecyl, 4-Butyloctaedecyl, 4-Propyloctadecyl, 4-Ethyloctadecyl, 4-Methyloctadecyl 4-Nonadecyleicosanyl, 4-Octadecyleicosanyl, 4-Heptadecyleicosanyl, 4-Hexadecyleicosanyl, 4-Pentadecyleicosanyl, 4-Tetradecyleicosanyl, 4-Tridecyleicosanyl, 4-Dodecyleicosanyl, 4-Undecyleicosanyl, 4-Decyleicosanyl, 4-Nonyleicosanyl, 4-Octyleicosanyl, 4-Heptyleicosanyl, 4-Hexyleicosanyl, 4-Pentyleicosanyl, 4-Butyleicosanyl, 4-Propyleicosanyl, 4-Ethyleicosanyl, 4-Methyeicosanyl 4-Eicosanyldocosanyl, 4-Nonadecyldocosanyl, 4-Octadecyldocosanyl, 4-Heptadecyldocosanyl, 4-Hexadecyldocosanyl, 4-Pentadecyldocosanyl, 4-Tetradecyldocosanyl, 4-Tridecyldocosanyl, 4-Undecyldocosanyl, 4-Decyldocosanyl, 4-Nonyldocosanyl, 4-Octyldocosanyl, 4-Heptyldocosanyl, 4-Hexyldocosanyl, 4-Pentyldocosanyl, 4-Butyldocosanyl, 4-Propyldocosanyl, 4-Ethyldocosanyl, 4-Methyldocosanyl 4-Tricosanyltetracosanyl, 4-Docosanyltetracosanyl, 4-Nonadecyltetracosanyl, 4-Octadecyltetracosanyl, 4-Heptadecyltetracosanyl, 4-Hexadecyltetracosanyl, 4-Pentadecyltetracosanyl, 4-Pentadecyltetracosanyl, 4-Tetradecyltetracosanyl, 4-Tridecyltetracosanyl, 4-Dodecyltetracosanyl, 4-Undecyltetracosanyl, 4-Decyltetracosanyl, 4-Nonyltetracosanyl, 4-Octyltetracosanyl, 4-Heptyltetracosanyl, 4-Hexyltetracosanyl, 4-Pentyltetracosanyl, 4-Butyltetracosanyl, 4-Propyltetracosanyl, 4-Ethyltetracosanyl, 4-Methyltetracosanyl 4-Heptacosanyloctacosanyl, 4-Hexacosanyloctacosanyl, 4-Pentcosanyloctacosanyl, 4-Tetracosanyloctcosanyl, 4-Tricosanyloctacosanyl, 4-Docosanyloctacosanyl, 4-Nonadecyloctacosanyl, 4-Octadecyloctacosanyl, 4-Heptadecyloctacosanyl, 4-Hexadecyloctacosanyl, 4-Hexadecyloctacosanyl, 4-Pentadecyloctacosanyl, 4-Tetradecyloctacosanyl, 4-Tridecyloctacosanyl, 4-Dodecyloctacosanyl, 4-Undecyloctacosanyl, 4-Decyloctacosanyl, 4-Nonyloctacosanyl, 4-Octyloctacosanyl, 4-Heptyloctacosanyl, 4-Hexyloctacosanyl, 4-Pentyloctacosanyl, 4-Butyloctacosanyl, 4-Propyloctacosanyl, 4-Ethyloctacosanyl, 4-Methyloctacosanyl,
in denen q für 4 steht, wie
5-Pentylhexyl, 5-Butylhexyl, 5-Propylhexyl, 5-Ethylhexyl, 5-Methylhexyl, 5-Hexylheptyl, 5-Pentylheptyl, 5-Butylheptyl, 5-Propylheptyl, 5-Ethylheptyl, 5-Methylheptyl, 5-Heptyloctyl, 5-Hexyloctyl, 5-Pentyloctyl, 5-Butyloctyl, 5-Propyloctyl, 5-Ethyloctyl, 5-Methyloctyl, 5-Octylnonyl, 5-Heptylnonyl, 5-Hexylnonyl, 5-Pentylnonyl, 5-Butylnonyl, 5-Propylnonyl, 5-Ethylnonyl, 5-Methylnonyl, 5-Nonyldecyl, 5-Octyldecyl, 5-Heptyldecyl, 5-Hexyldecyl, 5-Pentyldecyl, 5-Butyldecyl, 5-Propyldecyl, 5-Ethyldecyl, 5-Methyldecyl, 5-Decylundecyl, 5-Nonylundecyl, 5-Octylundecyl, 5-Heptylundecyl, 5-Hexylundecyl, 5-Pentylundecyl, 5-Butylundecyl, 5-Propylundecyl, 5-Ethylundecyl, 5-Methylundecyl, 5-Undecyldodecyl, 5-Decyldodecyl, 5-Nonyldodecyl, 5-Octyldodecyl, 5-Heptyldodecyl, 5-Hexyldodecyl, 5-Pentyldodecyl, 5-Butyldodecyl, 5-Propyldodecyl, 5-Ethyldodecyl, 5-Methyldodecyl, 5-Dodecyltridecyl, 5-Undecyltridecyl, 5-Decyltridecyl, 5-Nonyltridecyl, 5-Octyltridecyl, 5-Heptyltridecyl, 5-Hexyltridecyl, 5-Pentyltridecyl, 5-Butyltridecyl, 5-Propyltridecyl, 5-Ethyltridecyl, 5-Methyltridecyl, 5-Tridecyltetrdecyl, 5-Undecyltetradecyl, 5-Decyltetradecyl, 5-Nonyltetradecyl, 5-Octyltetradecyl, 5-Hetyltetradecyl, 5-Hexyltetradecyl, 5-Pentytetradecyl, 5-Butyltetradecyl, 5-Propyltetradecyl, 5-Ethyltetradecyl, 5-Methyltetradecyl, 5-Pentadecylhexadecyl, 5-Tetradecylhexadecyl, 5-Tridecylhexadecyl, 5-Dodecylhexadecyl, 5-Undecylhexadecyl, 5-Decylhexadecyl, 5-Nonylhexadecyl, 5-Octylhexadecyl, 5-Heptylhexadecyl, 5-Hexylhexadecyl, 5-Pentylhexadecyl, 5-Butylhexadecyl, 5-Propylhexadecyl, 5-Ethylhexadecyl, 5-Methylhexadecyl, 5-Hexadecyloctadecyl, 5-Pentadecyloctadecyl, 5-Tetradecyloctadecyl, 5-Tridecyloctadecyl, 5-Dodecyloctadecyl, 5-Undecyloctadecyl, 5-Decyloctadecyl, 5-Nonyloctadecyl, 5-Octylocatedecyl, 5-Heptyloctadecyl, 5-Hexyloctadecyl, 5-Pentyloctadecyl, 5-Butyloctaedecyl, 5-Propyloctadecyl, 5-Ethyloctadecyl, 5-Methyloctadecyl 5-Nonadecyleicosanyl, 5-Octadecyleicosanyl, 5-Heptadecyleicosanyl, 5-Hexadecyleicosanyl, 5-Pentadecyleicosanyl, 5-Tetradecyleicosanyl, 5-Tridecyleicosanyl, 5-Dodecyleicosanyl, 5-Undecyleicosanyl, 5-Decyleicosanyl, 5-Nonyleicosanyl, 5-Octyleicosanyl, 5-Heptyleicosanyl, 5-Hexyleicosanyl, 5-Pentyleicosanyl, 5-Butyleicosanyl, 5-Propyleicosanyl, 5-Ethyleicosanyl, 5-Methyeicosanyl, 5-Eicosanyldocosanyl, 5-Nonadecyldocosanyl, 5-Octadecyldocosanyl, 5-Heptadecyldocosanyl, 5-Hexadecyldocosanyl, 5-Pentadecyldocosanyl, 5-Tetradecyldocosanyl, 5-Tridecyldocosanyl, 5-Undecyldocosanyl, 5-Decyldocosanyl, 5-Nonyldocosanyl, 5-Octyldocosanyl, 5-Heptyldocosanyl, 5-Hexyldocosanyl, 5-Pentyldocosanyl, 5-Butyldocosanyl, 5-Propyldocosanyl, 5-Ethyldocosanyl, 5-Methyldocosanyl 5-Tricosanyltetracosanyl, 5-Docosanyltetracosanyl, 5-Nonadecyltetracosanyl, 5-Octadecyltetracosanyl, 5-Heptadecyltetracosanyl, 5-Hexadecyltetracosanyl, 5-Pentadecyltetracosanyl, 5-Pentadecyltetracosanyl, 5-Tetradecyltetracosanyl, 5-Tridecyltetracosanyl, 5-Dodecyltetracosanyl, 5-Undecyltetracosanyl, 5-Decyltetracosanyl, 5-Nonyltetracosanyl, 5-Octyltetracosanyl, 5-Heptyltetracosanyl, 5-Hexyltetracosanyl, 5-Pentyltetracosanyl, 5-Butyltetracosanyl, 5-Propyltetracosanyl, 5-Ethyltetracosanyl, 5-Methyltetracosanyl 5-Heptacosanyloctacosanyl, 5-Hexacosanyloctacosanyl, 5-Pentcosanyloctacosanyl, 5-Tetracosanyloctcosanyl, 5-Tricosanyloctacosanyl, 5-Docosanyloctacosanyl, 5-Nonadecyloctacosanyl, 5-Octadecyloctacosanyl, 5-Heptadecyloctacosanyl, 5-Hexadecyloctacosanyl, 5-Hexadecyloctacosanyl, 5-Pentadecyloctacosanyl, 5-Tetradecyloctacosanyl, 5-Tridecyloctacosanyl, 5-Dodecyloctacosanyl, 5-Undecyloctacosanyl, 5-Decyloctacosanyl, 5-Nonyloctacosanyl, 5-Octyloctacosanyl, 5-Heptyloctacosanyl, 5-Hexyloctacosanyl, 5-Pentyloctacosanyl, 5-Butyloctacosanyl, 5-Propyloctacosanyl, 5-Ethyloctacosanyl, 5-Methyloctacosanyl
in denen q für 5 steht, wie z. B.
6-Hexylheptyl, 6-Pentylheptyl, 6-Butylheptyl, 6-Propylheptyl, 6-Ethylheptyl, 6-Methylheptyl, 6-Heptyloctyl, 6-Hexyloctyl, 6-Pentyloctyl, 6-Butyloctyl, 6-Propyloctyl, 6-Ethyloctyl, 6-Methyloctyl, 6-Octylnonyl, 6-Heptylnonyl, 6-Hexylnonyl, 6-Pentylnonyl, 6-Butylnonyl, 6-Propylnonyl, 6-Ethylnonyl, 6-Methylnonyl, 6-Nonyldecyl, 6-Octyldecyl, 6-Heptyldecyl, 6-Hexyldecyl, 6-Pentyldecyl, 6-Butyldecyl, 6-Propyldecyl, 6-Ethyldecyl, 6-Methyldecyl, 6-Decylundecyl, 6-Nonylundecyl, 6-Octylundecyl, 6-Heptylundecyl, 6-Hexylundecyl, 6-Pentylundecyl, 6-Butylundecyl, 6-Propylundecyl, 6-Ethylundecyl, 6-Methylundecyl, 6-Undecyldodecyl, 6-Decyldodecyl, 6-Nonyldodecyl, 6-Octyldodecyl, 6-Heptyldodecyl, 6-Hexyldodecyl, 6-Pentyldodecyl, 6-Butyldodecyl, 6-Propyldodecyl, 6-Ethyldodecyl, 6-Methyldodecyl, 6-Dodecyltridecyl, 6-Undecyltridecyl, 6-Decyltridecyl, 6-Nonyltridecyl, 6-Octyltridecyl, 6-Heptyltridecyl, 6-Hexyltridecyl, 6-Pentyltridecyl, 6-Butyltridecyl, 6-Propyltridecyl, 6-Ethyltridecyl, 6-Methyltridecyl, 6-Tridecyltetrdecyl, 6-Undecyltetradecyl, 6-Decyltetradecyl, 6-Nonyltetradecyl, 6-Octyltetradecyl, 6-Hetyltetradecyl, 6-Hexyltetradecyl, 6-Pentytetradecyl, 6-Butyltetradecyl, 6-Propyltetradecyl, 6-Ethyltetradecyl, 6-Methyltetradecyl, 6-Pentadecylhexadecyl, 6-Tetradecylhexadecyl, 6-Tridecylhexadecyl, 6-Dodecylhexadecyl, 6-Undecylhexadecyl, 6-Decylhexadecyl, 6-Nonylhexadecyl, 6-Octylhexadecyl, 6-Heptylhexadecyl, 6-Hexylhexadecyl, 6-Pentylhexadecyl, 6-Butylhexadecyl, 6-Propylhexadecyl, 6-Ethylhexadecyl, 6-Methylhexadecyl, 6-Hexadecyloctadecyl, 6-Pentadecyloctadecyl, 6-Tetradecyloctadecyl, 6-Tridecyloctadecyl, 6-Dodecyloctadecyl, 6-Undecyloctadecyl, 6-Decyloctadecyl, 6-Nonyloctadecyl, 6-Octylocatedecyl, 6-Heptyloctadecyl, 6-Hexyloctadecyl, 6-Pentyloctadecyl, 6-Butyloctaedecyl, 6-Propyloctadecyl, 6-Ethyloctadecyl, 6-Methyloctadecyl 6-Nonadecyleicosanyl, 6-Octadecyleicosanyl, 6-Heptadecyleicosanyl, 6-Hexadecyleicosanyl, 6-Pentadecyleicosanyl, 6-Tetradecyleicosanyl, 6-Tridecyleicosanyl, 6-Dodecyleicosanyl, 6-Undecyleicosanyl, 6-Decyleicosanyl, 6-Nonyleicosanyl, 6-Octyleicosanyl, 6-Heptyleicosanyl, 6-Hexyleicosanyl, 6-Pentyleicosanyl, 6-Butyleicosanyl, 6-Propyleicosanyl, 6-Ethyleicosanyl, 6-Methyeicosanyl, 6-Eicosanyldocosanyl, 6-Nonadecyldocosanyl, 6-Octadecyldocosanyl, 6-Heptadecyldocosanyl, 6-Hexadecyldocosanyl, 6-Pentadecyldocosanyl, 6-Tetradecyldocosanyl, 6-Tridecyldocosanyl, 6-Undecyldocosanyl, 6-Decyldocosanyl, 6-Nonyldocosanyl, 6-Octyldocosanyl, 6-Heptyldocosanyl, 6-Hexyldocosanyl, 6-Pentyldocosanyl, 6-Butyldocosanyl, 6-Propyldocosanyl, 6-Ethyldocosanyl, 6-Methyldocosanyl 6-Tricosanyltetracosanyl, 6-Docosanyltetracosanyl, 6-Nonadecyltetracosanyl, 6-Octadecyltetracosanyl, 6-Heptadecyltetracosanyl, 6-Hexadecyltetracosanyl, 6-Pentadecyltetracosanyl, 6-Pentadecyltetracosanyl, 6-Tetradecyltetracosanyl, 6-Tridecyltetracosanyl, 6-Dodecyltetracosanyl, 6-Undecyltetracosanyl, 6-Decyltetracosanyl, 6-Nonyltetracosanyl, 6-Octyltetracosanyl, 6-Heptyltetracosanyl, 6-Hexyltetracosanyl, 6-Pentyltetracosanyl, 6-Butyltetracosanyl, 6-Propyltetracosanyl, 6-Ethyltetracosanyl, 6-Methyltetracosanyl 6-Heptacosanyloctacosanyl, 6-Hexacosanyloctacosanyl, 6-Pentcosanyloctacosanyl, 6-Tetracosanyloctcosanyl, 6-Tricosanyloctacosanyl, 6-Docosanyloctacosanyl, 6-Nonadecyloctacosanyl, 6-Octadecyloctacosanyl, 6-Heptadecyloctacosanyl, 6-Hexadecyloctacosanyl, 6-Hexadecyloctacosanyl, 6-Pentadecyloctacosanyl, 6-Tetradecyloctacosanyl, 6-Tridecyloctacosanyl, 6-Dodecyloctacosanyl, 6-Undecyloctacosanyl, 6-Decyloctacosanyl, 6-Nonyloctacosanyl, 6-Octyloctacosanyl, 6-Heptyloctacosanyl, 6-Hexyloctacosanyl, 6-Pentyloctacosanyl, 6-Butyloctacosanyl, 6-Propyloctacosanyl, 6-Ethyloctacosanyl, 6-Methyloctacosanyl,
in denen q für 6 steht, wie z. B.
7-Heptyloctyl, 7-Hexyloctyl, 7-Pentyloctyl, 7-Butyloctyl, 7-Propyloctyl, 7-Ethyloctyl, 7-Methyloctyl, 7-Octylnonyl, 7-Heptylnonyl, 7-Hexylnonyl, 7-Pentylnonyl, 7-Butylnonyl, 7-Propylnonyl, 7-Ethylnonyl, 7-Methylnonyl, 7-Nonyldecyl, 7-Octyldecyl, 7-Heptyldecyl, 7-Hexyldecyl, 7-Pentyldecyl, 7-Butyldecyl, 7-Propyldecyl, 7-Ethyldecyl, 7-Methyldecyl, 7-Decylundecyl, 7-Nonylundecyl, 7-Octylundecyl, 7-Heptylundecyl, 7-Hexylundecyl, 7-Pentylundecyl, 7-Butylundecyl, 7-Propylundecyl, 7-Ethylundecyl, 7-Methylundecyl, 7-Undecyldodecyl, 7-Decyldodecyl, 7-Nonyldodecyl, 7-Octyldodecyl, 7-Heptyldodecyl, 7-Hexyldodecyl, 7-Pentyldodecyl, 7-Butyldodecyl, 7-Propyldodecyl, 7-Ethyldodecyl, 7-Methyldodecyl, 7-Dodecyltridecyl, 7-Undecyltridecyl, 7-Decyltridecyl, 7-Nonyltridecyl, 7-Octyltridecyl, 7-Heptyltridecyl, 7-Hexyltridecyl, 7-Pentyltridecyl, 7-Butyltridecyl, 7-Propyltridecyl, 7-Ethyltridecyl, 7-Methyltridecyl, 7-Tridecyltetrdecyl, 7-Undecyltetradecyl, 7-Decyltetradecyl, 7-Nonyltetradecyl, 7-Octyltetradecyl, 7-Hetyltetradecyl, 7-Hexyltetradecyl, 7-Pentytetradecyl, 7-Butyltetradecyl, 7-Propyltetradecyl, 7-Ethyltetradecyl, 7-Methyltetradecyl, 7-Pentadecylhexadecyl, 7-Tetradecylhexadecyl, 7-Tridecylhexadecyl, 7-Dodecylhexadecyl, 7-Undecylhexadecyl, 7-Decylhexadecyl, 7-Nonylhexadecyl, 7-Octylhexadecyl, 7-Heptylhexadecyl, 7-Hexylhexadecyl, 7-Pentylhexadecyl, 7-Butylhexadecyl, 7-Propylhexadecyl, 7-Ethylhexadecyl, 7-Methylhexadecyl, 7-Hexadecyloctadecyl, 7-Pentadecyloctadecyl, 7-Tetradecyloctadecyl, 7-Tridecyloctadecyl, 7-Dodecyloctadecyl, 7-Undecyloctadecyl, 7-Decyloctadecyl, 7-Nonyloctadecyl, 7-Octylocatedecyl, 7-Heptyloctadecyl, 7-Hexyloctadecyt, 7-Pentyloctadecyl, 7-Butyloctaedecyl, 7-Propyloctadecyl, 7-Ethyloctadecyl, 7-Methyloctadecyl 7-Nonadecyleicosanyl, 7-Octadecyleicosanyl, 7-Heptadecyleicosanyl, 7-Hexadecyleicosanyl, 7-Pentadecyleicosanyl, 7-Tetradecyleicosanyl, 7-Tridecyleicosanyl, 7-Dodecyleicosanyl, 7-Undecyleicosanyl, 7-Decyleicosanyl, 7-Nonyleicosanyl, 7-Octyleicosanyl, 7-Heptyleicosanyl, 7-Hexyleicosanyl, 7-Pentyleicosanyl, 7-Butyleicosanyl, 7-Propyleicosanyl, 7-Ethyleicosanyl, 7-Methyleicosanyl, 7-Eicosanyldocosanyl, 7-Nonadecyldocosanyl, 7-Octadecyldocosanyl, 7-Heptadecyldocosanyl, 7-Hexadecyldocosanyl, 7-Pentadecyldocosanyl, 7-Tetradecyldocosanyl, 7-Tridecyldocosanyl, 7-Undecyldocosanyl, 7-Decyldocosanyl, 7-Nonyldocosanyl, 7-Octyldocosanyl, 7-Heptyldocosanyl, 7-Hexyldocosanyl, 7-Pentyldocosanyl, 7-Butyldocosanyl, 7-Propyldocosanyl, 7-Ethyldocosanyl, 7-Methyldocosanyl 7-Tricosanyltetracosanyl, 7-Docosanyltetracosanyl, 7-Nonadecyltetracosanyl, 7-Octadecyltetracosanyl, 7-Heptadecyltetracosanyl, 7-Hexadecyltetracosanyl, 7-Pentadecyltetracosanyl, 7-Pentadecyltetracosanyl, 7-Tetradecyltetracosanyl, 7-Tridecyltetracosanyl, 7-Dodecyltetracosanyl, 7-Undecyltetracosanyl, 7-Decyltetracosanyl, 7-Nonyltetracosanyl, 7-Octyltetracosanyl, 7-Heptyltetracosanyl, 7-Hexyltetracosanyl, 7-Pentyltetracosanyl, 7-Butyltetracosanyl, 7-Propyltetracosanyl, 7-Ethyltetracosanyl, 7-Methyltetracosanyl 7-Heptacosanyloctacosanyl, 7-Hexacosanyloctacosanyl, 7-Pentcosanyloctacosanyl, 7-Tetracosanyloctcosanyl, 7-Tricosanyloctacosanyl, 7-Docosanyloctacosanyl, 7-Nonadecyloctacosanyl, 7-Octadecyloctacosanyl, 7-Heptadecyloctacosanyl, 7-Hexadecyloctacosanyl, 7-Hexadecyloctacosanyl, 7-Pentadecyloctacosanyl, 7-Tetradecyloctacosanyl, 7-Tridecyloctacosanyl, 7-Dodecyloctacosanyl, 7-Undecyloctacosanyl, 7-Decyloctacosanyl, 7-Nonyloctacosanyl, 7-Octyloctacosanyl, 7-Heptyloctacosanyl, 7-Hexyloctacosanyl, 7-Pentyloctacosanyl, 7-Butyloctacosanyl, 7-Propyloctacosanyl, 7-Ethyloctacosanyl, 7-Methyloctacosanyl.

Bevorzugt sind folgende Verbindungen

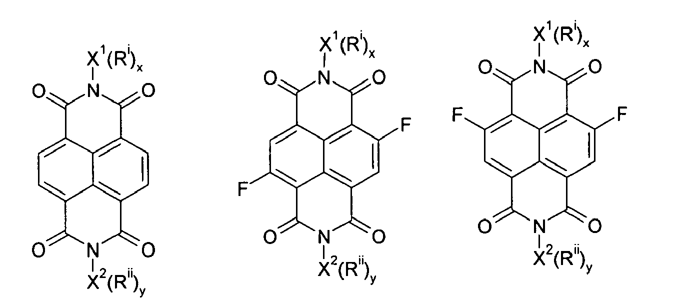

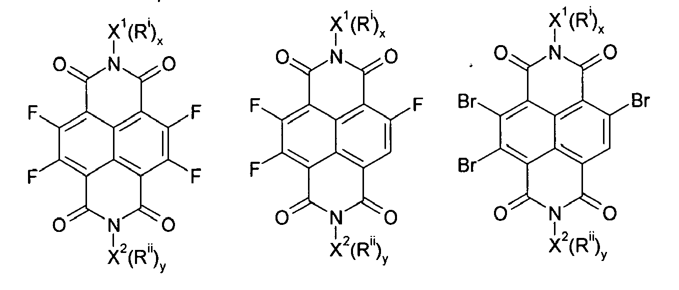

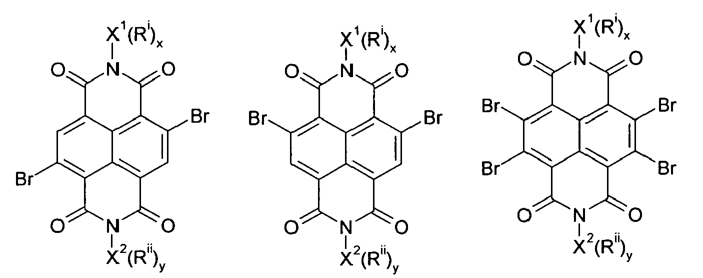

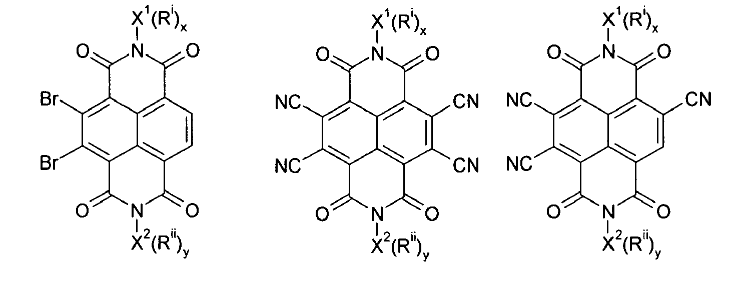

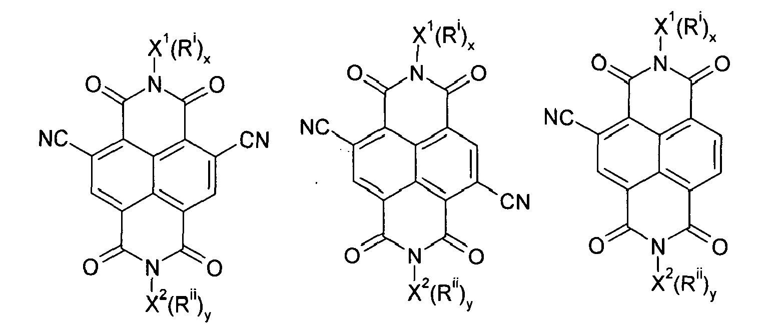

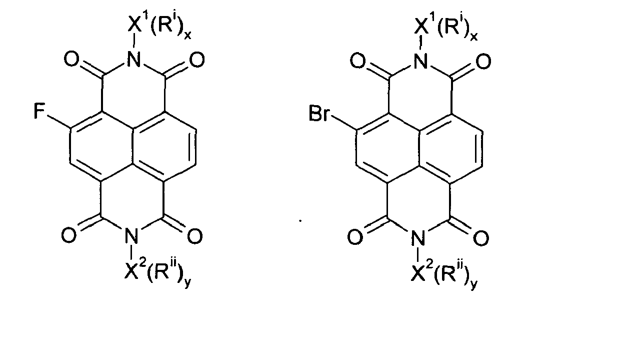

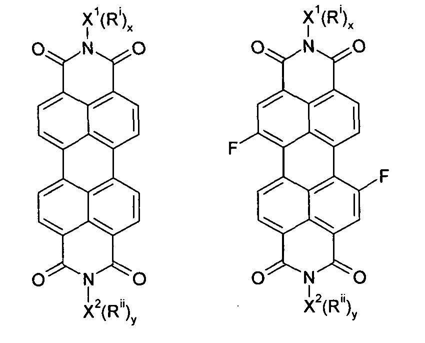

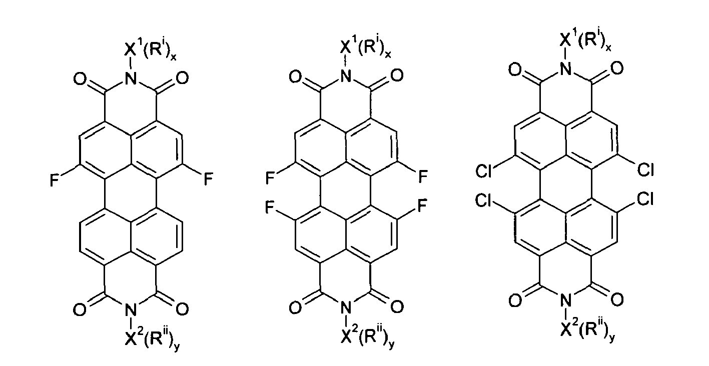

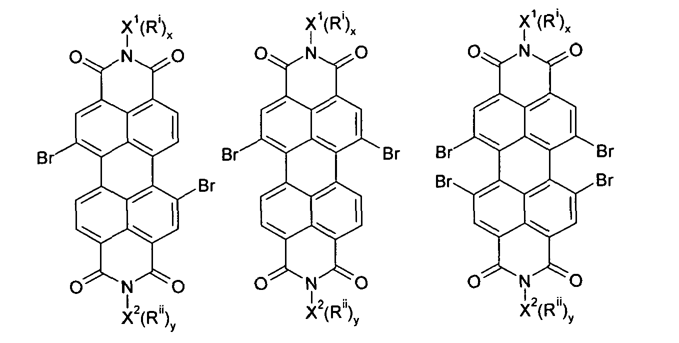

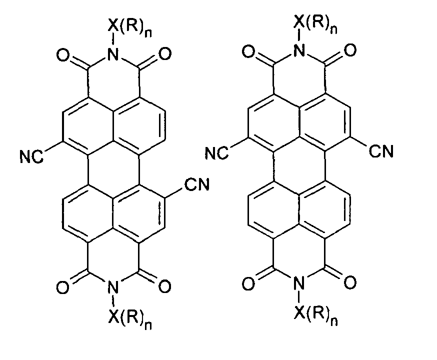

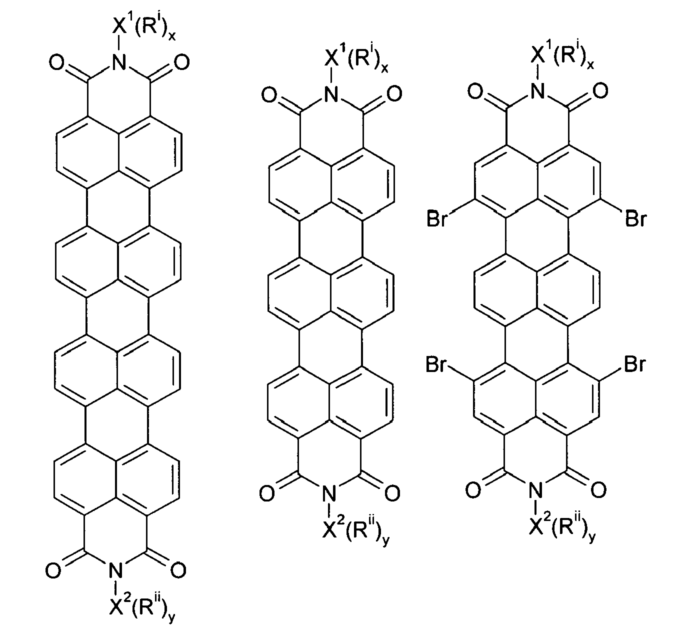

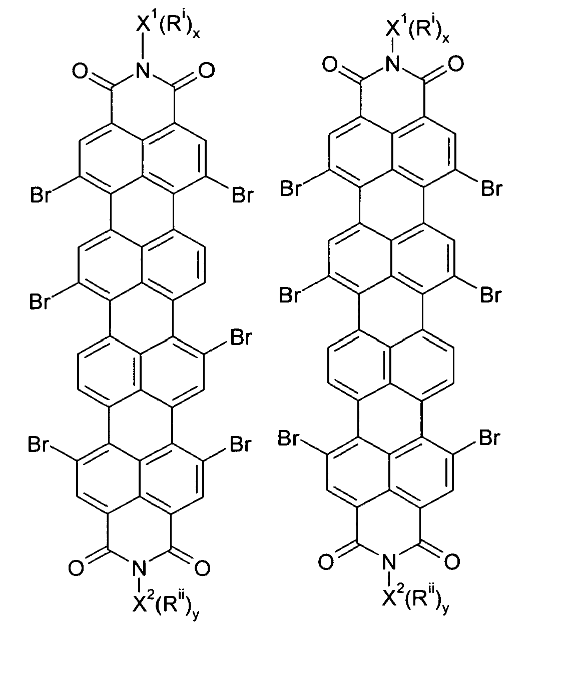

Bezüglich der Bedeutungen der Gruppen X¹(Rⁱ)ₓ bzw. X²(Rⁱⁱ)_{y} wird auf die eingangs gemachten Ausführungen Bezug genommen.

Im Folgenden werden einige besonders bevorzugte erfindungsgemäße Verbindungen wiedergegeben:

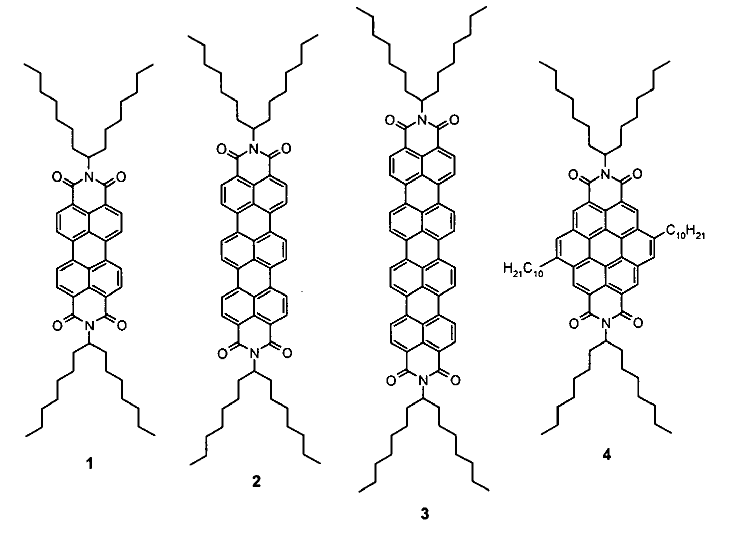

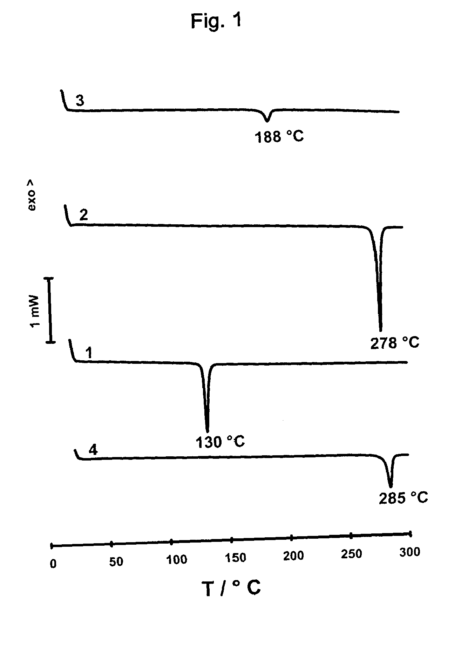

Die zuvor beschriebenen Verbindungen sind in der Regel thermisch stabil. Figur 1 zeigt das thermische Verhalten der Verbindungen 1 bis 4, bestimmt mittels DSC (differential scanning calorimetry). 1 zeigt einen direkten Übergang vom columnar geordneten Zustand zum isotropen Zustand bei 130°C. Die Isotropisierungstempereatur für 2 liegt bei 278°C und für 3 bei oberhalb 500°C. 3 zeigt einen weiteren flüssig-kristallinen Zustand bei 188°C. Corronen 4 erfährt einen direkten Übergang vom columnar geordneten Zustand zum isotropen Zustand bei 285°C.

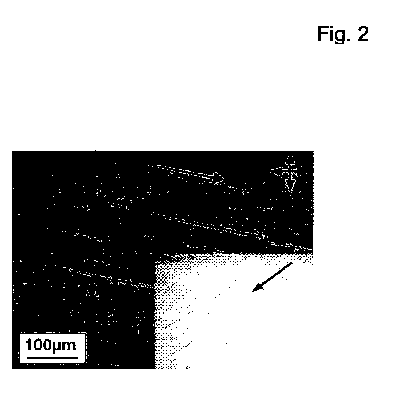

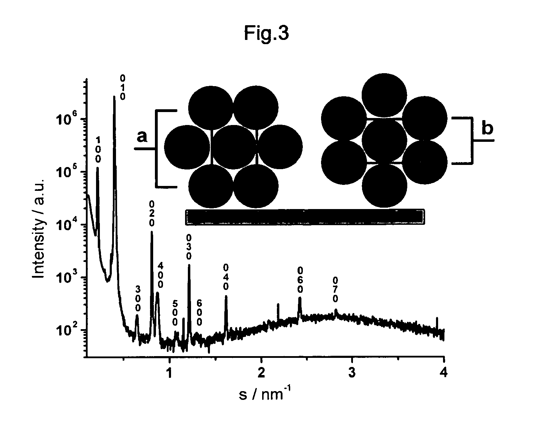

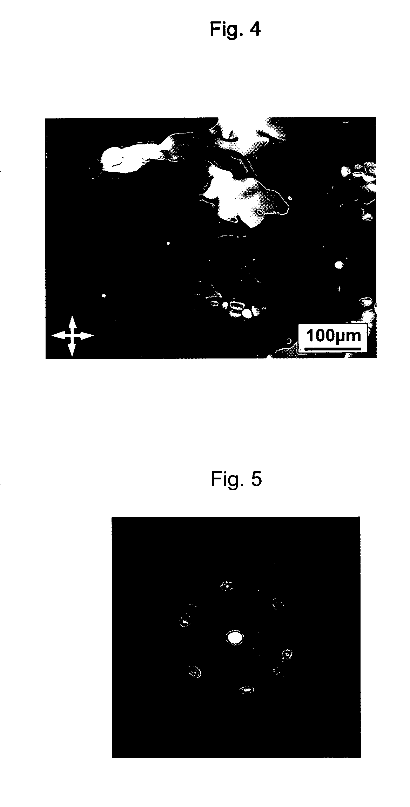

TDI (2) und PDI (1) wurden zwischen zwei Glasplättchen gebracht und aus der isotropen Phase abgekühlt. Figur 2 zeigt den TDI-Film unter einem Polarisationsmikroskop. TDI zeigte Selbstorganisation in großen Domänen mit Größen von Hunderten von Mikrometern. Die Doppelbrechung und die hohe optische Anisotropie weisen auf eine ausgeprägte uniaxial columnar Anordnung mit edge-on-ausgerichteten Discs hin. Diese Anordnung wird auch durch "large area X-Ray scattering" in Reflektion bestätigt. Das Vorhandensein einer großen Anzahl scharfer Reflexe in Figur 3 bestätigt die hohe Kristallinität des Films von TDI 2. Die hier beobachtete edge on Anordnung eignet sich vorteilhaft für Halbleiter in OFETs. Die Moleküle können sich in edge on-Ausrichtung auf dem Dielektrikum anordnen und Ladungsträger durch die π-Ebenen der Rylengerüste hindurch transportieren. CDI Moleküle (4) wurden ebenfalls zwischen zwei Glasplatten "gesandwiched" und aus der isotropen Phase abgekühlt. In Figur 4 wird die Aufnahme unter einem Polarisationsmikroskop gezeigt, welche vernachlässigbare Doppelbrechung zeigt. Durch eine WAXS wide angle XRay scattering Transmissionsaufnahme (Figur 5) wird belegt, dass eine hexagonale Anordnung der Moleküle in Richtung des einfallenden Strahls vorliegt. Die Moleküle ordnen sich also parallel zur Oberflächen-Normale an. Diese face on Orientierung ist besonders vorteilhaft für einen Einsatz in solchen Solarzellen, in denen die Absorbermoleküle halbleitende Eigenschaften aufweisen müssen. Derartige angeorndet Absorber können sehr gut mit dem einfallenden Licht wechselwirken und sie leiten die Ladungsträger direkt in Richtung der Substrate, bzw. Elektroden, auf denen sie angeordnet sind.

Es kann angenommen werden, dass sich durch entsprechende Vorbehandlung der Oberflächen, der Methode der Aufbringung des Halbleiters auf das Substrat (aus Lösung oder aus der Schmelze) die beiden Anordnungen gezielt einstellen lassen können und je nach Anwendung in einem OFET oder in einer Solarzelle entweder face on (homöotrop) oder edge on Anordnungen eingestellt werden können. Auf jeden Fall aber sollten mit den zuvor beschriebenen Terrylendiimiden, Perrylendiimiden und Quaterrylendiimiden gute OFETs gebaut werden können und mit den Corronen gute Solarzellen.

### Herstellung

### Variante 1 (Imidierung von Rylentetracarbonsäuredianhydriden):

Die Herstellung der erfindungsgemäßen Verbindungen der allgemeinen Formel I kann ausgehen von bekannten Rylentetracarbonsäuredianhydriden. Die Rylencarbonsäureimide können hergestellt werden durch Imidierung der entsprechend substituierten oder unsusbtituierten Anhydride, so weit diese verfügbar sind. Für n = 1 (Naphthalintetracarbonsäuredianhydride) sind dies auf jeden Fall unsubstituierte, 2- bis 4-fach bromierte, 2- bis 4-fach fluorierte und 2- bis 4-fach cyanierte und 2-fach chlorierte. Für n = 2 (Perylentetracarbonsäuredianhydride) sind dies auf jeden Fall 4-fach chlorierte, 2-fach und 4-fach bromierte und 2-fach fluorierte.

Ein weiterer Gegenstand der Erfindung ist daher ein Verfahren zur Herstellung von Verbindungen der Formel I 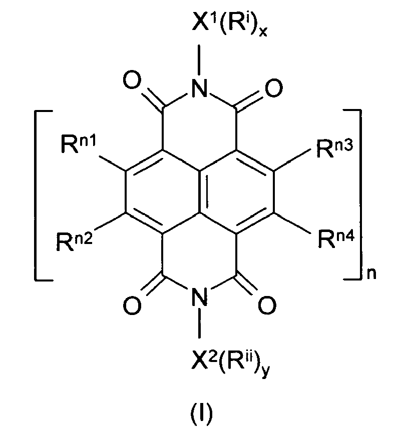 wobei
n für 1 oder 2 steht,
x und y unabhängig voneinander für eine ganze Zahl von 2 bis 6 stehen,
die Reste Rⁿ¹, Rⁿ², Rⁿ³ und Rⁿ⁴ unabhängig voneinander ausgewählt sind unter Wasserstoff, F, Cl, Br und CN,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man
a1) ein Rylendianhydrid der Formel Ia, 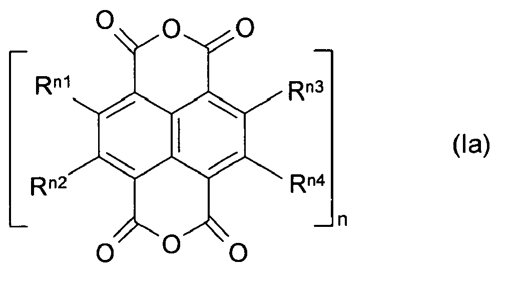 einer Umsetzung mit einem Amin der Formel H₂N-X¹(Rⁱ)ₓ und gegebenenfalls einem davon verschiedenen Amin der Formel H₂N-X²(Rⁱⁱ)_{y} unterzieht.

Die Imidierung von Carbonsäureanhydridgruppen ist prinzipiell bekannt. Vorzugsweise erfolgt die Umsetzung des Dianhydrids mit dem primären Amin in Gegenwart eines hochsiedenden Lösungsmittels.

Als Lösungsmittel für die Imidierung eignen sich unpolar aprotische Lösungsmittel, wie Kohlenwasserstoffe, z.B. Benzol, Toluol, Xylol, Mesitylen Petrolether Dekalin etc. Als Lösungsmittel sich auch polare aprotische Lösungsmittel, wie Trialkylamine, stickstoffhaltige Heterocyclen, N,N-disubstituierte aliphatische Carbonsäureamide (vorzugsweise N,N-Di(C₁-C₄-alkyl)-(C₁-C₄)carbonsäureamide) und N-Alkyllactame, wie Dimethylformamid, Diethylformamid, Dimethylacetamid, Dimethylbutyramid und N-Methylpyrrolidon, wobei N-Methylpyrrolidon bevorzugt ist.

Beispiele für besonders geeignete Lösungsmittel sind: Chinolin, Isochinolin, Chinaldin, Pyrimidin, N-Methylpiperidin und Pyridin; Dimethylformamid, Diethylformamid, Dimethylacetamid und Dimethylbutyramid; N-Methylpyrrolidon. Bevorzugtes Lösungsmittel dieser Gruppe ist Chinolin.

Als Lösungsmittel eignen sich auch protische Lösungsmittel, insbesondere aliphatische Carbonsäuren, vorzugsweise C₂-C₁₂-Carbonsäuren, wie Essigsäure, Propionsäure, Butansäure und Hexansäure, wobei Essigsäure und Propionsäure bevorzugte protische Lösungsmittel sind.

Je nach Reaktivität der Edukte sind entweder die aprotischen oder die protischen Lösungsmittel bevorzugt. So sind die aprotischen Lösungsmittel für die Umsetzung von nicht substituierten Rylencarbonsäureanhydriden vorzuziehen, während die protischen Lösungsmittel bei der Umsetzung der reaktiveren substituierten Rylencarbonsäureanhydride bevorzugt sind.

In der Regel werden 1 bis 100 ml, insbesondere 3 bis 70 ml, Lösungsmittel pro g Rylencarbonsäureanhydrid (la), verwendet.

Die Reaktion kann in Gegenwart eines Imidierungskatalysators vorgenommen werden. Als Imidierungskatalysatoren eignen sich Lewis- und Brönstedt-Säuren, z.B. organische und anorganische Säuren, z. B. Ameisensäure, Essigsäure, Propionsäure und

Phosphorsäure.

Der Einsatz einer Lewis-Säure ist insbesondere bei der Umsetzung der reaktionsträgeren nichtsubstituierten Rylencarbonsäureanhydride (la) zu empfehlen.

Als Lewis-Säure eignen sich vor allem Zink-, Kupfer- und Eisensalze, wobei im Fall von Kupfer auch die Oxide verwendet werden können. Bevorzugt sind die Zink- und Kupferverbindungen, wobei die Zinkverbindungen besonders bevorzugt sind.

Beispiele für geeignete Lewis-Säuren sind Zinkacetat, Zinkpropionat, Kupfer(I)oxid, Kupfer(11)oxid, Kupfer(I)chlorid, Kupfer(I)acetat und Eisen(III)chlorid, wobei Zinkacetat ganz besonders bevorzugt ist.

Kommt eine Lewis-Säure zum Einsatz, so werden in der Regel 0,5 bis 3, vorzugsweise 0,5 bis 1,5, Äquivalente pro mol umzusetzender Anhydridgruppe im Rylencarbonsäureanhydrid (la) verwendet.

Die Reaktionstemperatur hängt ebenfalls von der Reaktivität der Edukte ab und liegt im Allgemeinen im Bereich von 50 bis 250°C. Bei den reaktionsträgeren nichtsubstituierten Rylencarbonsäureanhydriden sind Temperaturen von 150 bis 230°C bevorzugt, die Umsetzung der reaktiveren substituierten Rylencarbonsäureanhydride (la) wird vorzugsweise bei 110 bis 170°C vorgenommen.

Gewünschtenfalls (bei der Umsetzung mit Diaminen zwingend) kann man das sich bildende Reaktionswasser sowie das gegebenenfalls durch die Hilfsstoffe eingebrachte Wasser während der Umsetzung abdestillieren.

Die Reaktion kann unter Schutzgas, z.B. Stickstoff oder Argon, erfolgen.

Eine verfahrenstechnisch geeignete Vorgehensweise zur Imidierung ist wie folgt:

Man erhitzt eine Mischung von Rylencarbonsäureanhydrid, Amin, Lösungsmittel und gegebenenfalls Lewis-Säure, gewünschtenfalls unter Abdestillieren des sich bildenden Wassers, 1 bis 48 h auf die gewünschte Reaktionstemperatur. Wird bei Abdestillieren des Wassers zu viel Lösungsmittel mitabdestilliert, so muß eine entsprechende weitere Menge ergänzt werden.

Die Isolierung der erhaltenen Verbindungen kann wie folgt vorgenommen werden:

Die gewünschten Verbindungen werden durch Abkühlen und Zugabe eines protischen Lösungsmittels, wie Wasser oder eines niederen aliphatischen Alkohols, z.B. eines C₁-C₄-Alkanols, ausgefällt bzw. auskristallisiert, abfiltriert, mit einem der vorstehenden Lösungsmittel und gegebenenfalls einer verdünnten Mineralsäure zur Entfernung von Rückstanden von Rylendicarbonsäureimidderivaten und/oder anorganischen Salzen gewaschen und getrocknet.

Die erhaltenen flüssig-kristallinen Verbindungen können gewünschtenfalls zur weiteren Reinigung einer Säulenchromatographie bzw. Säulenfiltration oder einer Umkristallisation bzw. fraktionierten Kristallisation unterzogen werden.

### Variante 2 (Suzuki-Reaktion):

Die Herstellung von erfindungsgemäßen Perylen-, Terrylen- und Quaterrylen-Verbindungen der allgemeinen Formel I, bei denen der Aromatenkern unsubstituiert ist, kann auch durch Suzuki-Kupplungsreaktion erfolgen, bevorzugt wird dieses Verfahren zur Herstellung von Terrylen- und Quaterrylen-Verbindungen der allgemeinen Formel 1 eingesetzt. Ein geeignetes Verfahren zur Herstellung von Terrylentetracarbonsäurediimiden durch Suzuki-Kupplung ist z.B. in der WO 2005/070894 beschrieben.

Ein weiterer Gegenstand der Erfindung ist daher ein Verfahren zur Herstellung von Verbindungen der Formel I 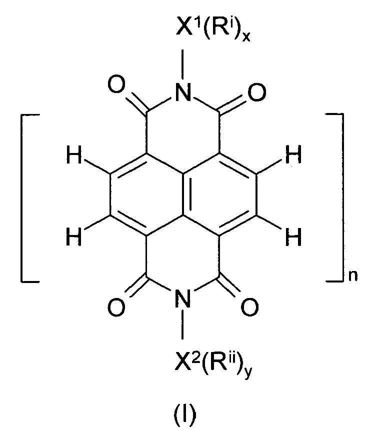 wobei
n für 2, 3 oder 4 steht,
x und y unabhängig voneinander für eine ganze Zahl von 2 bis 6 stehen,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man
α) eine Verbindung der Formel IIIa) 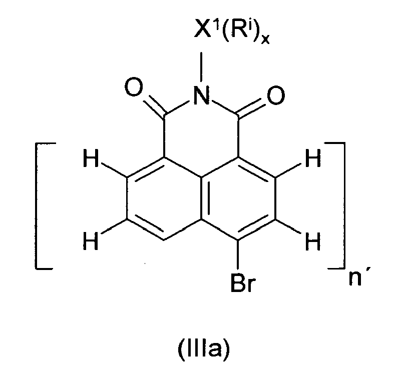 wobei
   n' für 1 oder 2 steht,
   mit einem Diboran der Formel IV 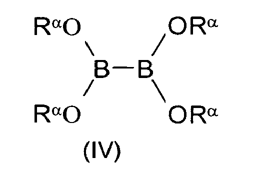 worin
   R^{α} gleich oder verschieden sind und unabhängig voneinander Wasserstoff, C₁-C₃₀-Alkyl, C₅-C₈-Cycloalkyl, Aryl oder Hetaryl bedeuten, wobei die Reste R^{α} auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl-oder Hetarylgruppen substituiert sein kann, miteinander verbunden sein können,
   unter Erhalt einer Verbindung der Formel V 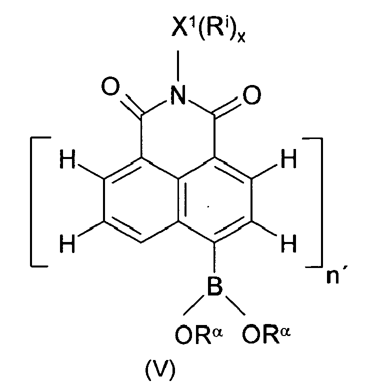 umsetzt,
β) die Verbindung der Formel V) mit einer Verbindung der Formel IIIb) 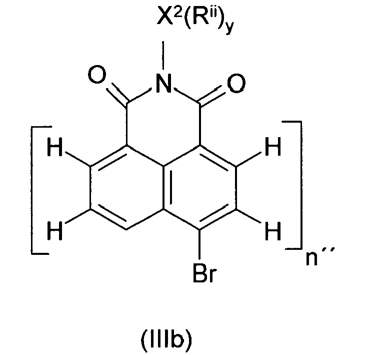 wobei
   n" für 1 oder 2 steht,
   in Gegenwart eines Übergangsmetallkatalysators und einer Base einer Suzuki-Kupplungsreaktion unter Erhalt einer Verbindung der Formel VI 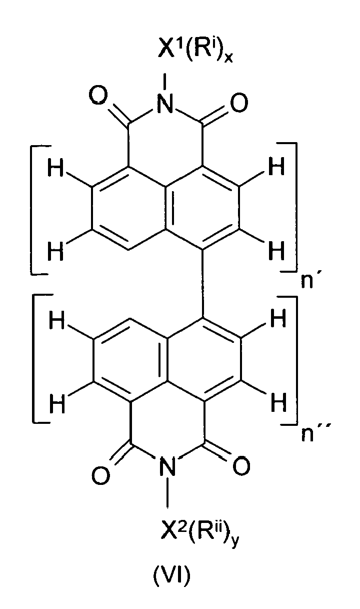
γ) die Verbindung der Formel VI) durch Cyclodehydrierung in einem Hydroxy- und Aminofunktionen aufweisenden und eine im Wesentlichen ungelöste Base enthaltenden organischen Reaktionsmedium in eine Verbindung der Formel I überführt, wobei n für die Summe aus n' und n" steht.

Zur Bereitstellung der Verbindungen der Formeln IIIa bzw. IIIb kann man Verbindungen der Formeln 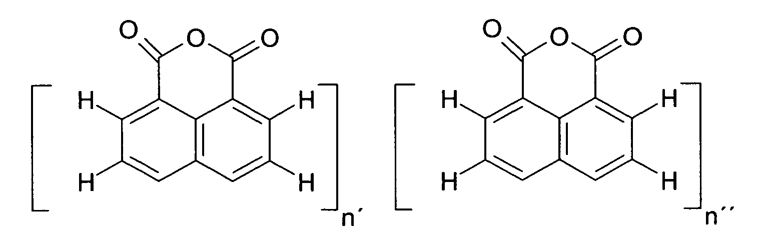 worin n' und n" unabhängig für 1 oder 2 steht,
einer Umsetzung mit einem Amin der Formel H₂N-X¹(Rⁱ)ₓ bzw. einem davon verschiedenen Amin der Formel H₂N-X²(Rⁱⁱ)_{y} und einer Bromierung unterziehen. Die Reihenfolge von Bromierung und Imidierung ist dabei in der Regel nicht kritisch. Die Imidierung kann unter den zuvor für Schritt a1) beschriebenen Bedingungen erfolgen.

Halogenierte Verbindungen der Formeln IIIa bzw. IIIb, worin n' und n" für 1 stehen 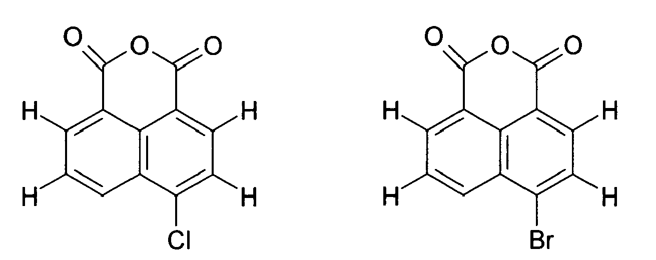 sind kommerziell erhältlich.

Die Bromierung von Perylenmonoanhydrid erfolgt vorzugsweise in H₂SO₄

Die Bromierung der Rylenmonoimide erfolgt vorzugsweise in einer Carbonsäure wie z. B. Essigsäure, Propionsäure oder Buttersäure. Dabei beträgt die Reaktionstemperatur vorzugsweise 20 bis 100°C, besonders bevorzugt 20 bis 50°C. Es werden werden bevorzugt 1 bis 10 Moläquivalente, bevorzugt 1-5 Moläquivalente Brom, bezogen auf die zu bromierende Verbindung, eingesetzt. Die Reaktion erfolgt z.B. unter Rühren für 1 bis 24 Stunden. Die Einsatzmenge an Carbonsäure beträgt bevorzugt 10 bis 100 bevorzugt 15 bis 50 ml Carbonsäure je g Rylenderivat. Bevorzugt werden zur Bromierung 1 bis 5 Gew.-%, besonders bevorzugt 1 bis 2 Gew.-% Jod, bezogen auf Brom, als Katalysator zugesetzt.

### Schritt α)

Die Umsetzung des Diborans IV mit dem Edukt IIIa in Schritt α) erfolgt bevorzugt in Gegenwart eines aprotischen organischen Lösungsmittels, eines Übergangsmetallkatalysators und einer Base.

Das Molverhältnis Diboran IV zu Edukt IIIa liegt dabei im allgemeinen bei 0,8 : 1 bis 3 : 1, insbesondere bei 1,5 : 1 bis 2 : 1.

Als Lösungsmittel sind für Schritt α) grundsätzlich alle unter den Reaktionsbedingungen gegen Basen stabilen aprotischen Lösungsmittel mit einem Siedepunkt oberhalb der gewählten Reaktionstemperatur geeignet, in denen sich die Edukte IIIa bei Reaktionstemperatur vollständig und die verwendeten Katalysatoren und Basen zumindest partiell lösen, so daß weitgehend homogene Reaktionsbedingungen vorliegen. Es können sowohl unpolar-aprotische als auch polar-aprotische Lösungsmittel eingesetzt werden, wobei die unpolar-aprotischen Lösungsmittel bevorzugt sind.

Beispiele für bevorzugte unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für besonders geeignete Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether.

Im Fall von Perylenen als Edukte IIIa sind die unpolar-aprotischen Lösungsmittel, vor allem Toluol, besonders bevorzugt, im Fall von Naphthalinen als Edukte IIIa sind polar-aprotische Lösungsmittel, insbesondere Dioxan, besonders bevorzugt.

Die Lösungsmittelmenge beträgt in der Regel 10 bis 1000 ml, bevorzugt 20 bis 300 ml, je g Edukt IIIa.

Als Übergangsmetallkatalysator eignen sich insbesondere Palladiumkomplexe, wie Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(It)chlorid, Bis(tricyclohexylphosphin)palladium(11)acetat, (2,2'-Bipyridyl)palladium(II)chlorid, Bis(triphenylphosphin)palladium(II)chlorid, Tris(dibenzylidenaceton)dipalladium(0), 1,5-Cyclooctadienpalladium(II)chlorid, Bis(acetonitril)palladium(11)chlorid und Bis(benzonitril)palladium(II)chlorid, wobei [1,1'-Bis(diphenylphosphino)ferrocen]palladium(II)chlorid und Tetrakis(triphenylphosphin)palladium(0) bevorzugt sind.

Üblicherweise wird der Übergangsmetallkatalysator in einer Menge von 1 bis 20 mol-%, vor allem 2 bis 10 mol-%, bezogen auf das Edukt IIIa, eingesetzt.

Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonat, zum Einsatz. Bevorzugte Basen sind die Acetate, vor allem Kaliumacetat.

Im Allgemeinen werden 1 bis 5 mol, bevorzugt 2 bis 4 mol, Base je mol Edukt IIIa verwendet.

Die Reaktionstemperatur liegt üblicherweise bei 20 bis 180°C, vor allem bei 60 bis 120°C.

Die Reaktionszeit beträgt in der Regel 0,5 bis 30 h, insbesondere 1 bis 20 h.

Verfahrenstechnisch geht man in Schritt α) zweckmäßigerweise wie folgt vor: Man legt Edukt IIIa und Lösungsmittel vor, gibt Diboran IV, den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Reinheit des so hergestellten Dioxaborolanylderivate V reicht im Allgemeinen für die Weiterverarbeitung aus. Gegebenenfalls kann das Rohprodukt durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens weiter aufgereinigt werden.

### Schritt β)

Die Umsetzung des Dioxaborolanylderivats V mit einer Verbindung IIIb (oder gegebenenfalls IIIa) wird vorzugsweise in Gegenwart eines organischen Lösungsmittels, gewünschtenfalls im Gemisch mit Wasser, durchgeführt.

Das Molverhältnis von V zu IIIb beträgt im Allgemeinen 0,8 : 1 bis 3 : 1.

Im Fall von Perylenen als Edukte IIIb beträgt das Molverhältnis von V zu IIIb in der Regel 0,8 : 1 bis 3 : 1, vorzugsweise 0,9 : 1 bis 2 : 1.

Im Fall von Naphthalinen als Edukte IIIb beträgt das Molverhältnis von V zu IIIb im allgemeinen 0,8 : 1 bis 3 : 1, bevorzugt 1,5 : 1 bis 2,5 : 1.

Als Lösungsmittel eignen sich für Schritt β) alle Lösungsmittel, in denen sich die Dioxaborolanylderivate V und die Edukte IIIb bei Reaktionstemperatur vollständig und die verwendeten Katalysatoren und Basen zumindest partiell lösen, so daß weitgehend homogene Reaktionsbedingungen vorliegen. Insbesondere geeignet sind die bereits für Schritt α) genannten Lösungsmittel, wobei auch hier die alkylsubstituierten Benzole bevorzugt sind. Die Lösungsmittelmenge liegt üblicherweise bei 10 bis 1000 ml, vorzugsweise bei 20 bis 100 ml, je g Dioxaborolanylderivat V.

Vorzugsweise setzt man in Schritt β) Wasser als zusätzliches Lösungsmittel ein. In diesem Fall werden in der Regel 10 bis 1000 ml, insbesondere 250 bis 500 ml, Wasser je l organisches Lösungsmittel verwendet.

Als Übergangsmetallkatalysatoren werden in Schritt β) ebenfalls vorzugsweise Palladiumkomplexe eingesetzt, wobei hier die gleichen Bevorzugungen wie in Schritt a) gelten. Die Einsatzmenge Katalysator beträgt üblicherweise 1 bis 20 mol-%, insbesondere 1,5 bis 5 mol-%, bezogen auf das Dioxaborolanylderivat V.

Als Base sind in Schritt β) wie in Schritt α) die Alkalimetallsalze schwacher Säuren bevorzugt, wobei die Carbonate, wie Natriumcarbonat und vor allem Kaliumcarbonat besonders bevorzugt sind. In der Regel liegt die Basenmenge bei 0,1 bis 10 mol, insbesondere bei 0,2 bis 5 mol, je mol Dioxaborolanylderivat V.

Die Reaktionstemperatur beträgt im Allgemeinen 20 bis 180°C, bevorzugt 60 bis 120°C. Wird in Schritt β) Wasser eingesetzt, so empfiehlt es sich, die Umsetzung nicht bei Temperaturen über 100°C vorzunehmen, da ansonsten unter Druck gearbeitet werden müßte.

Die Reaktion ist üblicherweise in 0,5 bis 48 h, insbesondere in 5 bis 20 h, beendet.

Verfahrenstechnisch geht man in Schritt β) zweckmäßigerweise wie folgt vor:

Man legt Dioxaborolanylderivat V und Edukt IIIb sowie Lösungsmittel vor, gibt Übergangsmetallkatalysator und die vorzugsweise in Wasser oder einem Wasser/Alkohol-Gemisch gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Die Reinheit des so hergestellten Kupplungsprodukts VI reicht im Allgemeinen für die Weiterverarbeitung aus. Gegebenenfalls kann das Rohprodukt durch Waschen mit Wasser und gewünschtenfalls einem geeigneten organischen Lösungsmittel, insbesondere einem chlorierten aliphatischen oder aromatischen Kohlenwasserstoff, oder durch Säulenchromatographie an Kieselgel mit einem Gemisch von Methylenchlorid und Hexan oder Pentan oder mit Toluol als Eluens weiter aufgereinigt werden.

### Schritt γ)

Die Cylodehydrierung von VI zu I erfolgt wie folgendermaßen geschrieben:

Die Cyclodehydrierung kann entweder in einem Hydroxy- und Aminofunktionen aufweisenden und eine im wesentlichen ungelöste Base enthaltenden organischen Reaktionsmedium oder in Gegenwart eines basenstabilen, hochsiedenden, organischen Lösungsmittels sowie einer alkali- oder erdalkalimetallhaltigen Base und einer stickstoffhaltigen Hilfsbase vorgenommen werden.

Bevorzugt ist die erste Verfahrensvariante, die im Folgenden näher beschrieben wird.

Als organisches Reaktionsmedium sind dabei vor allem Aminoalkohole geeignet, die 2 bis 20, vorzugsweise 2 bis 10 Kohlenstoffatome aufweisen. Die Kohlenstoffkette dieser Alkohole kann durch Sauerstoffatome in Etherfunktion unterbrochen sein. Beispiele für besonders geeignete Lösungsmittel sind Ethanolamin, Triethanolamin und Diethanolamin, wobei Ethanolamin bevorzugt ist. Es ist auch möglich, Mischungen von Alkoholen und Aminen zu verwenden, die jeweils einen Siedepunkt von mindestens 70°C haben und bei der Reaktionstemperatur flüssig sind.

Üblicherweise werden 1,5 bis 150 ml, bevorzugt 5 bis 50 ml, Reaktionsmedium je g Kupplungsprodukt VI eingesetzt.

Als im Reaktionsmedium im wesentlichen unlösliche Base eignen sich die Alkalimetallsalze, insbesondere die Natriumsalze und vor allem die Kaliumsalze, schwacher organischer und bevorzugt schwacher anorganischer Säuren, wie Formiate, Acetate, Propionate, Hydrogencarbonate und besonders bevorzugt Carbonate, insbesondere Natriumcarbonat und vor allem Kaliumcarbonat.

In der Regel beträgt die Basenmenge 1 bis 10 mol, bevorzugt 2 bis 5 mol, je mol Kupplungsprodukt VI.

Die Reaktionstemperatur liegt im Allgemeinen bei 40 bis 200°C, insbesondere bei 80 bis 160°C.

Die Reaktionszeit beträgt üblicherweise 0,5 bis 24 h, vorzugsweise 1 bis 12 h.

Verfahrenstechnisch geht man in Schritt γ) zweckmäßigerweise so vor, daß man eine Mischung Kupplungsprodukt VI, Lösungsmittel und Base 0,5 bis 24 h unter Schutzgas bei der gewünschten Reaktionstemperatur rührt und das gebildete Produkt I nach Abkühlen auf Raumtemperatur durch Zugabe eines Alkohols, wie Ethanol, oder von Wasser aus dem Reaktionsgemisch ausfällt, abfiltriert und mit Wasser wäscht.

Die Reinigung des erhaltenen Produkts 1 kann wie folgt vorgenommen werden: Katalysatorrückstände können durch eine schnelle Filtration über Kieselgel unter Waschen mit einem halogenierten aliphatischen Kohlenwasserstoff, wie Methylenchlorid, entfernt werden. Rückstände nichtumgesetzter Edukte auf Perylen- und Terrylenbasis können durch Säulenchromatographie an Kieselgel mit Methylen-chlorid als Eluens oder durch wiederholtes Waschen mit Hexan oder Pentan entfernt werden.

Die Herstellung von Terrylenen und Quaterrylenen durch Suzuki-Kupplung kann dann beispielsweise wie in folgendem Schema zusammengefaßt erfolgen:

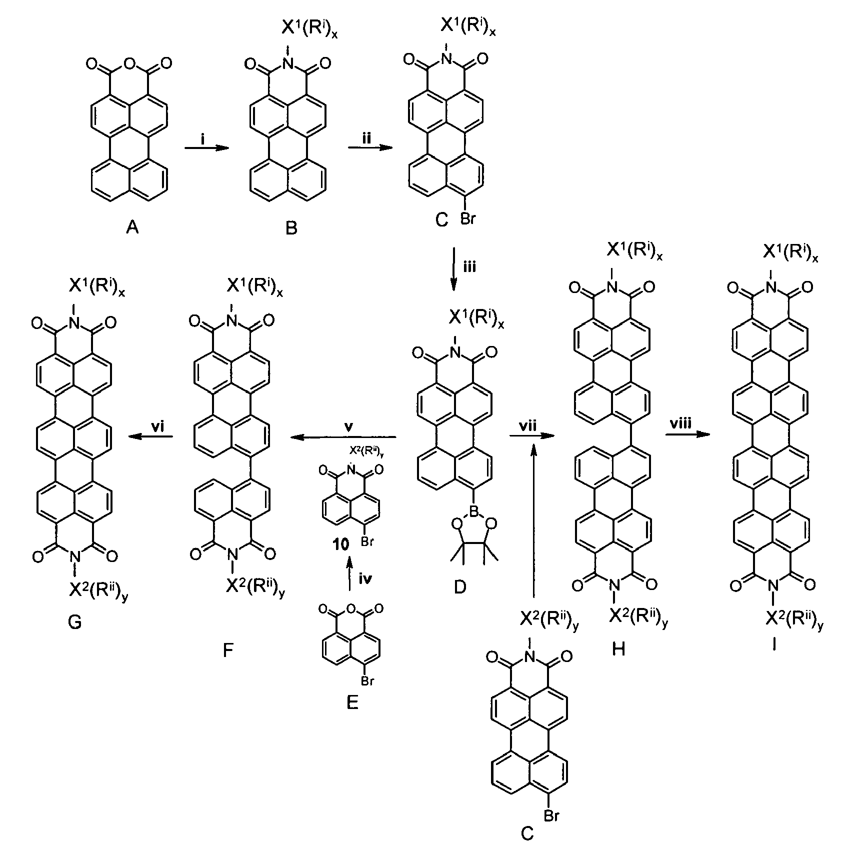

### Variante 3:

Die Herstellung von erfindungsgemäßen Terrylen- und Quaterrylen-Verbindungen der allgemeinen Formel I, kann auch durch Direktsynthese erfolgen.. Ein geeignetes Verfahren zur Herstellung von Terrylentetracarbonsäurediimiden durch Direktsynthese ist z.B. in der WO 2005/070895 beschrieben. Ein geeignetes Verfahren zur Herstellung von Quaterrylentetracarbonsäurediimiden durch Direktsynthese ist z.B. in der WO 2006/021307 beschrieben.

Ein weiterer Gegenstand der Erfindung ist daher ein Verfahren zur Herstellung von Verbindungen der Formel I 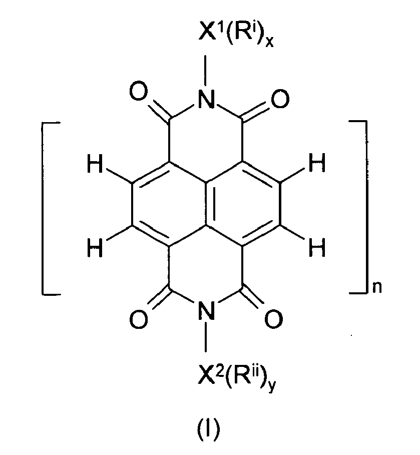 wobei
n für 3 oder 4 steht,
x und y unabhängig voneinander für eine ganze Zahl von 2 bis 6 stehen,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man ein Perylen-3,4-dicarbonsäureimid der allgemeinen Formel VII, 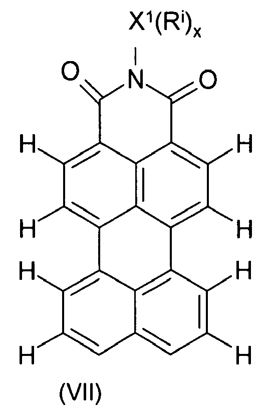 in Gegenwart eines basenstabilen Lösungsmittels und einer alkali- oder erdalkalimetallhaltigen Base mit einer Verbindung der allgemeinen Formel VIII 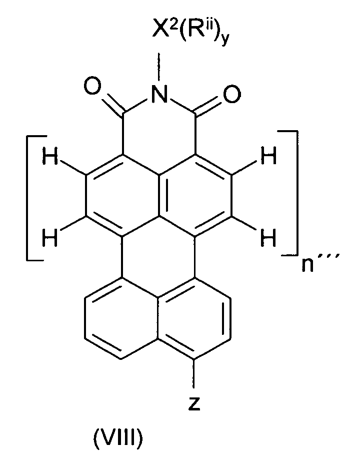 wobei n"' für 0 oder 1 steht und Z für Wasserstoff, Brom oder Chlor steht, umsetzt.

Als Edukte VIII können dabei sowohl in 4-Position chlorierte oder bromierte (n"'= 0) bzw. in 9-Position halogenierte (n"'= 1), als auch nichthalogenierte Verbindungen eingesetzt werden.

In einer ersten Ausführungsform zur Herstellung von Terrylenderivaten wird als Edukt VIII ein in 4-Position chloriertes oder bromiertes oder ein in 4-Position nicht halogeniertes Naphthalin-1,8-dicarbonsäureimid VIII (n"'= 0) eingesetzt.

Werden nichthalogenierte Edukte VIII verwendet, so empfiehlt es sich in der Regel, die Umsetzung bei verschärften Reaktionsbedingungen vorzunehmen, d.h. größere Überschüsse an Verbindung VIII und gegebenenfalls zusätzlich zu einer starken alkalimetallhaltigen Base eine stickstoffhaltige Hilfsbase sowie polar-aprotische Lösungsmittel einzusetzen.

Dementsprechend beträgt das Molverhältnis von Verbindung VIII zu Perylen-3,4-dicarbonsäureimid VII bei Verwendung von halogeniertem Edukt VIII (Z: Chlor oder Brom) üblicherweise 4 bis 1 : 1 und bevorzugt 2 bis 1 : 1, während es bei nichthalogeniertem Edukt VIII im allgemeinen bei 8 bis 1 : 1 und vorzugsweise bei 6 bis 2 : 1 liegt.

Als Lösungsmittel sind grundsätzlich alle unter den Reaktionsbedingungen gegen Basen stabile Lösungsmittel geeignet. Bevorzugt sind aprotische Lösungsmittel. Bevorzugt sind weiter Lösungsmittel mit einem Siedepunkt oberhalb der gewählten Reaktionstemperatur, in denen sich die Perylen-3,4-dicarbonsäureimide VII und die Verbindungen VIII bei Reaktionstemperatur vollständig und die verwendeten Basen zumindest partiell lösen, so daß weitgehend homogene Reaktionsbedingungen vorliegen. Es können sowohl unpolar-aprotische als auch polar-aprotische Lösungsmittel eingesetzt werden, wobei unpolar-aprotische Lösungsmittel und auf Ethern basierende aprotische Lösungsmittel bei Einsatz von halogenierten Edukten VIII und die polar-aprotischen Lösungsmittel bei Einsatz von nichthalogenierten Edukten VIII bevorzugt sind.

Beispiele für besonders geeignete unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für bevorzugte unpolar-aprotische Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin, 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ, und Alkylbenzolgemische vom Solvesso^{®} Typ. Besonders bevorzugte unpolar-aprotische Lösungsmittel sind Xylol (alle Isomere), Mesitylen und vor allem Toluol und Dekalin.

Beispiele für besonders geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für bevorzugte polar-aprotische Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether, wobei Diethylenglykoldiethylether, Diphenylether und vor allem Diethylenglykoldimethylether besonders bevorzugt sind.

Die Lösungsmittelmenge beträgt in der Regel 50 bis 250 ml unpolar-aprotisches Lösungsmittel bzw. 10 bis 50 ml polar-aprotisches Lösungsmittel je g Perylen-3,4-dicarbonsäureimid VII.

Als Base sind starke anorganische und organische alkali- oder erdalkalimetallhaltige Basen geeignet, wobei die alkalimetallhaltigen Basen besonders geeignet sind. Bevorzugte anorganische Basen sind Alkali- und Erdalkalimetallhydroxide und -amide, bevorzugte organische Basen sind Alkali- und Erdalkalimetallalkoholate (insbesondere die C₁-C₆-Alkoholate, vor allem tert.-C₄-C₆-Alkoholate), Alkali- und Erdalkalimetall-(phenyl)alkylamide (insbesondere die Bis(C₁-C₄-alkyl)amide) und Triphenylmethylmetallate. Besonders bevorzugt sind die Alkalimetallalkoholate. Bevorzugte Alkalimetalle sind Lithium, Natrium und Kalium, wobei Kalium ganz besonders bevorzugt ist. Besonders geeignete Erdalkalimetalle sind Magnesium und Calcium.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat und Kalium-tert.-butylat; Lithiumdimethylamid, Lithiumdiethylamid, Lithiumdiisopropylamid, Natriumdiisopropylamid, Triphenylmethyllithium, Triphenylmethylnatrium und Triphenylmethylkalium.

Ganz besonders bevorzugte Basen sind Lithiumdiisopropylamid, Natriummethylat, Natrium-tert.-butylat, vor allem Kaliummethylat und Kaliumhydroxid und insbesondere Kalium-tert.-butylat.

Bei Verwendung der Methylate und der Hydroxide sowie generell bei Verwendung von nichthalogenierten Edukten VIII empfiehlt sich zur Erhöhung der Reaktivität der Zusatz einer stickstoffhaltigen Hilfsbase mit geringer nucleophiler Wirkung. Geeignete Basen sind bei den Reaktionstemperaturen flüssige Alkylamine, insbesondere Tri-C₃-C₆-alkylamine, wie Tripropylamin und Tributylamin, Alkoholamine, insbesondere Mono-, Di- und Tri-C₂-C₄-alkoholamine, wie Mono-, Di- und Triethanolamin, und insbesondere heterocyclische Basen, wie Pyridin, N-Methylpiperidin, N-Methyl-piperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU). Geeignete Einsatzmengen für die Hilfsbase liegen im Fall der halogenierten Edukte VIII im allgemeinen bei 1 bis 15 g, vorzugsweise bei 1 bis 5 g, je g Perylen-3,4-dicarbon-säureimid VII und im Fall der nichthalogenierten Edukte III in der Regel bei 1 bis 60 g, bevorzugt bei 5 bis 20 g, je g Perylen-3,4-dicarbonsäureimid II. Von der Alkalimetallbase werden bei halogenierten Edukten VIII üblicherweise 2 bis 10 mol, insbesondere 2 bis 4 mol, je mol Perylen-3,4-dicarbonsäureimid VII und bei nichthalogenierten Edukten VIII im allgemeinen 2 bis 20 mol, vorzugsweise 8 bis 20 mol, je mol Perylen-3,4-dicarbonsäureimid VII, eingesetzt.

Die Alkalimetallbase kann in fester oder in gelöster Form eingesetzt werden. Wenn die Alkalimetallbase in Kombination mit einem unpolar-aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 50 bis 210°C, bevorzugt bei 70 bis 180°C.

Insbesondere bei Abwesenheit eine Hilfsbase kann es vorteilhaft sein, zunächst eine Reaktionstemperatur im oberen Bereich zu wählen, um das Perylen-3,4-dicarbonsäureimid VII in 9-Stellung zu deprotonieren. Die anschließende Kupplungsreaktion mit dem Edukt VIII kann dann in der Regel bei niedrigerer Temperatur durchgeführt werden, was sich insbesondere bei Edukten VIII mit basenlabilen Substituenten am Imidstickstoffatom empfiehlt.

Die Reaktionszeit beträgt in der Regel 1 bis 3 h bei halogenierten Edukten VIII und 2 bis 8 h bei nichthalogenierten Edukten VIII.

Verfahrenstechnisch geht man beim Einsatz nichthalogenierter Edukte VIII zweckmäßigerweise wie folgt vor:

Man legt Perylen-3,4-dicarbonsäureimid VII, Verbindung VIII und Base vor, gibt Lösungsmittel und gegebenenfalls Hilfsbase unter Schutzgas zu und erhitzt die Mischung die gewünschte Zeit unter Rühren und unter Schutzgas auf die gewünschte Reaktionstemperatur. Nach Abkühlen auf Raumtemperatur fällt man die Verbindungen I durch Zugabe von einem protischen Lösungsmittel, das die anderen Komponenten löst, z.B. von C₁-C₃-Alkoholen und insbesondere Wasser, aus. Man filtriert ab und wäscht mit einem der genannten Lösungsmittel, insbesondere mit einem der Alkohole.

Bei Verwendung halogenierter Edukte VIII kann analog vorgehen. Man kann jedoch auch zunächst nur ein Gemisch von Perylen-3,4-dicarbonsäureimid VII, Base, gegebenenfalls Hilfsbase sowie Lösungsmittel unter Rühren und Schutzgas auf eine Temperatur im Bereich von 120 bis 210°C erhitzen (Deprotonierung) und das Edukt VIII anschließend, gegebenenfalls nach Absenken der Temperatur auf 50 bis 120°C, zugeben.

Zur weiteren Reinigung kann man die Produkte I z.B. aus einem Gemisch von halogenierten Lösungsmitteln, wie Chlorform und Methylenchlorid, und Alkoholen, wie Methanol, Ethanol und Isopropanol, umkristallisieren. Alternativ kann man auch eine Säulenchromatographie an Kieselgel unter Verwendung von Methylenchlorid oder Aceton als Eluens vornehmen.

Mit Hilfe der dritten Variante des erfindungsgemäßen Verfahrens unter Einsatz eines in 4-Position chlorierten oder bromierten oder eines in 4-Position nicht halogenierten Naphthalin-1,8-dicarbonsäureimid VIII (n"'= 0) können Terrylen-3,4:11,12-tetracarbonsäuredümide I in guten Ausbeuten (in der Regel von 40 bis 80%) und hohen Reinheiten (üblicherweise 95 bis 99%) auf wirtschaftliche Weise in einem Schritt hergestellt werden. Sowohl an den Imidstickstoffatomen symmetrisch als auch unsymmetrisch substituierte Verbindungen I sind auf vorteilhafte Weise zugänglich.

In einer zweiten Ausführungsform zur Herstellung von Quaterrylenderivaten wird als Edukt VIII ein in 9-Position chloriertes oder bromiertes oder ein in 9-Position nicht halogeniertes Perylen-3,4-dicarbonsäureimid VIII (n"'= 1) eingesetzt.

Mit Hilfe des erfindungsgemäßen Verfahrens können die Quaterrylen-3,4:13,14-tetracarbonsäurediimide I in einem Schritt durch Umsetzung eines Perylen-3,4-dicarbonsäureimids VIII (im folgenden Imid VIII genannt) mit einem Perylen-3,4-dicarbonsäureimid VII (im folgenden Imid VII genannt) in Gegenwart eines basenstabilen, hochsiedenden, organischen Lösungsmittels und einer alkali- oder erdalkalimetallhaltigen Base hergestellt werden.

Als Imid VIII kann dabei sowohl ein in 9-Position halogeniertes, also chloriertes oder insbesondere bromiertes, als auch ein nichthalogeniertes Imid, das am Imidstickstoffatom einen Rest X¹(Rⁱ)ₓ tragen kann, der mit dem Rest X²(Rⁱⁱ)_{y} am Imidstickstoffatom des Imids VII übereinstimmt oder von diesem verschieden ist, eingesetzt werden.

Der Einsatz von halogeniertem Imid VIII ermöglicht dabei die gezielte Synthese unsymmetrischer Quaterrylen-3,4:13,14-tetracarbonsäurediimide I (R ≠ R'). In diesem Fall ist es vorteilhaft, ein Molverhältnis VIII zu VII von 4 bis 1 : 1, insbesondere von 2 bis 1 : 1, zu verwenden.

Wird nichthalogeniertes Imid VIII verwendet, so empfiehlt es sich in der Regel, die Umsetzung bei verschärften Reaktionsbedingungen vorzunehmen, d.h. zusätzlich zu einer starken alkalimetallhaltigen Base eine stickstoffhaltige Hilfsbase einzusetzen.

Als Lösungsmittel sind grundsätzlich alle unter den Reaktionsbedingungen gegen Basen stabilen, hochsiedenden Lösungsmittel (Siedepunkt > 100°C und oberhalb der gewählten Reaktionstemperatur) geeignet, in denen sich die verwendeten Basen bei Reaktionstemperatur vollständig und die Imide VII und VIII zumindest partiell, bevorzugt vollständig lösen, so daß weitgehend homogene Reaktionsbedingungen vorliegen. Es können sowohl aprotische (unpolar-aprotische und polar-aprotische) als auch protische Lösungsmittel eingesetzt werden. Selbstverständlich können auch Lösungsmittelmischungen verwendet werden.

Beispiele für geeignete unpolar-aprotische Lösungsmittel sind bei > 100°C siedende Kohlenwasserstoffe aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für bevorzugte unpolar-aprotische Lösungsmittel seien im einzelnen genannt:

Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan;

Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin, 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ, und Alkylbenzolgemische vom Solvesso^{®} Typ.

Besonders bevorzugte unpolar-aprotische Lösungsmittel sind Xylol (alle Isomere), Mesitylen und vor allem Dekalin.

Beispiele für geeignete polar-aprotische Lösungsmittel sind stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für bevorzugte polar-aprotische Lösungsmittel seien im einzelnen genannt:
Chinolin, Isochinolin, Chinaldin, Pyrimidin, N-Methylpiperidin und Pyridin;
Dimethyl- und Tetramethyltetrahydrofuran und Dioxan;
Diphenylether; Ethylenglykoldiethyl-, -dipropyl-, -diisopropyl-, -di-n-butyl-, -di-sec.-butyl- und -di-tert.-butylether und Ethylenglykolmethylethylether, Di- und Triethylenglykoldimethyl-, -diethyl-, -dipropyl-, -diisopropyl-, -di-n-butyl-, -di-sec.-butyl- und -di-tert.-butylether und Di- und Triethylenglykolmethylethylether.

Dabei sind Diethylenglykoldiethylether, Diphenylether und vor allem Diethylenglykoldimethylether besonders bevorzugt.

Beispiele für geeignete protische Lösungsmittel sind bei > 100°C siedende einwertige und mehrwertige, aliphatische und aromatische Alkohole (insbesondere einwertige C₄-C₁₈-Alkanole, mehrwertige C₂-C₄-Alkohole und deren Oligomere, wie C₂-C₃-Alkylenglykole, die bis zu 6 Alkylenoxideinheiten enthalten können, und Phenole), Etheralkohole (insbesondere Mono-C₁-C₆-alkyl- und -phenylether von monomeren und oligomeren C₂-C₃-Alkylenglykolen, die bis zu 6 Alkylenoxideinheiten enthalten können, vor allem Ethylenglykolmono-C₄-C₆-alkylether) und Aminoalkohole (insbesondere Mono-, Di- und Tri-C₂-C₄-alkoholamine).

Als Beispiele für bevorzugte protische Lösungsmittel seien im einzelnen genannt:
n-Butanol, Isobutanol, tert.-Butanol, n-Pentanol, Isopentanol, 2-Methylbutanol, 2-Methyl-2-butanol (tert.-Amylalkohol), Hexanol, 2-Methylpentanol, 3-Methyl-3-pentanol, Heptanol, 1-Ethylpentanol, 3-Ethyl-3-pentanol, 2,3-Dimethyl-3-pentanol, Octanol, 2-Ethylhexanol, 2,4,4-Trimethyl-2-pentanol, Isooctylakohol, Nonanol, Isononylalkohol, Decanol, 2,2,3,4,4-Pentamethyl-3-pentanol, Isodecylalkohol, Undecanol, Dodecanol, Tridecanol, Isotridecylalkohol, Tetradecanol, Pentadecanol, Hexadecanol, Heptadecanol und Octadecanol;
Ethylenglykol, Diethylenglykol, Triethylenglykol, Tetraethylenglykol, Pentaethylenglykol und Hexaethylenglykol, Propylenglykol, 1,3-Propandiol, Glycerin und 1,2-, 1,3- und 1,4-Butandiol;
Ethylenglykolmonomethyl-, -ethyl-, -propyl-, -isopropyl-, -n-butyl-, -sec.-butyl-, -tert.-butyl-, -n-pentyl- und -n-hexylether und Ethylenglykolmonophenylether und Di- und Triethylenglykolmonomethyl-, -ethyl-, -propyl-, -isopropyl-, -n-butyl-, -sec.-butyl-, -tert.-butyl-, -n-pentyl- und -n-hexylether und Di- und Triethylenglykolmonophenylether;
Monoethanolamin, Diethanolamin und Triethanolamin.

Besonders bevorzugte protische Lösungsmittel sind Ethylenglykol und Ethanolamin.

Die Lösungsmittelmenge beträgt in der Regel 1 bis 20 g, bevorzugt 2 bis 10 g und besonders bevorzugt 2 bis 5 g je g Imid VII und VIII.

Als Base sind starke anorganische und organische alkali- oder erdalkalimetallhaltige Basen geeignet, wobei die alkalimetallhaltigen Basen besonders geeignet sind. Bevorzugte anorganische Basen sind Alkali- und Erdalkalimetallhydroxide und -amide, bevorzugte organische Basen sind Alkali- und Erdalkalimetallalkoholate (insbesondere die C₁-C₁₀-Alkoholate, vor allem tert.-C₄-C₁₀-Alkoholate), Alkali- und Erdalkalimetall-(phenyl)alkylamide (insbesondere die Bis(C₁-C₄-alkyl)amide) und Triphenylmethylmetallate. Besonders bevorzugt sind die Alkalimetallalkoholate. Bevorzugte Alkalimetalle sind Lithium, Natrium und Kalium, wobei Kalium ganz besonders bevorzugt ist. Besonders geeignete Erdalkalimetalle sind Magnesium und Calcium.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat und Kalium-tert.-butylat; Lithium-(1,1-dimethyl)octylact, Natrium-(1,1-dimethyl)octylat, Kalium-(1,1-dimethyl)octylat, Lithiumdimethylamid, Lithiumdiethylamid, Lithiumdiisopropylamid, Natriumdiisopropylamid, Triphenylmethyllithium, Triphenylmethylnatrium und Triphenylmethylkalium.

Ganz besonders bevorzugte Basen sind Lithiumdüsopropylamid, Natriummethylat, Natrium-tert.-butylat, vor allem Kaliummethylat und Kaliumhydroxid und insbesondere Kalium-tert.-butylat.

Bei Verwendung der Methylate und der Hydroxide sowie generell bei Verwendung von nichthalogenierten Imiden VIII empfiehlt sich zur Erhöhung der Reaktivität der Zusatz einer stickstoffhaltigen Hilfsbase mit geringer nukleophiler Wirkung, wenn nicht bereits ein stickstoffhaltiger Heterocyclus oder ein Alkoholamin als Lösugnsmittel anwesend ist. Geeignete Basen sind bei den Reaktionstemperaturen flüssige Alkylamine, insbesondere Tri-C₃-C₆-alkylamine, wie Tripropylamin und Tributylamin, Alkoholamine, insbesondere Mono-, Di- und Tri-C₂-C₄-alkoholamine, wie Mono-, Di- und Triethanolamin, und insbesondere heterocyclische Basen, wie Pyridin, N-Methylpiperidin, N-Methyl-piperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU).

Geeignete Einsatzmengen für die Hilfsbase liegen im allgemeinen bei 0,5 bis 25 g, bevorzugt 1 bis 10 g, besonders bevorzugt 1 bis 3 g, je g Imid VII und VIII.

Die Alkali- oder Erdalkalimetallbase wird in der Regel in Mengen von 2 bis 20 mol, insbesondere 2 bis 10 mol, je mol Imid VII und VIII eingesetzt.

Die Alkalimetallbase kann in fester oder in gelöster Form eingesetzt werden. Wenn die Alkalimetallbase in Kombination mit einem unpolar-aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 70 bis 210°C, bevorzugt bei 120 bis 180°C.

Insbesondere bei Abwesenheit eine Hilfsbase kann es zur Herstellung unsymmetrischer Quaterrylen-3,4:13,14-tetracarbonsäurediimide vorteilhaft sein, zunächst eine Reaktionstemperatur im oberen Bereich zu wählen, um das Imid VII in 9-Stellung zu deprotonieren. Die anschließende Kupplungsreaktion mit dem halogenierten Imid VIII kann dann in der Regel bei niedrigerer Temperatur durchgeführt werden, was sich insbesondere bei der Anwesenheit von basenlabilen Substituenten (z.B. Cyclohexyl) am Imidstickstoffatom empfiehlt.

Die Reaktionszeit beträgt in der Regel 1 bis 3 h bei Einsatz halogenierter Imide VIII und 2 bis 12 h bei Einsatz nichthalogenierter Imide VIII.

Verfahrenstechnisch geht man beim Einsatz nichthalogenierter Imide VIII, also insbesondere einer Homokondensation, zweckmäßigerweise wie folgt vor: Man erhitzt Lösungsmittel, Base und gegebenenfalls Hilfsbase zur Homogenisierung unter Schutzgas und gibt Imid VII und Imid VIII gegebenenfalls nach vorherigem Abkühlen unter Schutzgas zu und erhitzt die Mischung die gewünschte Zeit unter Rühren und unter Schutzgas auf die gewünschte Reaktionstemperatur. Nach Abkühlen auf Raumtemperatur fällt man die Quaterrylen-3,4:13,14-tetracarbonsäurediimide I durch Zugabe von einem protischen Lösungsmittel, das die anderen Komponenten löst, z.B. von C₁-C₆-Alkoholen oder Wasser, aus. Man filtriert ab und wäscht mit einem der genannten Lösungsmittel, insbesondere mit einem der Alkohole.

Bei Verwendung halogenierter Imide VIII kann man verfahrenstechnisch wie bei Verwendung nichthalogenierter Imide VIII vorgehen. Man kann jedoch auch zunächst nur ein Gemisch von Imid VII, Base, gegebenenfalls Hilfsbase sowie Lösungsmittel unter Rühren und Schutzgas auf eine Temperatur im Bereich von 120 bis 210°C erhitzen (Deprotonierung) und das Imid VIII anschließend, gegebenenfalls nach Absenken der Temperatur auf 50 bis 120°C, zugeben.

Gelegentlich kann es zweckmäßig sein, das Reaktionsprodukt einer Oxidation zu unterziehen. Dies kann am einfachsten durch Einblasen von Luftsauerstoff in die noch warme Reaktionsmischung geschehen. Es ist jedoch auch möglich, Oxidationsmittel, wie vorzugsweise Wasserstoffperoxid, aber auch aldehydgruppenhaltige Zucker, z.B. Glukose, insbesondere nach der Reaktion zuzugeben.

Zur weiteren Reinigung kann man die Produkte I z.B. aus einem Gemisch von halogenierten Lösungsmitteln, wie Chloroform und Methylenchlorid, und Alkoholen, wie Methanol, Ethanol und Isopropanol, umkristallisieren. Alternativ kann man auch eine Säulenchromatographie an Kieselgel unter Verwendung von Methylenchlorid oder Aceton als Eluens vornehmen.

Eine weitere Reinigungsmethode besteht darin, die Produkte I aus N,N-disubstituierten aliphatischen Carbonsäureamiden, wie N,N-Dimethylformamid und N,N-Dimethylacetamid, oder stickstoffhaltigen Heterocyclen, wie N-Methylpyrrolidon, oder deren Gemischen mit Alkoholen, wie Methanol, Ethanol und Isopropanol, umzukristallisieren oder mit diesen Lösungsmitteln zu waschen.

Schließlich können die Produkte I auch aus Schwefelsäure fraktioniert werden.

Mit Hilfe des erfindungsgemäßen Verfahrens können die Quaterrylen-3,4:13,14-tetracarbonsäurediimide I in guten Ausbeuten (in der Regel von 30 bis 60%) und hohen Reinheiten (üblicherweise 90 bis 99%) auf wirtschaftliche Weise in einem Schritt hergestellt werden. Sowohl an den Imidstickstoffatomen symmetrisch als auch unsymmetrisch substituierte Quaterrylen-3,4:13,14-tetracarbonsäurediimide sind auf vorteilhafte Weise zugänglich.

Corronene der allgemeinen Formel II, worin R^{a} und R^{b} für Alkyl stehen, sind prinzipiell bekannt (Müllen, J. Mater. Chem., 11, 1789(2001)).

Die erfindungsgemäßen und nach dem erfindungsgemäßen Verfahren erhältlichen Verbindungen eignen sich besonders vorteilhaft als organische Halbleiter. Sie fungieren dabei als n-Halbleiter und zeichnen sich durch ihre Luftstabilität aus. Weiterhin verfügen sie über eine hohe Ladungstransportmobilität und haben ein hohes on/off-Verhältnis. Sie eignen sich in besonders vorteilhafter Weise für organische Feldeffekttransistoren. Die erfindungsgemäßen Verbindungen eignen sich vorteilhaft zur Herstellung von integrierten Schaltkreisen (ICs), für die bislang übliche n-Kanal MOSFET (metal oxide semiconductor field-effect transistor (MOSFET) zum Einsatz kommen. Dabei handelt es sich dann um CMOS analoge Halbleiterbausteine, z.B. für Microprozessoren, Microcontroller, statische RAM, und andere digitale logic circuits. Zur Herstellung von Halbleitermaterialien können die erfindungsgemäßen Verfahren nach einem der folgenden Verfahren weiterverarbeitet werden: Drucken (Offset, Flexo, Gravur, Screen, Inkjet, Elektrofotografie), Verdampfen, Lasertransfer, Fotolithografie, Dropcasting. Sie eignen sich insbesondere für einen Einsatz in Displays und RFID-Tags.

Geeignete organische Solarzellen sind in der Regel schichtförmig aufgebaut und umfassen in der Regel zumindest die folgenden Schichten: Anode, photoaktive Schicht und Kathode. Diese Schichten befinden sich in der Regel auf einem dafür üblichen Substrat. Geeignete Substrate sind z.B. oxidische Materialien (wie Glas, Quartz, Keramik, SiO₂, etc.), Polymere (z.B. Polyvinylchlorid, Polyolefine, wie Polyethylen und Polypropylen, Polyester, Fluorpolymere, Polyamide, Polyurethane, Polyalkyl(meth)acrylate, Polystyrol und Mischungen und Komposite davon) und Kombinationen davon.

Als Elektroden (Kathode, Anode) eignen sich prinzipiell Metalle (vorzugsweise der Gruppen 8, 9, 10 or 11 des Periodensystems, z.B. Pt, Au, Ag, Cu, Al, In, Mg, Ca), Halbleiter (z.B. dotiertes Si, dotiertes Ge, Indium-Zinn-Oxid (ITO), Gallium-Indium-Zinn-Oxid (GITO), Zink-Indium-Zinn-Oxid (ZITO), etc.), Metalllegierungen (z.B. auf Basis Pt, Au, Ag, Cu, etc., speziell Mg/Ag-Legierungen), Halbleiterlegierungen, etc. Bevorzugt wird als Anode ein gegenüber einfallendem Licht im Wesentlichen transparentes Material eingesetzt. Dazu zählt z.B. ITO, dotiertes ITO, ZnO, TiO₂, Ag, Au, Pt. Bevorzugt wird als Kathode ein das einfallende Licht im Wesentlichen reflektierendes Material eingesetzt. Dazu zählen z.B. Metallfilme, z.B aus Al, Ag, Au, In, Mg, Mg/Al, Ca, etc.

Die photoaktive Schicht ihrerseits umfaßt wenigstens eine oder besteht aus wenigstens einer Schicht, die als organisches Halbleitermaterial wenigsten eine Verbindung, die ausgewählt ist unter Verbindungen der Formeln I und II, wie zuvor definiert, enthält. In der Regel umfaßt die photoaktive Schicht wenigstens eine Schicht, die ein organisches Akzeptormaterial enthält (electron transport layer, ETL) und wenigstens eine Schicht, die ein organisches Donormaterial enthält (hole transport layer, HTL). Diese zwei Schichten können ganz oder zum Teil gemischt sein. Zusätzlich zu der photoaktive Schicht kann es eine oder mehrere weitere Schichten geben, z.B ETL, HTL, (die nicht absorbieren müssen), blockierende Schichten (z.B. excition blocking layers, EBL) (die nicht absorbieren sollen), Multiplikatorschichten (multiplication layers).

Der Aufbau organischer Solarzellen ist z.B. in US 2005/0098726 A1 und US 2005/0224905 A1 beschrieben, worauf hier in vollem Umfang Bezug genommen wird.

Die Erfindung wird anhand der folgenden, nicht einschränkenden Beispiele näher erläutert.

### Beispiele

### Beispiel 1: N,N'-Di(1-Heptyloctyl)terrylen-3,4:11,12-tetracarbonsäurediimid (2)

### 1.1 N-(1-Heptyloctyl)- 4-bromnaphthalin-1,8-dicarbonsäureimid

5 g (18 mmol) 4-Bromnaphthalin-1,8-dicarbonsäureanhydrid wurden mit 6,1 g (27 mmol) 1-Heptyloctylamin in 50 mL Ethylenglycol 4 Stunden auf 160 °C erwärmt. Die erhaltene Lösung ließ man auf Raumtemperatur abkühlen. Danach verdünnte man die Lösung mit 50 mL Methanol und 50 mL destilliertem Wasser. Man extrahierte die wässrige Lösung mit Diethylether, trocknete die Lösung über MgSO₄ und verdampfte das Lösungsmittel. Das erhaltene gelbe Öl reinigte man durch Säulenchromatographie mit Dichlormethan als Eluierungsmittel auf, wobei man 5,5 g (63 %) der Titelverbindung als farbloses Öl erhielt.
¹H-NMR (300 MHz, C₂D₂Cl₄, 25 °C): δ [ppm] = 8,53 (m, 1H), 8,44 (d, 1H, J = 8,5 Hz), 8,29 (m, 1H), 7,95 (d, 1H, J = 7,9 Hz), 7,76 (t, 1H, J = 7,6 Hz), 5,08-4,96 (m, 1H), 2,15-2,06 (m, 2H), 1,77-1,70 (m, 2H), 1,17-1,10 (m, 20 H), 0,77-0,72 (m, 6H).
¹³C-NMR (75 MHz, C₂D₂Cl₄, 25 °C): δ [ppm] = 165,02, 163,88, 133,19, 132,67, 131,38, 131,22, 130,69, 130,21, 129,35, 128,41, 123,98, 123,25, 123,12, 122,39, 54,91, 32,57, 32,15, 29,57, 27,22, 22,98, 14,50.
IR (NaCl): v (cm⁻¹) = 2924, 2854, 2362, 1704, 1663, 1619, 1588, 1508, 1461, 1400, 1342, 1239, 783
MS (FD): 486,1 (100 %) [M⁺] (berechnet für C₂₇H₃₆NBrNO₂ 486,50)

### 1.2 N-(1-Heptyloctyl)-perylen-3,4-dicarbonsäureimid

6 g (19 mmol) Perylen-3,4-dicarbonsäureanhydrid wurden mit 10,7 g (47 mmol) 1-Heptyloctylamin in 100 mL Chinolin 12 Stunden bei 160 °C unter Argon gerührt. Danach ließ man das Reaktionsgemisch auf Raumtemperatur abkühlen und verdünnte mit Salzsäure. Der Niederschlag wurde abfiltriert und unter vermindertem Druck getrocknet. Das rohe Produkt wurde durch Chromatographie an Kieselgel mit Dichlormethan als Eluierungsmittel aufgereinigt, wobei man 8, 4 g (83 %) eines roten Feststoffs mit einem Schmelzpunkt von 156 °C erhielt.
¹H-NMR (250 MHz, C₂D₂Cl₄, 25 °C): δ [ppm] = 8,38 (m, 2H), 8,17 (d, 2H, J = 7,9 Hz), 8,12 (d, 2H, J = 8,2 Hz), 7,75 (d, 2H, J = 7,9 Hz), 7,47 (t, 2H, J = 7,6 Hz), 5,17-5,05 (m, 1H), 2,23-2,16 (m, 2H), 1,89-1,81 (m, 2H), 1,27-1,20 (m, 20H), 0,82-0,77 (m, 6H).
¹³C-NMR (62,5 MHz, C₂D₂Cl₄, 25 °C): δ [ppm] = 165,24, 164,24, 136,85, 134,22, 131,90, 131,20, 131,00, 129,83, 129,09, 127,79, 127,16, 126,53, 123,77, 121,61, 120,84, 120,21, 54,58, 32,65, 32,15, 29,91, 29,58, 27,39, 22,97, 14,50.
IR(KBr): v (cm⁻¹) = 2924, 2853, 2365, 1697, 1653, 1594, 1572, 1450, 1460, 1408, 1355, 1292, 1244, 1172, 1136, 1109, 840, 754.
UV-Vis (CHCl₃): λₘₐₓ (ε) = 511 (50000), 489 nm (50000 M⁻¹ cm⁻¹)
Fluoreszenz (CHCl₃): λₘₐₓ = 571, 544 nm.
MS (FD): 531,2 (100 %) [M⁺] (berechnet für C₃₇H₄₁NO₂ 531,74)

### 1.3 N-(1-Heptyloctyl)-9-bromperylen-3,4-dicarbonsäureimid

Man suspendierte 8 g (15,05 mmol) N-(1-Heptyloctyt)-perylen-3,4-dicarbonsäureimid aus Beispiel 1.2 30 Minuten in 100 mL Essigsäure. Danach gab man 150 mg (0,6 mmol) Iod und 9,6 g (60,2 mmol) Brom zu der Mischung und rührte das erhaltene Gemisch 4,5 Stunden bei Raumtemperatur unter Lichtausschluss. Zur Entfernung von überschüssigem Brom leitete man Argon in den Kolben und fällte die Mischung mit 100 ml Methanol und rührte über Nacht. Man filtrierte das Produkt ab und wusch mit 150 ml Methanol. Nach dem Trocknen unter vermindertem Druck erhielt man 8,9 g (97 %) der Titelverbindung mit einem Schmelzpunkt von 163 °C.
¹H-NMR (500 MHz, CD₂Cl₂, 25 °C): δ [ppm] = 8,63 (d, 1H, J = 8,2 Hz), 8,61 (d, 1H, J = 8,2 Hz), 8,45 (d, 1H, J = 8,2 Hz), 8,42 (d, 1H, J = 8,2 Hz), 8,36 (d, 1H, J = 7,6 Hz), 8,27 (d, 1H, J = 8,2 Hz), 8,20 (d, 1H, J = 8,2 Hz), 7,87 (d, 1H, J = 8,2 Hz), 7,70 (t, 1H, J = 7,6 Hz), 5,17-5,05 (m, 1H), 2,23-2,16 (m, 2H), 1,89-1,81 (m, 2H), 1,27-1,20 (m, 20 H), 0,82-0,77 (m, 6H).
¹³C-NMR (Spinecho, 125 MHz, CD₂Cl₂, 25 °C): δ [ppm] = 165,16,164,12,136,16, 132,73, 132,01, 131,26, 129,90, 129,73, 129,47, 128,93, 128,20, 126,14, 126,08, 124,38, 123,67, 122,06, 121,31, 120,75, 120,48, 54,69, 32,65, 32,15, 29,90, 29,59, 27,40, 22,98, 14,53.
IR (KBr): v (cm⁻¹) = 2924, 2853, 2365, 1697, 1653, 1594, 1572, 1450, 1460, 1408, 1355, 1292, 1244, 1172, 1136, 1109, 840, 810, 754.
UV-Vis (CHCl₃): λₘₐₓ (ε) = 514 (51000), 489 nm (52000 M⁻¹ cm⁻¹)
Fluoreszenz (CHCl₃): λₘₐₓ = 571, 544 nm.
MS (FD): 611,1 (100 %) [M⁺] (berechnet für C₃₇H₄₀BrNO₂ 610,64)

### 1.4 N-(1-Heptyloctyl)-9-(4,4,5,5-tetramethyl-1,3,2-dioxaborolan-2-yl)-perylen-3,4-dicarbonsäureimid

Zu 1,2 g (2 mmol) N-(1-Heptyloctyl)-9-bromperylen-3,4-dicarbonsäureimid aus Beispiel 1.3 und 558 mg (2,5 mmol) Bis(pinacolato)diboron gab man unter einem schwachen Argonstrom 588 mg (5.3 mmol) Kaliumacetat in 20 mL Dioxan. Anschließend gab man 44 mg (0.1 mmol) [1,1'-Bis(diphenylphosphino)ferrocen]-palladium(II)-chlorid-Methylenchlorid ([PdCl₂(dppf)]xCH₂Cl₂) zu und rührte das Reaktionsgemisch unter einer Argonatmosphäre 16 Stunden bei 70 °C. Nach dem Abkühlen auf Raumtemperatur extrahierte man das Gemisch mit Dichlormethan und wusch zweimal mit destilliertem Wasser. Die organische Schicht trennte man ab, trocknete über Magnesiumsulfat und reinigte das rohe Produkt durch Säulenchromatographie mit Dichlormethan als Eluierungsmittel, wobei man 1,0 g (78 %) der Titelverbindung als roten Feststoff mit einem Schmelzpunkt von 213 °C erhielt.
¹H-NMR (300 MHz, THF-d₈, 25 °C): δ [ppm] = 8,87 (d, 1H, J = 7,7 Hz), 8,55-8,47 (m, 6H), 8,15 (d, 1H, J = 7,7 Hz), 7,59 (t, 1H, J = 7,7 Hz), 5,27-5,17 (m, 1 H), 2,40-2,28 (m, 2H), 1,84-1,77 (m, 2H), 1,44 (s, 12H), 1,34-1,24 (m, 20H), 0,85-0,81 (t, 6H, J = 6,8 Hz).
¹³C-NMR (75 MHz, THF-d8, 25 °C): δ [ppm] = 165,24, 164,33, 139,04, 137,83, 137,28, 132,81, 132,20, 131,45, 130,64, 130,02, 128,60, 127,81, 127,40, 124,38, 123,34, 123,25, 122,04, 121,90, 121,28.
IR (KBr): v (cm⁻¹) = 2925, 2854, 2362, 2337, 1691, 1653, 1592, 1507, 1461, 1416, 1376, 1332, 1272, 1246, 1209, 1142, 1113, 1068, 966, 858, 811, 754, 674
UV-Vis (CHCl₃): λₘₐₓ (ε) = 514 (47000), 489 nm (45000 M⁻¹ cm⁻¹).
Fluoreszenz (CHCl3): λₘₐₓ = 577,546 nm.
MS (FD): 657,2 (100 %) [M⁺] (berechnet für C₄₃H₅₂BN₂O₄ 657,71)

### 1.5 N-(1-Heptyloctyl)-9-(4-N-(1-Heptyloctyl)-naphthalin-1,8-dicarbonsäureimid)-perylen-3,4-dicarbonsäureimid

1,0 g, (1,52 mmol) N-(1-Heptyloctyl)-9-(4,4,5,5-tetramethyl-1,3,2-dioxaborolan-2-yl)-perylen-3,4-dicarbonsäureimid aus Beispiel 1.4 und 0.813 g (1.67 mmol) N-(1-Heptyloctyl)- 4-bromnaphthalin-1,8-dicarbonsäureimid aus Beispiel 1.1 wurden in 76 mL Toluol gelöst. Man gab eine Lösung von Na₂CO₃ in Wasser (63 mL, 1 M) und Ethanol (5 ml) zu und spülte die Lösung mit Argon. Danach gab man [Pd(PPh₃)₄] (80 mg, 0,06 mmol) zu und rührte das Reaktionsgemisch 16 h bei 80 °C. Man ließ das Reaktionsgemisch auf Raumtemperatur abkühlen. Man trennte die organische Phase ab und verdampfte das Lösungsmittel unter vermindertem Druck. Das Produkt wurde durch Chromatographie an Kieselgel mit Dichlormethan als Eluierungsmittel aufgereinigt, wobei man 1,1 g (79 %) als roten Feststoff mit einem Schmelzpunkt von 129 °C erhielt.
¹H-NMR (700 MHz, C₂D₂Cl₄, 130 °C) δ [ppm] = 8,68 (d, 1H, J = 6,7 Hz), 8,64-8,56 (m, 3H), 8,53 (d, 1H), 8,47 (d, 1H), 8,45-8,39 (m, 2H), 7,85-7,75 (m, 2H), 7,62 (d, 1H, J = 6,8 Hz), 7,59-7,54 (m, 1H), 7,45 (d, 1H, J = 7,1 Hz), 7,41 (d, 1H, J = 7,8 Hz), 5,22-5,11 (m, 2H), 2,31-2,19 (m, 4H), 1,98-1,86 (m, 4H), 1,41-1,19 (m, 40H), 0,93-0,77 (m, 12H).
¹³C-NMR (175 MHz, C₂D₂Cl₄,130 °C): δ [ppm] = 164,77, 164,63, 164,53, 144,02, 139,39, 136,84, 136,57, 133,87, 131,96, 131,64, 131,60, 131,36, 131,33, 130,58, 130,51, 130,27, 130,24, 129,32, 129,09, 129,03, 127,71, 127,32, 127,06, 124,18, 123,91, 123,83, 122,94, 122,64, 122,50, 120,89, 120,80, 55,16, 55,00, 32,93, 32,90, 31,93, 31,90, 29,64, 29,24, 27,24, 22,61, 13,92.
IR (KBr): v (cm⁻¹) = 2956, 2927, 2850, 1697, 1654, 1590, 1577, 1348, 811.
UV-Vis (CHCl₃): λₘₐₓ (ε) = 508 (40000), 482 (39000), 350 (14.000), 335 nm (16000 M⁻¹ cm⁻¹)
MS (FD): 937,5 (100 %) [M⁺] (berechnet für C₆₄H₇₆N₂O₄ 937,33)

### 1.6 N,N'-Di(1-Heptyloctyl)terrylen-3,4:11,12-tetracarbonsäurediimid

0,9 g (0,96 mmol) N-(1-Heptyloctyl)-9-(4-N-(1-Heptyloctyl)-naphthalin-1,8-dicarbonsäureimid)-perylen-3,4-dicarbonsäureimid, 6,42 g (46,5 mmol) K₂CO₃ und 9,0 g, (0,147 mol) Ethanolamin wurden unter Argon 3 h bei 160 °C gerührt. Nach dem Abkühlen auf Raumtemperatur goss man die Lösung in Methanol (20 mL). Den Niederschlag filtrierte man ab, wusch mit Wasser, trocknete unter vermindertem Druck und reinigte durch Säulenchromatographie an Kieselgel (CH₂Cl₂), wobei man ein blaues Produkt (0,763 g, 85 %) mit einem Schmelzpunkt von 278,13 °C erhielt.
¹H-NMR (250 MHz, THF-d₈, 25 °C): δ [ppm] = 8,25 (s, 4H), 8,18 (d, 8H, J = 8,5 Hz), 5,21 (m, 2H), 2,37 (m, 4H, J = 6,95 Hz), 1,92 (m, 4H), 1,42-1,30 (m, 40H), 0,89-0,84 (m, 12H).
¹³C-NMR (125 MHz, THF-d₈, 25 °C): δ [ppm] = 164,43, 163,52, 135,18, 130,52, 129,77, 127,85, 125,76, 124,35, 122,90, 122,04, 121,34, 54,78, 33,20, 32,79, 30,51, 30,17, 28,06, 23,43, 14,34.
IR (KBr): v (cm⁻¹) = 2923, 2852, 1694, 1652, 1585, 1379, 1353, 1323, 807.
UV-Vis (CHCl3): λₘₐₓ (ε) = 651 (127000), 598 (66000), 547 nm (21000 M⁻¹ cm⁻¹).
Fluoreszenz (CHCl₃): λₘₐₓ = 669, 729 nm
MS (FD): 935,6 (100 %) [M⁺] (berechnet für C₆₄H₇₄N₂O₄ 935,31)

### Beispiel 2: N,N'-Di(1-Heptyloctyl)quaterrylene-3,4:13,14-tetracarbonsäurediimid (3)

### 2.1 N,N'-Bis(1-Heptyloctyl)-9,9'-biperylen-3,4:3',4'-bis(dicarbonsäureimid)

0,2 g (0,3 mmol) N-(1-Heptyloctyl)-9-(4,4,5,5-tetramethyl-1,3,2-dioxaborolan-2-yl)-perylen-3,4-dicarbonsäureimid aus Beispiel 1.4 und 0,37 g, (0,6 mmol) N-(1-Heptyloctyl)-9-bromperylen-3,4-dicarbonsäureimid aus Beispiel 1.3 wurden in Toluol (15 mL) gelöst. Man gab eine Lösung von Na₂CO₃ in Wasser (10 mL, 1 M) und Ethanol (5 ml) zu und spülte das Gemisch mit Argon. Danach gab man [Pd(PPh₃)₄] (16 mg, 0,01 mmol) zu und rührte das Reaktionsgemisch 16 h bei 80 °C. Das Reaktionsgemisch ließ man auf Raumtemperatur abkühlen. Die organische Phase trennte man ab und verdampfte das Lösungsmittel unter vermindertem Druck. Man reinigte das rohe Produkt durch Chromatographie an Kieselgel mit Dichlormethan als Eluierungsmittel auf, wobei man 0,24 g (76 %) eines roten Feststoffs mit einem Schmelzpunkt von 304 °C erhielt.
¹H-NMR (700 MHz, C₂D₂Cl₄, 130 °C): δ [ppm] = 8,65-8,57 (m, 4H), 8,54 (d, 2H, J = 7,4 Hz), 8,47 (d, 2H, J = 7,7 Hz), 8,45-8,40 (m, 4H), 7,68 (d, 2H, J = 7,3 Hz), 7,55 (d, 2H, J = 8,0 Hz), 7,46 (t, 2H, J = 8,0 Hz), 5,22-5,11 (m, 2H), 2,30-2,19 (m, 4H), 1,97-1,85 (m, 4H), 1,37-1,19 (m, 40H), 0,94-0,780,84 (m, 12H).
¹³C-NMR (75 MHz, C₂D₂Cl₄, 130 °C): δ [ppm] = 164,58, 140,68, 137,01, 136,80, 134,15, 131,63, 130,29, 130,19, 130,10, 129,45, 129,24, 128,83, 127,56, 127,08, 123,88, 123,18, 122,46, 122,39, 120,77, 120,65, 54,99, 32,91, 31,91, 29,66, 29,26, 27,26, 22,62, 13,93.
IR (KBr): v (cm ⁻¹) = 2954, 2925, 2854, 1693, 1653, 1593, 1572, 1352, 812
UV-Vis (CHCl₃): λₘₐₓ (ε) = 527 nm (97000 M⁻¹ cm⁻¹)
MS (FD): 1060,0 (100 %) [M⁺] (berechnet für C₇₄H₈₀N₂O₄ 1061,47)

### 2.2 N,N'-Di(1-Heptyloctyl)quaterrylene-3,4:13,14-tetracarbonsäurediimid

0,2 g (0,19 mmol) N,N'-Bis(1-Heptyloctyl)-9,9'-biperylen-3,4:3',4'-bis(dicarbonsäureimid) aus Beispiel 2.1, 1,26 g (9.1 mmol) K₂CO₃ und 1,7 g, (0.,028 mol) Ethanolamin wurden 3 h bei 160 °C unter Argon gerührt. Nach Abkühlen auf Raumtemperatur goss man die Lösung in Methanol (10 mL). Den Niederschlag filtrierte man ab, wusch mit Wasser, trocknete unter vermindertem Druck und reinigte durch Säulenchromatographie an Kieselgel mit Dichlormethan als Eluierungsmittel auf, wobei man ein blaugrüne Produkt (0,165 g, 83 %) mit einem Schmelzpunkt von oberhalb 350 °C erhielt.
¹H-NMR (300 MHz, C₂D₂Cl₄, 120 °C): δ [ppm] = 8,47 (m, 16H), 5,17 (m, 2H), 2,25 (m, 4H), 1,93 (m, 4H), 1,32 (m, 40H), 0,84 (m, 12H)
¹³C-NMR (75 MHz, THF-d8, 25 °C): δ [ppm] = 164,14, 163,79, 140,81, 135,80, 131,51, 130,38, 129,34, 127,99, 127,17, 126,34, 124,15, 123,21, 120,68, 55,03, 33,51, 33,03, 30,81, 30,43, 28,38, 23,65, 14,56
IR (KBr): v (cm⁻¹) = 2956, 2925, 2856, 1693, 1652, 1575, 1349, 1286, 809
UV-Vis (CHCl3): λₘₐₓ (ε) = 762 nm (162000 M⁻¹ cm⁻¹)
MS (FD): 1058,1 (100 %) [M⁺] (berechnet für C₇₄H₇₈N₂O₄ 1059,4)

### Beispiel 3: N,N'-Di(1-heptyloctyl)-5,11-didecylcoronen-2,3:8,9-tetracarbonsäurediimid (4)

### 3.1 N,N'-Bis(1-heptyloctyl)-1,7-dibromperylen-3,4:9,10-tetracarbonsäurediimid

Eine Mischung aus 0,5 g, (0,909 mmol) 1,7-Dibromperylen-3,4:9,10-tertacarbonsäuredianhydrid und 0,75 g, (3,298 mmol) 1-Heptyloctylamin in 50 mL N-Methylpyrrolidon wurde 4 h bei 150 °C gerührt. Nach Abkühlen auf Raumtemperatur goss man die Lösung in verdünnte Salzsäure (400 mL). Den Niederschlag filtrierte man ab, wusch mit Wasser und Methanol, trocknete unter vermindertem Druck und reinigte durch Säulenchromatographie an Kieselgel (Petrolether / CH₂Cl 3/2), wobei man (0,34 g, 39 %) eines roten Produkts erhielt.
¹H-NMR (300 MHz, CD₂Cl, 25 °C): δ [ppm] = 9,53 (s, 1H); 9,50 (s, 1H); 8,89 (sb, 2H); 8,66 (dd, J = 7,2 Hz, 2H); 5,20 - 5,12 (m, 2H); 2,25 - 2,20 (m, 4H); 1,86 - 1,80 (m, 4H); 1,28 - 1,22 (m, 40H); 0,85 - 0,81 (m, 12H).
¹³C-NMR (75 MHz, CD₂Cl, 25 °C): δ [ppm] = 162,55 (C=O); 138,21; 137,13; 132,86; 132,66; 131,52; 130,10; 129,28; 128,48; 127,18; 120,61; 54,75; 41,08; 31,77; 29,44; 29,18; 26,85; 22,59; 13,79.
IR (KBr): v (cm⁻¹) = 2956, 2925, 2854, 1703, 1660, 1589, 1381, 1329, 1240, 810, 748
UV-Vis (CHCl3): λₘₐₓ (ε) = 390 (6000); 460 (15000) ; 490 (37000); 526 nm (55000 M⁻¹ cm⁻¹)
MS (FD): 971,3 (100 %) [M⁺] (berechnet für C₅₄H₆₈Br₂N₂O₄ 968,96)

### 3.2 N,N'-Bis(1-heptyloctyl)-1,7-didocen-1-yneperylen-3,4:9,10-tetracarbonsäurediimid

0,2 g (0,206 mmol) N,N'-Bis(1-heptyloctyl)-1,7-dibromperylen-3,4:9, 10-tetracarbonsäurediimid aus Beispiel 3.1, 0,14 g (0,824 mmol) 10 Dodec-1-in, 25 mg (0,021 mmol), 6 mg (0,020 mmol) [Pd(PPh₃)₂Cl], Triphenylphosphin und Kupfer(I)-iodid (4 mg, 0,020 mmol) wurden in einem Gemisch aus 20 mL Triethylamin und 20 mL Tetrahydrofuran 16 h bei 80 °C gerührt. Nach Abkühlen auf Raumtemperatur goss man die Lösung in verdünnte HCl (100 mL) und extrahierte das Gemisch mit CH₂Cl. Man verdampfte das Lösungsmittel unter vermindertem Druck und reinigte den erhaltenen Feststoff durch Säulenchromatographie an Kieselgel (Pentan / CH₂Cl 3/1), wobei man ein rotes Produkt (0,15 g, 64 %) erhielt. Das Produkt enthielt geringe Mengen an N,N'-Di(1-heptyloctyl)-5,11-didecylcoronen-2,3:8,9-tetracarbonsäurediimid, die durch sehr sorgfältige Säulenchromatographie an Kieselgel (Pentan / CH₂Cl 3/1) abgetrennt werden können.
¹H-NMR (300 MHz, C₂D₂Cl₄, 100 °C): δ [ppm] = 10,13 (d, J = 8,2 Hz, 2H); 8,72 (s, 2H); 8,60 (d, J = 8,2 Hz, 2H); 5,12 (sept, J = 6,1 Hz, 2H); 2,63 (t, J = 7,1 Hz, 4H); 2,22 - 2,17 (m, 4H); 1,89 - 1,74 (m, 8H); 1,58 - 1,53 (m, 4H); 1,26 - 1,22 (m, 64H); 0,84 - 0,79 (m, 18H).
¹³C-NMR (75 MHz, C₂D₂Cl₄, 100 °C, Spinechoexperiment): δ [ppm] = 164,11 (C=O); 138,34 (t); 134,44 (q); 134,00 (q); 130,49 (t); 127,97 (q); 127,85 (q); 127,18 (t); 123,66 (q); 122,71 (q); 121,28 (q); 102,06 (q); 82,82 (q); 55,18 (CH); 32,77 (CH₂); 32,03 (CH₂); 31,96 (CH₂); 29,73 (CH₂); 29,67 (CH₂); 29,43 (CH₂); 29,40 (CH₂); 29,33 (CH₂); 29,29 (CH₂); 28,61 (CH₂); 27,19 (CH₂); 22,76 (CH₂); 22,70 (CH₂); 20,52 (CH₂); 14,12 (CH₃); 14,09 (CH₃).
IR (KBr): v (cm⁻¹) = 2958, 2925, 2856, 2214, 1699, 1657, 1601, 1589, 1466, 1410, 1342, 1327, 1259, 1246, 812, 756, 706
UV-Vis (CHCl₃): λₘₐₓ (ε) = 413 (7000); 477 (13000); 512 (28000); 553 nm (48000 M⁻¹ cm⁻¹)
MS (FD): 1139,7 (100 %) [M⁺] (berechnet für C₇₈H₁₁₀N₂O₄ 1139,76)

### 3.3 N,N'-Di(1-heptyloctyl)-5,11-didecylcoronen-2,3:8,9-tetracarbonsäurediimid

Man löste 0,1 g (0,088 mmol) N,N'-Di(1-heptyloctyl)-1,7-di(dodec-1-ynyl)perylen-3,4:9,10-tetracarbonsäurediimid aus Beispiel 3.2 in 30 ml Toluol und entfernte den Sauerstoff aus der Lösung mit Argon. Danach gab man 0,1 mL 1,8-Diazabicyclo[5.4.0]undec-7-en (DBU) zu und rührte das Gemisch 20 h bei 110°C. Nach Abkühlen auf Raumtemperatur goss man die Lösung in kalte verdünnte HCl (300 mL) und extrahierte die Mischung mit Toluol. Man verdampfte das Lösungsmittel unter vermindertem Druck und reinigte den erhaltenen Feststoff durch Säulenchromatographie an Kieselgel (Petrolether / CH₂Cl 3/1), wobei man ein gelbes Produkt (40 mg, 40 %) mit einem Schmelzpunkt von 285 °C erhielt.
¹H-NMR (300 MHz, C₂D₂Cl₄, 100 °C): δ [ppm] = 10,14 (s, 2H); 9,88 (s, 2H); 8,96 (s, 2H); 5,40 (sept, J = 5,9 Hz, 2H); 3,89 (t, J = 7,7 Hz, 4H); 2,47 - 2,36 (m, 4H); 2,23 - 2,18 (m, 4H); 2,09 - 2,02 (m, 4H); 1,71 - 1,66 (m, 4H); 1,48 - 1,21 (m, 64H); 0,87 - 0,76 (m, 18H).
¹³C-NMR (75 MHz, C₂D₂Cl₄, 100 °C, Spinechoexperiment): δ [ppm] = 142,09 (C=O); 130,46 (t); 130,20 (q); 129,64 (q); 128,58 (t); 127,16 (q); 126,75 (t); 124,36 (q); 123,16 (q); 123,03 (q); 122,58 (q); 122,35 (q); 121,71 (q); 55,59 (CH); 34,06 (CH₂); 33,09 (CH₂); 32,04 (CH₂); 31,97 (CH₂); 31,70 (CH₂); 30,02 (CH₂); 29,77 (CH₂); 29,43 (CH₂); 29,34 (CH₂); 27,41 (CH₂); 22,76 (CH₂); 22,69 (CH₂); 14,13 (CH3); 14,07 (CH₃).
IR (KBr): v (cm⁻¹) = 2960, 2927, 2856, 1703, 1660, 1606, 1469, 1335, 926, 810.
UV-Vis (CHCl₃): λₘₐₓ (ε) = 334 (70000); 337 (70000); 382 (8000); 404 (28000), 429 (58000), 477 (10000), 511 nm (18000 M⁻¹ cm⁻¹).
Fluoreszenz (CHCl₃): λₘₐₓ = 515, 555, 601 nm.
MS (FD): 1139,8 (100 %) [M⁺] (berechnet für C₇₈H₁₁₀N₂O₄ 1139,76).

### Beispiel 5: N,N'-Di(1-Heptyloctyl)perrylen-3,4:9,10-tetracarbonsäuredümid (1)

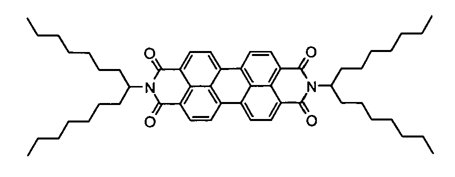

Diese Verbindung wurde gemäß H. Langhals, S. Demmig, T. Potrawa, J. prakt. Chem. 1991, 333, 733-748 hergestellt.

## Patentansprüche

1. Verwendung von Verbindungen der allgemeinen Formeln I und II 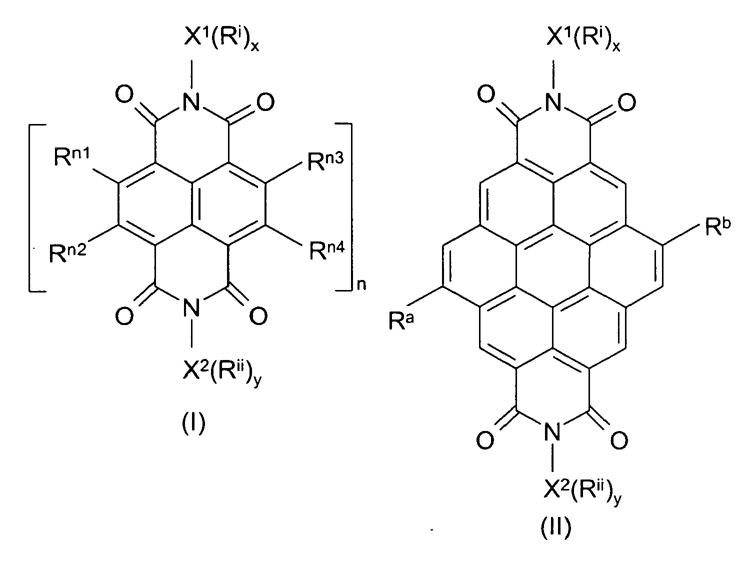 wobei
n für 1, 2, 3 oder 4 steht,
x und y unabhängig voneinander für eine ganze Zahl von 2 bis 6 stehen,
die Reste Rⁿ¹, Rⁿ², Rⁿ³ und Rⁿ⁴ für n = 1 oder 2 unabhängig voneinander ausgewählt sind unter Wasserstoff, F, Cl, Br und CN, für n = 3 oder 4 unabhängig voneinander ausgewählt sind unter Wasserstoff, F, Cl und Br,
die Reste R^{a} und R^{b} unabhängig voneinander ausgewählt sind unter Wasserstoff und Alkyl,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
als n-Halbleiter für organische Feldeffekttransistoren oder Solarzellen.

2. Verwendung nach Anspruch 1, wobei in den Verbindungen der Formel I x und y unabhängig voneinander für 2 oder 3 stehen.

3. Verwendung nach einem der vorhergehenden Ansprüche, wobei die Reste Rⁱ und Rⁱⁱ jeweils unabhängig voneinander ausgewählt sind unter linearem C₄- bis C₃₀-Alkyl, welche durch ein oder mehrere nicht benachbarte Sauerstoffatom(e) unterbrochen sein können.

4. Verwendung nach einem der vorhergehenden Ansprüche, wobei die Gruppen X¹(Rⁱ)ₓ und X²(Rⁱⁱ)_{y} ausgewählt sind unter Gruppen der Formeln II.1 bis II.14, wobei für die Gruppen X²(Rⁱⁱ)_{y} in den folgenden Formeln Rⁱⁱ statt Rⁱ und y statt x steht:
#―(A)ₚ―C(Rⁱ)ₓ (II.1) 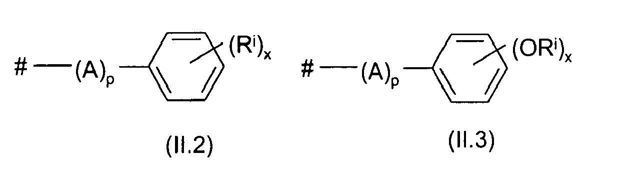 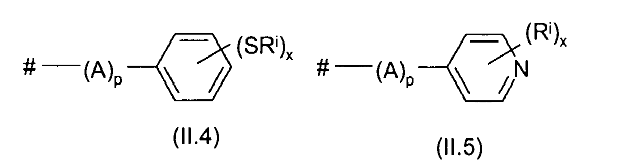 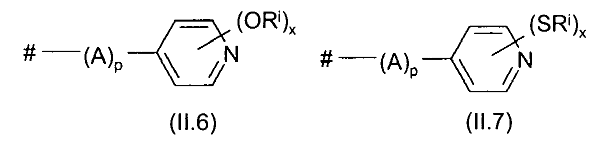 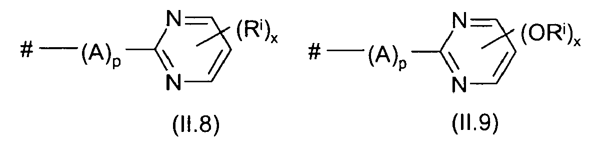 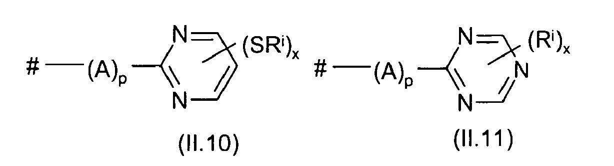 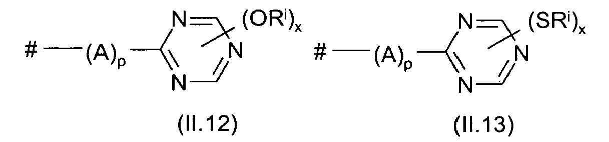 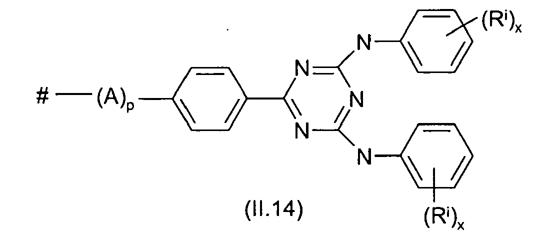
worin
# für die Verknüpfungsstelle zum Imidstickstoffatom steht,
p für 0 oder 1 steht,
x für 2 oder 3 steht, wobei in den Verbindungen der Formeln ll.11, ll.12 und 11.13 x für 2 steht und wobei in den Verbindungen der Formel ll.14 jedes x für 2 oder 3 stehen kann,
A soweit vorhanden, für eine C₁-C₁₀-Alkylengruppe steht, die durch eine oder mehrere nicht benachbarte Gruppen, die ausgewählt sind unter -O- und -S-, unterbrochen sein kann, und
Rⁱ die in Anspruch 1 oder 3 angegebene Bedeutung besitzt.

5. Verwendung nach einem der Ansprüche 1 bis 4, wobei in den Verbindungen der Formel I die Reste Rⁿ¹, Rⁿ², Rⁿ³ und Rⁿ⁴ alle für Wasserstoff stehen.

6. Verwendung nach einem der Ansprüche 1 bis 4, wobei in den Verbindungen der Formel I wenigstens einer der Reste Rⁿ¹, Rⁿ², Rⁿ³ und Rⁿ⁴ für einen von Wasserstoff verschiedenen Rest steht.

7. Verwendung nach Anspruch 1, wobei in den Verbindungen der Formel II wenigstens einer der Reste R^{a} und R^{b} für einen von Wasserstoff verschiedenen Rest steht.

8. Verwendung nach Anspruch 7, wobei in den Verbindungen der Formel II die Reste R^{a} und R^{b} beide für Alkyl stehen.

9. Verwendung von Verbindungen der allgemeinen Formeln I, wie in einem der Ansprüche 1 bis 8 definiert, zur Herstellung von organischen Feldeffekttransistoren.

10. Verwendung von Verbindungen der allgemeinen Formeln II, wie in einem der Ansprüche 1 bis 8 definiert, zur Herstellung von Solarzellen.

11. Verfahren zur Herstellung von Verbindungen der Formel I 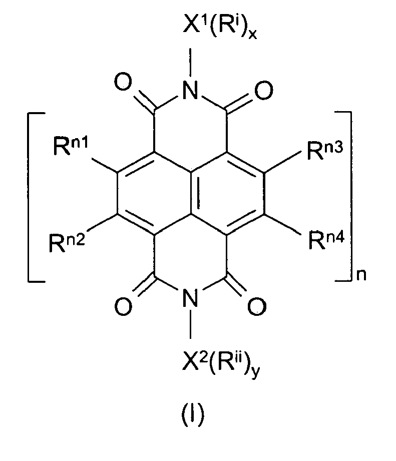 wobei
n für 1 oder 2 steht,
x und y unabhängig voneinander für eine ganze Zahl von 2 bis 6 stehen,
die Reste Rⁿ¹, Rⁿ², Rⁿ³ und Rⁿ⁴ unabhängig voneinander ausgewählt sind unter Wasserstoff, F, Cl, Br und CN,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man
a1) ein Rylendianhydrid der Formel la, 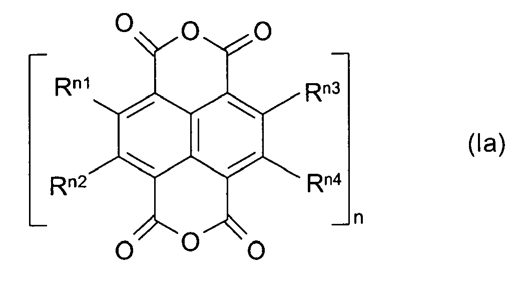
einer Umsetzung mit einem Amin der Formel H₂N-X¹(Rⁱ)ₓ und gegebenenfalls einem davon verschiedenen Amin der Formel H₂N-X²(Rⁱⁱ)_{y} unterzieht.

12. Verfahren zur Herstellung von Verbindungen der Formel I 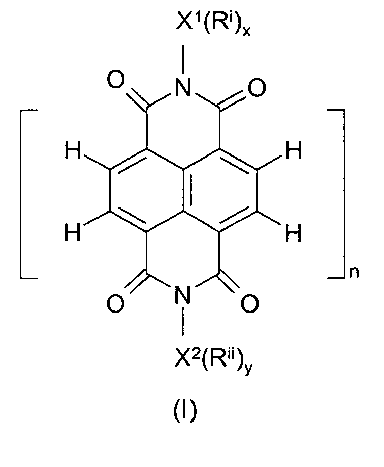 wobei
n für 2, 3 oder 4 steht,
x und y unabhängig voneinander für eine ganze Zahl von 2 bis 6 stehen,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man
α) eine Verbindung der Formel IIIa) 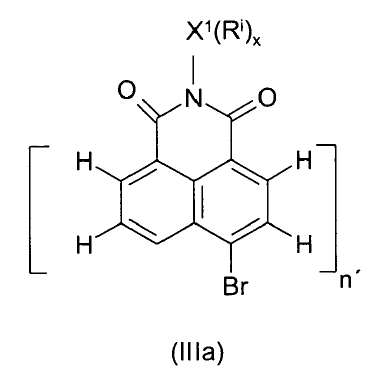 wobei
n' für 1 oder 2 steht,
mit einem Diboran der Formel IV 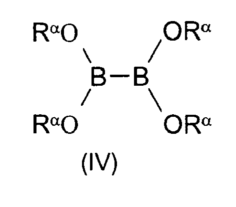 worin
R^{α} gleich oder verschieden sind und unabhängig voneinander Wasserstoff, C₁-C₃₀-Alkyl,C₅-C₈-Cycloalkyl, Aryl oder Hetaryl bedeuten, wobei die Reste R^{α} auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl-oder Hetarylgruppen substituiert sein kann, miteinander verbunden sein können,
unter Erhalt einer Verbindung der Formel V 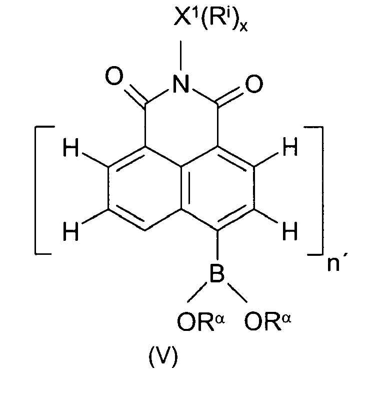 umsetzt,
β) die Verbindung der Formel V) mit einer Verbindung der Formel IIIb) 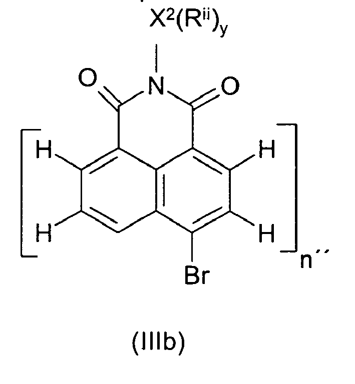 wobei
n" für 1 oder 2 steht,
in Gegenwart eines Übergangsmetallkatalysators und einer Base einer Suzuki-Kupplungsreaktion unter Erhalt einer Verbindung der Formel VI 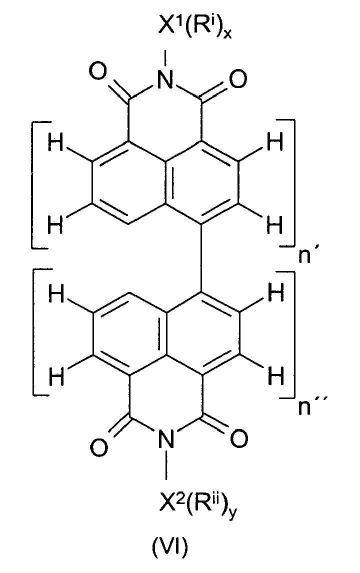
γ) die Verbindung der Formel VI) durch Cyclodehydrierung in einem Hydroxy- und Aminofunktionen aufweisenden und eine im Wesentlichen ungelöste Base enthaltenden organischen Reaktionsmedium in eine Verbindung der Formel I überführt, wobei n für die Summe aus n'und n" steht.

13. Verfahren zur Herstellung von Verbindungen der Formel I 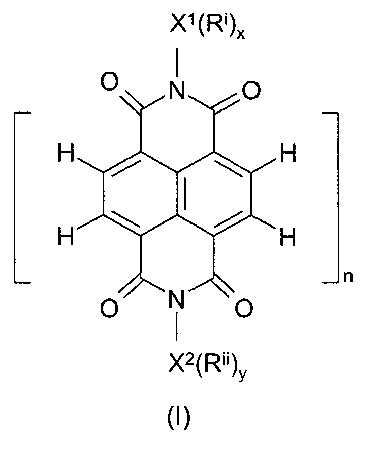 wobei
n für 3 oder 4 steht,
x und y unabhängig voneinander für eine ganze Zahl von 2 bis 6 stehen,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können,
bei dem man ein Perylen-3,4-dicarbonsäureimid der allgemeinen Formel VII, 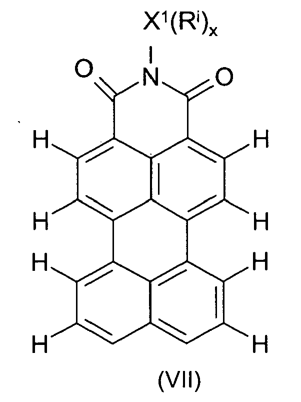 in Gegenwart eines basenstabilen Lösungsmittels und einer alkali- oder erdalkalimetallhaltigen Base mit einer Verbindung der allgemeinen Formel VIII 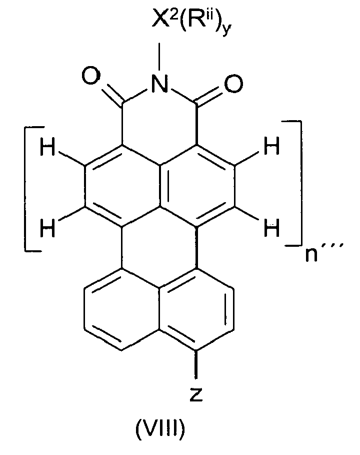 wobei n"' für 0 oder 1 steht und Z für Wasserstoff, Brom oder Chlor steht, umsetzt.

14. Verbindungen der allgemeinen Formeln I oder II 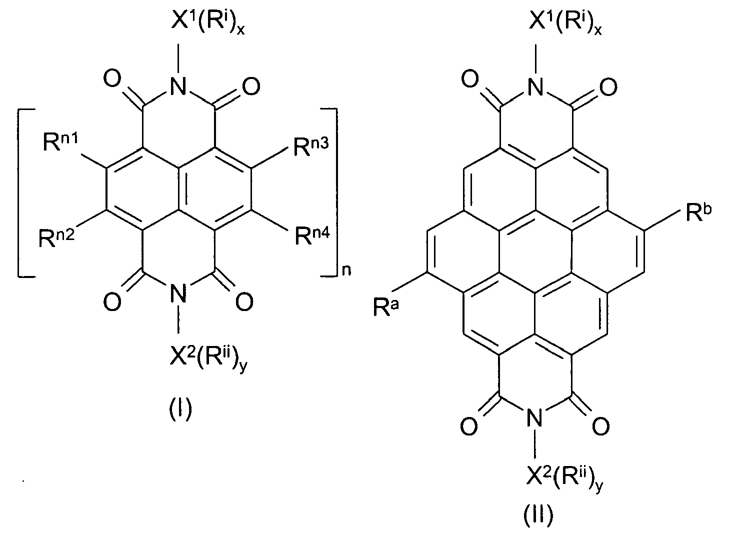 wobei
n für 1, 2, 3 oder 4 steht,
die Reste Rⁿ¹, Rⁿ², Rⁿ³ und Rⁿ⁴ für n = 1 oder 2 unabhängig voneinander ausgewählt sind unter Wasserstoff, F, Cl, Br und CN, für n = 3 oder 4 unabhängig voneinander ausgewählt sind unter Wasserstoff, F, Cl und Br,
die Reste R^{a} und R^{b} unabhängig voneinander ausgewählt sind unter Wasserstoff und Alkyl,
X¹ für einen (x+1)-wertigen Rest steht,
X² für einen (y+1)-wertigen Rest steht,
die Reste Rⁱ und Rⁱⁱ jeweils unabhängig voneinander ausgewählt sind unter C₄- bis C₃₀-Alkyl, welche durch ein oder mehrere nicht benachbarte Sauerstoffatom(e) unterbrochen sein können.
